# EUROPEAN PATENT SPECIFICATION

(11) **EP 3 467 119 B1**
(45) Date of publication and mention of the grant of the patent: **23.02.2022**
(21) Application number: 18205893.3
(22) Date of filing: 12.11.2012
(51) Int. Cl.: C12Q 1/37, C12Q 1/66

(54) **MUTANT PROTEASE BIOSENSORS WITH ENHANCED DETECTION CHARACTERISTICS**
MUTANTE PROTHEASE-BIOSENSOREN MIT VERBESSERTEN ERKENNUNGSEIGENSCHAFTEN
BIOCAPTEURS DE PROTÉASE MUTANTE PRÉSENTANT DE MEILLEURES CARACTÉRISTIQUES DE DÉTECTION

(30) Priority: 11.11.2011 US 201161558796 P
(43) Date of publication of application: 10.04.2019
(62) Divisional of application: 12791055.2
(73) Proprietor: Promega Corporation, Madison, WI 53711-5399 (US)
(72) Inventor: Binkowski, Brock, Lodi, WI 53555 (US); Butler, Braeden L., Madison, WI 53704 (US); Encell, Lance P., Fitchburg, WI 53711 (US); Fan, Frank, Madison, WI 53711 (US); Hook, Brad, Fitchburg, WI 53711 (US); Otto, Paul, Madison, WI 53704 (US); Risma, Kimberly, Cincinnati, OH 45242 (US); Vidugiris, Gediminas, Fitchburg, WI 53711 (US); Wigdal, Susan, Belleville, WI 53508 (US); Zimmerman, Kristopher, Madison, WI 53711 (US)
(74) Representative: Hoffmann Eitle

(56) References cited:
- EP-A1- 2 154 159
- WO-A1-01/20002
- WO-A1-2011/143339
- WO-A2-00/24878
- WO-A2-2004/059294
- WO-A2-2007/120522
- US-A1- 2007 184 493
- SUSAN S. WIGDAL ET AL: "A Novel Bioluminescent Protease Assay Using Engineered Firefly Luciferase", CURRENT CHEMICAL GENOMICS, vol. 2, no. 1, 30 October 2008 (2008-10-30), pages 16-28, XP055003020, ISSN: 1875-3973, DOI: 10.2174/1875397300802010016
- FAN FRANK ET AL: "Novel genetically encoded biosensors using firefly luciferase", ACS CHEMICAL BIOLOGY, AMERICAN CHEMICAL SOCIETY, WASHINGTON, DC, US, vol. 3, no. 6, 20 June 2008 (2008-06-20), pages 346-351, XP002553151, ISSN: 1554-8929, DOI: 10.1021/CB8000414 [retrieved on 2008-06-20]
- BINKOWSKI B ET AL: "Engineered luciferases for molecular sensing in living cells", CURRENT OPINION IN BIOTECHNOLOGY, LONDON, GB, vol. 20, no. 1, 1 February 2009 (2009-02-01), pages 14-18, XP026095186, ISSN: 0958-1669, DOI: 10.1016/J.COPBIO.2009.02.013 [retrieved on 2009-03-18]
- KANNO AKIRA ET AL: "Detection of apoptosis using cyclic luciferase in living mammals", METHODS IN MOLECULAR BIOLOGY; [METHODS IN MOLECULAR BIOLOGY; ISSN 1064-3745; VOL. 1310], HUMANA PR, US, vol. 574, 1 January 2009 (2009-01-01), pages 105-114, XP009188244, ISBN: 978-1-61779-291-5
- B. Z. PACKARD ET AL: "Granzyme B Activity in Target Cells Detects Attack by Cytotoxic Lymphocytes", THE JOURNAL OF IMMUNOLOGY, vol. 179, no. 6, 4 September 2007 (2007-09-04), pages 3812-3820, XP055375698, US ISSN: 0022-1767, DOI: 10.4049/jimmunol.179.6.3812
- C. BACKES ET AL: "GraBCas: a bioinformatics tool for score-based prediction of Caspase- and Granzyme B-cleavage sites in protein sequences", NUCLEIC ACIDS RESEARCH, vol. 33, no. Web Server, 1 July 2005 (2005-07-01), pages W208-W213, XP055227558, ISSN: 0305-1048, DOI: 10.1093/nar/gki433

## Description

### FIELD OF INVENTION

The present invention relates to the field of biochemical assays and reagents. More specifically, this invention relates to modified luciferases and methods for their use. The present invention relates, in general, to non-radioactive methods for the detection of cell-mediated cytotoxicity. The methods of the present invention can be used to detect cell-mediated cytotoxicity by cytotoxic lymphocyte or natural killer cells, in particular activated cytotoxic lymphocyte and activated natural killer cells. The methods of the present invention can be used in cellular assays to monitor granzyme B activity in cellular processes such as β-arrestin localization. The present invention also provides novel Granzyme B recognition sequences and biosensors as well as methods of their use.

### BACKGROUND

Luciferases are enzymes that catalyze the oxidation of a substrate (e.g., luciferin or coelenterazine) with the concomitant release of photons of light. Luciferases have been isolated from numerous species, including Coleopteran arthropods and many sea creatures as well as bacteria. Because it is easily detectable and its activity can be quantified with high precision, luciferases have been widely used to study gene expression and protein localization. Unlike green fluorescent protein (GFP), which requires up to 30 minutes to form its chromophore, the products of luciferases can be detected immediately upon completion of synthesis of the polypeptide chain (if substrate and oxygen are also present). In addition, no post-translational modifications are required for enzymatic activity, and the enzyme contains no prosthetic groups, bound cofactors, or disulfide bonds. Luciferases are useful reporters in numerous species and in a wide variety of cells.

Luciferases possess additional features that render them particularly useful as reporter molecules for biosensing, i.e., molecules which reveal molecular properties of a system. Most catalytic reactions generate less than the energy of hydrolysis for two molecules of ATP, or about 70 kJ/mole. However, the luminescence elicited by luciferases has much higher energy content. For instance, the reaction catalyzed by firefly luciferase (560 nm) emits 214 kJ/mole of energy. Furthermore, luciferases are also highly efficient at converting chemical energy into photons, i.e., they have high quantum yields. Luciferases are thus extremely efficient for generating detectable signals.

Cytotoxic T lymphocytes (CTLs) and natural killer (NK) cells are effector lymphocytes that share common cytotoxic pathways that are necessary for defense against virus-infected or transformed cells such as tumor cells. NK cells play a major role in the rejection of tumors and cells infected by viruses. They kill cells by releasing, via exocytosis, into target cells small cytoplasmic granules of proteins called perforin and granzyme that cause the target cell to die by apoptosis, i.e., programmed cell death. The granule-exocytosis pathway powerfully activates cell-death pathways that operate through the activation of apoptotic cysteine proteases (caspases), but it also leads to cell death in the absence of activated caspases. Perforin and granzymes induce target-cell apoptosis cooperatively. Granzymes, a family of structurally related serine proteases, are necessary for triggering apoptosis in target cells, but they depend on being appropriately delivered by perforin.

Granzyme B and granzyme B isoforms, which cleave target-cell proteins at specific aspartate residues, is a potent activator of caspase-mediated, as well as caspase-independent cell death. The uptake of granzyme B into target cells is believed to be mediated (at least in part) by endocytosis through the cation-independent mannose 6-phosphate receptor. Granzyme B mainly triggers caspase activation indirectly rather than by direct caspase processing. It achieves this by directly activating pro-apoptotic 'BH3-only'members of the BCL-2 family, such as BH3-interacting domain death agonist (BID), which results in the leakage of pro-apoptotic mitochondrial mediators, such as cytochrome c, into the cytosol. BID-independent pathways for granzyme-mediated apoptosis also seem to exist. Some viruses encode potent granzyme-B inhibitors, and the endogenous granzyme-B inhibitor PI9 might be expressed aberrantly by some cancer cells.

CTLs and NK cells are critical effector cells of the immune system. The components of NK function include: NK numbers, granule number/content, immune synapse formation, polarization toward synapse, docking to membrane, priming, fusion, and delivery of granzyme. Measurement of target cell damage has historically been an important measure of CTL and NK cell function. The gold standard for CTL-mediated cytotoxicity has been the chromium release assay ("[51Cr] release assay"). The [51Cr] release assay involves the labeling of viable cells with [51Cr] which binds tightly to most intracellular proteins. After washing, the cells are incubated with a cytotoxic agent, e.g., effector cells. Labeled proteins are released into the cell media due to damage and/or leakage of the cell membrane caused by the triggering of cell death by the cytotoxic agent. Radioactivity is detected, thereby indicating cell death. As the [51Cr] release assay involves the use of radioactivity, and is time intensive, there exists a need to provide a non-radioactive, sensitive assay for the measurement of cell-mediated cytotoxicity. WO 2007/120522 A2 states that it provides a modified luciferase protein which is a sensor for molecules including cAMP, cGMP, calcium, chelators thereof, kinases or phosphatases.

Wigdal et al., 2008. Curr Chem Genomics. 2: 16-28 states that it further demonstrates the utility of a bioluminescent protease assay using a circularly permuted form of firefly luciferase for the evaluation of protease recognition sequence specificity and subsequent establishment of an optimized assay for the identification and characterization of protease inhibitors using high throughput screening.

WO 01/20002 A1 states that it discloses luciferase enzymes with greatly increased thermostability.

WO 2011/143339 A1 states that it discloses a polynucleotide encoding a biosensor polypeptide comprising a modified circularly- permuted thermostable luciferase and a linker linking the C-terminal portion of the thermostable luciferase to the N-terminal portion of the thermostable luciferase.

Fan et al., 2008. ACS Chem Biol. 3(6):346-51 states that the authors genetically engineered firefly luciferase to detect specific intermolecular interactions through the modulation of its luminescence activity.

Packard et al., 2007. J Immunol. 179(6):3812-20 states that the authors combined a Granzyme B substrate with a second fluorogenic substrate selective for caspase 3 to allow both flow cytometry and fluorescence confocal microscopy studies of cytotoxicity.

US 2007/0184493 Al discloses that it provides a non-radioactive assay to monitor and quantify the target-cell killing activities mediated by cytotoxic T lymphocytes.

Backes et al., 2005. Nucleic Acids Res. 33(Web Server issue):W208-13 states that the authors present a bioinformatics tool "GraBCas" that provides score-based prediction of potential cleavage sites for the caspases 1-9 and granzyme B including an estimation of the fragment size.

### SUMMARY

The present invention provides a polynucleotide encoding a circularly-permuted thermostable luciferase comprising a peptide linker linking the original N- and C-termini of the non-permuted thermostable luciferase, wherein the peptide linker comprises a granzyme B recognition site, wherein the granzyme B recognition site is GRIEADSE (SEQ ID NO:80), KSVGPDFG (SEQ ID NO:81), or GIETDSG (SEQ ID NO:82), wherein the circularly-permuted thermostable luciferase has increased luminescence following cleavage by granzyme B when compared to an uncleaved circularly-permuted thermostable luciferase; wherein the modified circularly-permuted thermostable luciferase has a substitution of at least one amino acid at positions 5, 17, 21, 23, 26, 39, 44, 51, 81, 101, 103, 110, 114, 115, 119, 123, 126, 128, 133, 137, 186, 191, 192, 193, 196, 208, 211, 214, 226, 228, 230, 233, 264, 273, 275, 286, 287, 294, 295, 297, 302, 303, 304, 306, 308, 309, 313, 324, 329, 331, 343, 348, 353, 364, 374, 385, 389, 409, 420, 426, 427, 428, 431, 449, 456, 460, 461, 465, 466, 468, 471, 473, 482, 484, 485, 489, 493, 494, 497, 503, 507, 509, 510, 513, 516, 517, 521, 522, 523, 526, 530, 533, 536, 537, 542, or 543 corresponding to SEQ ID NO: 2; wherein the modified circularly-permuted thermostable luciferase has an enhanced response after interaction of the modified circularly-permuted thermostable luciferase with granzyme B relative to the parental circularly-permuted thermostable luciferase corresponding to SEQ ID NO: 2 in the presence of granzyme B. In some embodiments, the granzyme B recognition site is flanked by peptide linkers.

The present invention also provides a vector comprising the polynucleotide of the present invention. Further, the prese invention provides a cell or kit comprising the polynucleotide of the present invention or the vector of the present invention.

The present invention also provides a circularly-permuted thermostable luciferase biosensor encoded by the polynucleotide of the present invention.

The present invention also provides a method for detecting at least one of the presence or amount of active granzyme B in a cell or sample, the method comprising:
a) contacting the cell or sample with: (i) the polynucleotide of the present invention, the vector of the present invention, or the biosensor of the present invention, and (ii) a substrate for the circularly-permuted thermostable luciferase biosensor; and
b) detecting luminescence in the cell or sample, wherein the granzyme B is capable of cleaving the peptide linker in the circularly-permuted thermostable luciferase biosensor, thereby detecting the presence or amount of active granzyme B.

The present invention also provides a method for detecting at least one of the presence or amount of active granzyme B in a cell or sample, the method comprising:
a) contacting the cell or sample with:
   i) the polynucleotide of the present invention, the vector of the present invention, or the biosensor of the present invention;
   ii) a substrate for the circularly-permuted thermostable luciferase biosensor; and
   iii) a cytotoxic lymphocyte; and
b) detecting luminescence in the cell or sample, wherein the granzyme B is capable of cleaving the peptide linker of the circularly-permuted thermostable luciferase biosensor, thereby detecting the presence or amount of active granzyme B released from cytotoxic lymphocytes. In some embodiments, the cytotoxic lymphocytes are patient or patient-derived cytotoxic T lymphocytes or cytokine-activated natural killer cells; and/or the detection of active granzyme B determines the cytotoxic function of the cytotoxic lymphocyte. In some embodiments, the detection of active granzyme B indicates cell-mediated cytotoxicity.

The present invention also provides a method of screening for one or more modulators of granzyme B activity, the method comprising:
a) contacting a cell or sample with:
   i) the polynucleotide of the present invention, the vector of the present invention, or the biosensor of the present invention;
   ii) one or more test agents; and
   iii) a substrate for the circularly-permuted thermostable luciferase biosensor; and
b) detecting luminescence in the cell or sample, wherein the granzyme B is capable of cleaving the peptide linker of the circularly-permuted thermostable luciferase biosensor, thereby identifying modulators of granzyme B. In some embodiments, the modulator is an inhibitor of granzyme B or an enhancer of granzyme B.

The present invention also provides a method of screening for one or more modulators of a cytotoxic function of a cell, the method comprising:
a) contacting a target cell population with:
   i) the polynucleotide of the present invnetion, the vector of the present invention, or the biosensor of the present invention; and
   ii) a substrate for the circularly-permuted thermostable luciferase biosensor;
b) contacting the target cell population and/or cells mediating cytotoxicity with one or more test agents; and
c) detecting luminescence in the target cell population, wherein the granzyme B released from the cells mediating cytotoxicity is capable of cleaving the peptide linker of the circularly-permuted thermostable luciferase biosensor in the target cell population, thereby identifying modulators of the cytotoxic function.

The present invention also provides a method of detecting granzyme B activity in a cellular process, the method comprising:
a) contacting a cell or sample with:
   i) one or more test agents;
   ii) the polynucleotide of the present invention, the vector of the present invention, or the biosensor of the present invention; and
   iii) a substrate for the circularly-permuted thermostable luciferase biosensor; and
b) detecting luminescence in the cell or sample, wherein the granzyme B is capable of cleaving the peptide linker in the circularly-permuted thermostable luciferase biosensor, thereby detecting granzyme B activity.

Further, the present invention provides a method of screening for one or more modulators of cytotoxic function, the method comprising:
a) contacting a target cell population with:
   i) the polynucleotide of the present invention, the vector of the present invention, or the biosensor of the present invention; and
   ii) a substrate for the circularly-permuted thermostable luciferase biosensor; and
b) contacting the target cell population and/or cells mediating cytotoxicity with one or more test agents; and
c) detecting luminescence in the target cell population, wherein granzyme B released from the cells mediating cytotoxicity cleaves and activates the circularly-permuted thermostable luciferase biosensor in the target cell population, thereby identifying modulators of the cytotoxic function.

In some embodiments, the cytotoxic lymphocyte(s) is a patient or patient-derived cytotoxic lymphocyte(s) or a cytokine-activated natural killer cell. In some embodiments, the detection of granzyme B determines the cytotoxic function of the cytotoxic lymphocyte. In other embodiments, the detection of granzyme B indicates cell-mediated cytotoxicity. In some embodiments, the screening is performed in high-throughput, e.g., in a multi-well plate.

In some embodiments, the modulator is an inhibitor or an enhancer of granzyme B. In some embodiments, the inhibitor is a siRNA. In some embodiments, the enhancer is a small molecule, peptide, or protein. In some embodiments, the screening is performed in high-throughput, e.g., in a multi-well plate.

In some embodiments, the modulator is an inhibitor or an enhancer of granzyme B. In some embodiments, the inhibitor is a siRNA. In some embodiments, the enhancer or inhibitor is a small molecule, peptide, or protein. In some embodiments, the cells mediating cytotoxicity is a cytotoxic lymphocyte(s) or a cytokine-activated natural killer (NK) cell(s). In some embodiments, the screening is performed in high-throughput, e.g., in a multi-well plate.

In some embodiments, the screening is performed in high-throughput, e.g., in a multi-well plate.

In some embodiments, the disclosure relates to a non-human animal comprising a cell comprising a polynucleotide the of the present invention encoding the circularly-permuted thermostable luciferase of the present invention

### BRIEF DESCRIPTION OF THE DRAWINGS

FIG. 1 illustrates the position of the amino acid substitutions, I471T, S503G, T507I, and S193P in the corresponding starting sequence TL-CP358-DEVD:DD.
FIGS. 2A-B show the normalized RLU for the variant 01 :A-05 and the corresponding starting sequence TL-CP358-DEVD:DD after treatment with TNF-α-related apoptosis inducing ligand (TRAIL) treatment (FIG. 2A) and the fold-induction (response) after 2 and 10 hrs (FIG. 2B).
FIGS. 3A-B show the normalized RLU for the variants FC7:24, FC7:43, and FC7:49, compared to corresponding starting sequence TL-CP358-DEVD:DD after treatment with TRAIL (FIG. 3A) and the fold-induction (response) after 2 and 10 hrs (FIG. 3B).
FIG. 4 shows the effect of the linker on the performance of the Caspase-3/7 BioSensor (CBS).
FIG. 5 shows the kinetic profile of Caspase 8 activation by TRAIL over time at 37°C using TL-CP233-Caspase 8 and TL-CP358-Caspase 8 Biosensors.
FIG. 6 shows the fold response of Caspase 8 activation by TRAIL over time using TL-CP233-Caspase 8 and TL-CP358-Caspase 8 Biosensors.
FIGS. 7A-D show the kinetic profile of Caspase 8 activation by TRAIL over time at 37°C using FF-CP359 Caspase 8 (FIG. 7A), TL-CP233-Caspase 8 (FIG. 7B), TL-CP358-Caspase 8 (FIG. 7C), and TL-CP358-Caspase3 (FIG. 7C) Biosensors.
FIG. 8 shows fold response of Caspase 8 activation by TRAIL over time using TL-CP358-Caspase 3, TL-CP233-Caspase 8, TL-CP358-Caspase 8 and FF-CP359-Caspase 8 Biosensors.
FIG. 9 shows TEV protease Biosensors detect TEV co-expressed in CHO cells.
FIGS. 10A-D show the luminescence (photon counts/sec) of D54-MG cells expressing the various thermostable biosensors upon treatment with TRAIL at various time points (FIG. 10A), the fold induction (FIG. 10B), the average photon counts/sec at baseline, 2, 4 and 6 hrs post treatment (FIG. 10C) and a Western blot showing reporter expression (FIG. 10D).
FIGS. 11A-C show the luminescence (photon counts/sec) of D54-MG reporter xenografted nude mice treated with 8 mg/kg of TRAIL (FIG. 11A), the fold induction (FIG. 11B), and average photon counts/sec at baseline and 6 hrs post treatment (FIG. 11C).
FIGS. 12A-D show the normalized data compared to pre-treatment values for intratibial implanted MDA-MB23101833 cells stably expressing TL-CP233-Caspase 3 treated with TRAIL (FIG. 12A), the Z factor calculated for every time point (FIG. 12B), representative images taken at the indicated time points (FIG. 12C), and fold induction of xenografted animals tested treated with TRAIL (FIG. 12D).
FIGS. 13A-D show the relative luminescence upon compound treatment (max) from compounds in the NIH Clinical Collection Biofocus Library (FIG. 13A) and the TimTec Kinase Inhibitor Library (FIG. 13C) and the heat map of data acquired for the NIH Clinical Collection Biofocus Library (FIG. 13B) and the TimTec Kinase Inhibitor Library (FIG. 13D).
FIG. 14 shows SDS-PAGE gel analysis of the proteins at various stages during the purification process.
FIG. 15 shows the fold increase over control (background from MMP-2 sensor).
FIGS. 16A-B show the luminescence of the MMP-2 protein using the SensoLyte assay, (FIG. 16A) and the fold-induction (FIG. 16B).
FIG. 17 shows cleavage of CBS-HT by Caspase-3 detected by SDS-PAGE gel analysis.
FIGS. 18A-B show illustrations of the immobilization of CBS to a HaloLink resin (FIG. 18A) or a microtiter plate (FIG. 18B).
FIG. 19 shows the luminescence of protease biosensor expressed in a cell-free environment.
FIG. 20 shows the luminescence of protease biosensor expressed in *E. coli.*
FIGS. 21A-B show SDS-PAGE analysis of samples labeled with CA-TAM (FIG. 21A) and the luminescence of purified protease biosensor (FIG. 21B).
FIG. 22 shows the luminescence of the protease biosensor immobilized to a solid support.
FIG. 23 shows the kinetic data using NK92 cells and the DEVD construct at 37°C measured with a Biotek Reader.
FIG. 24 shows luminescence of NK92 cells and the DEVD construct at room temperature measured with a Veritas reader.
FIG. 25 shows the luminescence of the basal signal (i.e., no effectors) of the granzyme B constructs compared to the controls measured with a Veritas reader.
FIGS. 26A-E show the kinetic data of the DEVD construct (FIG. 26A and FIG. 26E), the granzyme B cleavable D+ construct (FIG. 26B and FIG. 26D), and the granzyme B negative control (D-) construct (FIG. 26C).
FIGS. 27A-B show that activated cytotoxic T cells (CTL) from P14 mice trigger caspase 3/7 and granzyme B (C+) biosensor in murine EL4 target cells through antigen specific recognition.
FIGS. 28A-B show that the caspase 3/7 biosensor (DEVD) is dependent on the delivery of granzyme B into the target cells and perforin.
FIG. 29 shows that the granzyme B biosensor (C+) is dependent on delivery of granzyme B into the target cells.
FIGS. 30A-B show that constructs with granzyme B cleavage sites have dose dependent increases in luminescence with increasing concentrations of granzyme B enzyme.
FIG. 31 shows granzyme B delivery induced luminescence in IL-2 activated NK cell lines from healthy control (WT/WT) and patient with biallelic perforin mutations (MUT/MUT).
FIGS. 32A-B show the use of caspase 3/7 biosensor and the granzyme B biosensor with primary NK and NK92 cells
FIG. 33 shows that the inhibition of perforin-dependent killing blocks activation of the granzyme B biosensor by granzyme B.
FIG. 34 shows NKs are able to activate luminescence from a Granzyme B biosensor after prolonged culture with IL-2.
FIG. 35 shows umbilical cord derived, IL-15 stimulated cells are capable of releasing Granzyme B and activating luminescence from target cells containing a Granzyme B biosensor.
FIGS. 36A-B show the use of caspase 3/7 biosensor and the granzyme B biosensor in Jurkat target cells.

### DETAILED DESCRIPTION

In the following description of the methods of the invention, process steps are carried out at room temperature (about 22°C) and atmospheric pressure unless otherwise specified. It also is specifically understood that any numerical range recited herein includes all values from the lower value to the upper value. For example, if a concentration range or beneficial effect range is stated as 1% to 50%, it is intended that values such as 2% to 40%, 10% to 30%, or 1% to 3%, etc. are expressly enumerated in this specification. Similarly, if a sequence identity range is given as between, e.g., 60% to <100%, it is intended that 65%, 75%, 90%, etc. are expressly enumerated in this specification. These are only examples of what is specifically intended, and all possible numerical values from the lowest value to the highest value are considered expressly stated in the application.

The term "thermostable luciferase" includes a luciferase that has enhanced stability at a given temperature (e.g., 22°C) compared to a corresponding wild-type luciferase. For the luciferases disclosed herein, the term "TL" is used to refer to a thermostable variant of Ppe2, where Ppe2 is a luciferase from *Photuris pennsylvanica.* One skilled in the art would recognize that other thermostable luciferases are known. For example, a luciferase from *Photinus pyralis*, as well as luciferases from *Luciola cruciata, Luciola lateralis, Pyrocoelia miyako, Lampyris noctiluca, Photuris pennsylvanica, Phengodes sp., Luciola mingrelica,* and *Photinus pyralis. (See* Ye et al., Biochimica et Biophysica Acta, 1339:39-52 (1997)).

The term "CP" refers to circularly-permuted. For example, "TL-CP" refers to a circularly-permuted thermostable variant of the Ppe2 luciferase from *Photuris pennsylvanica.* The term "DEVD:DD" refers to a linker, i.e., an amino acid sequence that connects the N- and C-terminals of a circularly-permuted luciferase, that contains the DEVD caspase 3/7 recognition site and the three amino acids, GSL, that are on the C-terminal side of the DEVD caspase recognition site.

The term "biosensor" refers to an amino acid sequence containing a sensor region which can interact with a target molecule. When the target molecule interacts with the sensor region, molecular properties of a system are revealed.

The terms "Caspase-3/7 BioSensor" and "CBS" refers to a biosensor comprising a thermostable variant of the Ppe2 luciferase from *Photuris pennsylvanica* circularly-permuted with a caspase-3/7 recognition site, i.e., one containing the caspase-3/7 recognition site, DEVD, at the junction between the modified TL fragments. For example, "TL-CP358-DEVD:DD" refers to a CBS circularly-permuted at position 358 relative to SEQ ID NO:2 and comprises the DEVD:DD linker connecting the N- and C- terminal ends of the circularly-permuted thermostable luciferase. The term "CBS variant" refers to a CBS with one or more amino acid substitutions relative to CBS.

The amino acid numbering used throughout this application to identify substituted residues is specified relative to the positions in the polypeptide sequence of the wild-type Ppe2 luciferase from *Photuris pennsylvanica*, i.e., SEQ ID NO:2

The term "caspase-3 or caspase-7 recognition site" refers to a peptide sequence that is recognized and cleaved by either a caspase-3 or caspase-7 isoform. For example, the caspase-3 or caspase-7 recognition site may include DEVDG (SEQ ID NO:55).

The term "granzyme B recognition site" refers to a peptide sequence that is recognized and cleaved by a granzyme B isoform.

The term "target molecule" refers to a molecule of interest that interacts with the biosensor, e.g., a protease, a kinase, a G-protein coupled receptor, cAMP, cGMP, enzyme cofactors, ions (e.g., calcium ion; hydrogen ion for use as a pH sensor), an antibody, a peptide, or a sugar that causes the biosensor to reveal molecular properties of a system.

The term "identity" in the context of two or more nucleic acids or polypeptide sequences, refers to two or more sequences or subsequences that are the same or have a specified percentage of amino acid residues or nucleotides that are the same when compared and aligned for maximum correspondence over a comparison window or designated region as measured using any number of sequence comparison algorithms or by manual alignment and visual inspection. Methods of alignment of sequence for comparison are well-known in the art.

The terms "cell," "cell line," and "host cell," as used herein, are used interchangeably, and all such designations include progeny or potential progeny of these designations. The term "transformed cell" refers to a cell into which (or into an ancestor of which) has been introduced a nucleic acid molecule of the invention. Optionally, a nucleic acid molecule of the invention may be introduced into a suitable cell line so as to create a stably-transfected cell line capable of producing the protein or polypeptide encoded by the nucleic acid molecule of the invention. Vectors, cells, and methods for constructing such cell lines are well known in the art. The words "transformants" or "transformed cells" include the primary transformed cells derived from the originally transformed cell without regard to the number of transfers. All progeny may not be precisely identical in DNA content, due to deliberate or inadvertent mutations. Nonetheless, mutant progeny that have the same functionality as screened for in the originally transformed cell are included in the definition of transformants.

As used herein, the term "heterologous" nucleic acid sequence or protein refers to a sequence that, relative to a reference sequence, has a different source, e.g., originates from a foreign species, or, if from the same species, it may be substantially modified from the original form. The term "homology" refers to a degree of complementarity between two or more sequences. There may be partial homology or complete homology (i.e., identity).

The term "nucleic acid molecule," "polynucleotide," or "nucleic acid sequence" as used herein, refers to nucleic acid, DNA or RNA, that comprises coding sequences necessary for the production of a polypeptide or protein precursor. The encoded polypeptide may be a full-length polypeptide, a fragment thereof (less than full-length), or a fusion of either the full-length polypeptide or fragment thereof with another polypeptide, yielding a fusion polypeptide.

A polynucleotide encoding a protein or polypeptide means a nucleic acid sequence comprising the coding region of a gene, or in other words, the nucleic acid sequence encodes a gene product. The coding region may be present in a cDNA, genomic DNA or RNA form. When present in a DNA form, the oligonucleotide may be single stranded (i.e., the sense strand) or double stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Other control or regulatory elements include, but are not limited to, transcription factor binding sites, splicing signals, polyadenylation signals, termination signals, and enhancer elements.

As used herein, "parental" refers to the starting amino acid or nucleotide sequence that is used to generate the variants with further manipulations of the present invention. For example a wild-type *Photuris pennsylvanica* Ppe2 luciferase (SEQ ID NO:2), can be used as the starting sequence to generate the variants

By "peptide," "protein" and "polypeptide" is meant any chain of amino acids, regardless of length or post-translational modification (e.g., glycosylation or phosphorylation).

As used herein, "pure" means an object species is the predominant species present (i.e., on a molar basis it is more abundant than any other individual species in the composition), and in one embodiment a substantially purified fraction is a composition wherein the object species comprises at least about 50% (on a molar basis) of all macromolecular species present. Generally, a "substantially pure" composition will comprise more than about 80% of all macromolecular species present in the composition, in one embodiment more than about 85%, about 90%, about 95%, or about 99%. In one embodiment, the object species is purified to essential homogeneity (contaminant species cannot be detected in the composition by conventional detection methods) wherein the composition consists essentially of a single macromolecular species.

Nucleic acids are known to contain different types of mutations. A "substitution" refers to an alteration in the sequence of a nucleotide at one or more base position(s) from the parental sequence. Mutations may also refer to insertion or deletion of one or more bases, so that the nucleic acid sequence differs from a parental sequence (e.g., a wild-type) or has a replacement stop codon.

The term "responsivity" refers to the alteration in luminescence, e.g., increased or decreased luminescence, due to the interaction of the biosensor with the target molecule.

As used herein, a "sample" may refer to a cell, an animal, cell lysate, or an *in vitro* transcription/translation mixture.

The term "vector" refers to nucleic acid molecules into which fragments of DNA may be inserted or cloned and can be used to transfer DNA segment(s) into a cell and capable of replication in a cell. Vectors may be derived from plasmids, bacteriophages, viruses, cosmids, and the like.

The term "wild-type" as used herein, refers to a gene or gene product that has the characteristics of that gene or gene product isolated from a naturally occurring source. The gene or gene product can be naturally occurring or synthetic. A wild-type gene is that which is most frequently observed in a population and is thus arbitrarily designated the "wild-type" form of the gene. In contrast, the term "mutant" or "variant" refers to a gene or gene product that displays modifications in sequence and/or functional properties (i.e., altered characteristics) when compared to the wild-type gene or gene product. It is noted that naturally-occurring and synthetic mutants can be isolated and are identified by the fact that they have altered characteristics when compared to the wild-type gene or gene product.

Luminescence refers to the light output of a luciferase polypeptide under appropriate conditions, e.g., in the presence of a suitable substrate such as a luciferin. The light output may be measured as an instantaneous or near-instantaneous measure of light output (which is sometimes referred to as "T=0" luminescence or "flash") upon start of the luminescence reaction, which may start upon addition of the luciferin substrate. The luminescence reaction in various embodiments is carried out in a solution containing lysate, for example, from the cells in a prokaryotic or eukaryotic expression system. In other embodiments, expression occurs in an *in vitro* system, or the luciferase protein is secreted into an extracellular medium, such that, in this latter case, it is not necessary to produce a lysate. In other embodiments, the luciferase is expressed in a whole cell(s). In some embodiments, the reaction is started by injecting appropriate materials, e.g., luciferin, into a reaction chamber (e.g., a well of a multiwell plate such as a 96-well plate) containing the luciferase protein. The reaction chamber may be situated in a reading device which can measure the light output, e.g., using a luminometer or photomultiplier. When the luciferase is expressed in whole cell(s), the reaction is started by the administration of a luciferase substrate, e.g., luciferin. For a whole cell(s), this administration may include addition of the luciferase substrate into the cell media. The light output or luminescence may also be measured over time, for example in the same reaction chamber, cell(s) for a period of seconds, minutes, hours, etc. The light output or luminescence may be reported as the average over time, the half-life of decay of signal, the sum of the signal over a period of time, or as the peak output. Luminescence can also be detected via imaging, e.g., *in vivo* imaging.

Enhanced response includes the differential activity before and after the TL-CP biosensor interacts with a target molecule. The basal activity of the TL-CP biosensor is defined as the activity at assay time (0), before the biosensor interacts with a target molecule. The induced activity is defined as the activity at some later time (t) after the TL-CP biosensor has been interacted with a target molecule. The response or fold increase in activity is the ratio of induced to basal activity.

Enhanced luminescence includes increased light output as determined by suitable comparison of comparably-obtained measurements. As disclosed herein, one or more suitable amino acid substitutions to the TL-CP biosensor sequence produce TL-CP biosensor polypeptides which exhibit enhanced luminescence. Changes in the nucleotide sequence from the parental thermostable luciferase nucleotide sequence may contribute to enhanced luminescence by leading to an amino acid substitution and/or by enhancing protein expression.

Enhanced signal stability includes an increase in how long the signal from a luciferase continues to luminescence, for example, as measured by the half-life of decay of the signal in a time-course.

Enhanced protein stability includes increased thermostability (e.g., stability at elevated temperatures) and chemical stability (e.g., stability in the presence of denaturants such as detergents, including e.g., Triton X-100).

Luciferase biosensors have been previously described, see e.g., U.S. Patent Publication No. 2005/0153310,. The sensor regions are cloned into a circularly-permuted luciferase such that when the luciferase biosensor interacts with a target molecule, an enhanced or increased luminescence is generated relative to a luciferase biosensor which has not been contact with a target molecule. Alternatively, the sensor regions are cloned into a circularly-permuted luciferase such that when the luciferase biosensor interacts with a target molecule, a decrease or no luminescence is generated relative to a luciferase biosensor which has not been in contact with a target molecule. The sensor regions may be useful for detecting the activity of a protease, the binding of cyclic nucleotides such as cAMP and cGMP, the presence or concentration of calcium, other ions or antibodies, the presence or concentrations of one or more G-protein coupled receptor ligands, a change in pH, the activity of a phosphatase or kinase or other enzymes, binding proteins or molecules of interest such as a peptide or a sugar known to those of skill in the art.

In some embodiments, a polynucleotide of the invention is optimized for expression in a particular host. As used herein, optimization includes codon optimization as well as, in eukaryotic cells, introduction of a Kozak sequence, and/or one or more introns. Thus, a nucleic acid molecule may have a codon composition that differs from that of a wild-type nucleic acid sequence encoding an unmodified luciferase at more than 30%, 35%, 40% or more than 45%, e.g., 50%, 55%, 60% or more of the codons.

In some embodiments of the invention, the codons that are different are those employed more frequently in a mammal, while in another embodiment the codons that are different are those employed more frequently in a plant. A particular type of mammal, e.g., human, may have a different set of preferred codons than another type of mammal. Likewise, a particular type of plant may have a different set of preferred codons than another type of plant. In some embodiments of the invention, the majority of the codons which differ are ones that are preferred codons in a desired host cell, as those optimized sequences can increase the strength of the signal for luciferase. Preferred codons for mammals (e.g., humans) and plants are known to the art (e.g., Wada et al. NAR 18: 2367(1990); Murray et al. NAR 17: 477 (1989); WO 02/16944).

### Luciferases from a Coleopteran species are known in the art,

e.g., *Luciola cruciata, Luciola lateralis, Pyrocoelia miyako, Lampyris noctiluca, Photuris pennsylvanica, Phengodes sp., Luciola mingrelica,* and *Photinus pyralis.* (See Ye et al., Biochimica et Biophysica Acta, 1339:39-52 (1997)).

The amino acid sequence of the modified TL-CP biosensor is different than the amino acid sequence of a corresponding unmodified TL-CP biosensor (parental), e.g., a mutant luciferase with one or more substitutions in the luciferase sequences. of the invention

In some embodiments, the TL-CP of the invention may include the deletion of one or more amino acids, e.g., at a site(s) or in a region(s) tolerant to modification including the N- and/or C-terminus of the unmodified thermostable luciferase, so long as the resulting TL-CP biosensor has bioluminescent activity before and/or after the interaction with the target, e.g., bioluminescent activity is altered after interaction with the target molecule, such as an alteration in light intensity, color or kinetic profile.

In some embodiments, the circularly-permuted thermostable luciferase includes other modifications, including but not limited to, insertions and/or deletions internal to the N- or C-terminus of the circularly-permuted thermostable luciferase, for instance, another insertion and/or a deletion, e.g., at or near the N- and C-terminus of the corresponding unmodified thermostable luciferase such as at residues corresponding to residues 1 to about 10 or about 30, or any integer in between, of the N-terminus and/or corresponding to the last residue or about the last 30, e.g., last 15, or any integer in between 1 and 30, residues of the C-terminus of the corresponding unmodified thermostable luciferase.

In some embodiments, a thermostable beetle luciferase may be circularly-permuted at a residue, for instance, residue 7, 37, 47, 75, 83, 107, 121, 144, 160, 174, 188, 198, 205, 225, 233, 242, 255, 268, 308, 316, 358, 377, 403, 435, 490 or 540, or in a region corresponding to residue 2 to 12; residue 32 to 53, e.g., residue 32 to 43 or residue 42 to 52; residue 70 to 88, e.g., residue 70 to 80 or residue 78 to 88; residue 102 to 126, e.g., residue 102 to 112 or residue 116 to 126; residue 139 to 165; residue 183 to 203; residue 220 to 247, e.g., residue 228 to 238; residue 262 to 273; residue 303 to 313; residue 353 to 408; residue 485 to 495; or residue 535 to 546 of SEQ ID NO:2 The residue numbering is based on that of an unmodified (native) firefly luciferase sequence. Corresponding positions may be identified by aligning luciferase sequences using, for instance, sequence alignment programs. Residues or regions in a luciferase tolerant to modification may be employed as sites to circularly permute the luciferase or for an insertion.

In some embodiments, the linker has at least one of the following sequences:
G S S G G S G G S G G G (SEQ ID NO:23),
G S S S D S D S S A G S (SEQ ID NO:24),
G S N D S S G G S E G G (SEQ ID NO:25),
G S N G G F D S S E G G (SEQ ID NO:26),
G S I R W S G L S G G D (SEQ ID NO:27),
G S R G G S V Y S E G G (SEQ ID NO:28),
G S S E G S S D F G G D (SEQ ID NO:29),
G S I V V S C S S E G G (SEQ ID NO:30),
G S N W D S G C S R E G (SEQ ID NO:31),
G S N W D S G C S R E C (SEQ ID NO:32),
G S S G C T G D A G G S (SEQ ID NO:33),
G S N W D S G C S R Q C (SEQ ID NO:34),
G S S/N S/D/G D/S/G S/F D/G S/G S A/E G S/G (SEQ ID NO:35),
G S I/R/S R/G/E W/G S G/V/S L/Y/D S/F G/E G D/G (SEQ ID NO: 36),
G S I/N/S V/W/G V/D/C S/T C/G S/C/D S/A E/R/G G/E G/S (SEQ ID NO:37),
G S I/S V/G/A V/G S/C G/D G/D/S S/A G/E G/E G/N (SEQ ID NO:38),
G S I/N/S V/W/G/A V/D/C/G S/T/C C/G S/C/D S/A E/R/G G/E G/S (SEQ ID NO:39),
G S I A G C G D A G E G (SEQ ID NO:40),
G S N W D S G C S R E (SEQ ID NO:41),
G S I A G C G D A G E G (SEQ ID NO:42),
G S N W D S G C S R E G (SEQ ID NO:43),
N W D S G C S R E G (SEQ ID NO:44), or
I A G C G D A G E G (SEQ ID NO:45).

The "/" mark indicates that the amino acid before or after the "/" may be used in that position. A linker employed in the biosensor of the invention is an amino acid sequence, the presence of which in the biosensor does not substantially decrease the activity of that biosensor, e.g., does not decrease the activity by more than 10-fold, such as by no more that 4-fold, or no more than 2-fold, relative to a corresponding biosensor that lacks the linker(s), and/or the presence of the linker employed in the biosensor of the invention increases luminescence or response to interacting with its target, relative to a corresponding biosensor that lacks the linker(s) or a corresponding biosensor having the linker(s) GSSGGSGGSGGG (SEQ ID NO:23), or relative to both corresponding biosensors.

In some embodiments, in the absence of granzyme B, the activity of a modified circularly-permuted thermostable luciferase biosensor of the invention is less than the activity of a corresponding parental (unmodified) circularly-permuted thermostable luciferase biosensor, e.g., the luminescence activity of the modified circularly-permuted thermostable luciferase biosensor is about 0.001%, 0.01%, 0.1%, 1%, 10%, 20%, 50%, 70% or more, but less than 100% that of a corresponding parental (unmodified) circularly-permuted thermostable luciferase biosensor, the activity of which circularly-permuted modified thermostable luciferase biosensor is optionally detectable. In other embodiments, in the absence of granzyme B, the activity of a modified circularly-permuted thermostable luciferase biosensor of the invention is substantially the same or greater than the activity of a parental (unmodified) circularly-permuted thermostable luciferase biosensor, e.g., the luminescence activity of the modified circularly-permuted thermostable luciferase biosensor of the invention is about 1.5-fold, e.g., at least 2-, 3- or 5-fold or more, that of a parental (unmodified) circularly-permuted thermostable luciferase biosensor. In the presence of granzyme B, the activity of the modified circularly-permuted thermostable luciferase biosensor of the invention is detectably increased. For instance, a detectable increase in activity of a modified circularly-permuted thermostable luciferase biosensor in the presence of a target molecule is an alteration of at least 0.001%, 0.01%, 0.1%, 1%, 10%, or 100%, and up to 2-fold, 4-fold, 10-fold, 100-fold, 1,000-fold, 10,000-fold or more, relative to the activity of the modified circularly-permuted thermostable luciferase biosensor in the absence of granzyme B. Thus, the physical proximity of granzyme B increases the activity of the modified circularly-permuted thermostable luciferase biosensor.

The invention includes circularly-permuted biosensors, which luciferase sequence may include deletions of residues at the original (wild type) N- or C-termini, or both, e.g., deletion of 1 to 3 or more residues at the N-terminus and 1 to 6 or more residues at the C-terminus. The luciferase sequences of a modified circularly-permuted thermostable luciferase are substantially the same as the amino acid sequence of an unmodified circularly-permuted thermostable luciferase biosensor. A polypeptide or peptide having substantially the same sequence means that an amino acid sequence is largely, but may not entirely be, the same and retains a functional activity of the sequence to which it is related. In general, two amino acid sequences are substantially the same or substantially homologous if they are at least 80% identical, e.g., have at least 85%, 90%, 95%, 99%, or more identity.

The invention also includes a stable cell line that expresses a modified circularly-permuted thermostable luciferase biosensor, comprises an expression cassette comprising a nucleic acid molecule encoding the modified circularly-permuted thermostable luciferase biosensor of the invention, and/or comprises a vector (e.g., a plasmid, virus, or defective viral particles) capable of expressing the nucleic acid molecule of the invention in a host cell. In one embodiment, the expression cassette comprises a promoter, e.g., a constitutive or regulatable promoter, operably linked to the nucleic acid sequence. In one embodiment, the expression cassette contains an inducible promoter. Also provided is a host cell, e.g., a prokaryotic cell or an eukaryotic cell such as a plant or vertebrate cell, e.g., a mammalian cell, including but not limited to a human, non-human primate, canine, feline, bovine, equine, ovine or rodent (e.g., rabbit, rat, ferret or mouse) cell, which comprises the expression cassette or vector of the invention, and a kit which comprises the nucleic acid molecule, expression cassette, vector, host cell or modified circularly-permuted thermostable luciferase biosensor of the invention.

For instance, a vector encoding a modified circularly-permuted thermostable luciferase biosensor is mixed with a sample, e.g., a cell, cell lysate, *in vitro* transcription/translation mixture, or supernatant, and the activity of the modified circularly-permuted thermostable luciferase biosensor in the sample detected or determined, e.g., optionally at one or more time points, or relative to a control sample without granzyme B or having a differing amount of granzyme B. An alteration in luminescent activity in the sample, for instance, over time, and/or relative to a control, e.g., a cell having a specified amount of granzyme B, indicates the presence or amount of granzyme B in the sample, or change in amount of granzyme B related to experimental condition. In some embodiment, a cell is contacted with a vector comprising a promoter, e.g., a regulatable or constitutive promoter, and a nucleic acid sequence encoding a modified circularly-permuted thermostable luciferase of the invention In some embodiments, a transfected cell is cultured under conditions in which the promoter induces transient expression of the modified circularly-permuted thermostable luciferase biosensor, and the presence or amount of luminescence determined. In other embodiments, a modified circularly-permuted thermostable luciferase biosensor of the invention and a sample suspected of having granzyme B are mixed, and the amount of luminescence determined.

A modified circularly-permuted thermostable luciferase biosensor of the invention may be employed in applications where unmodified circularly-permuted thermostable luciferase biosensor cannot, such as, as a functional reporter to measure or detect various conditions and/or target molecules in a cell or in an animal, e.g., a mouse. For instance, a vector encoding the modified circularly-permuted thermostable luciferase biosensor, is introduced to a cell, an animal, cell lysate, *in vitro* transcription/translation mixture, or supernatant, and the activity of the modified circularly-permuted thermostable luciferase biosensor detected or determined, e.g., at one or more time points and relative to a corresponding unmodified circularly-permuted thermostable luciferase biosensor. An alteration in luminescent activity in the cell, an animal, cell lysate, *in vitro* transcription/translation mixture, or supernatant over time, and/or relative to a control, e.g., a cell having the corresponding unmodified circularly-permuted thermostable luciferase biosensor, indicates the presence of the protease. In some embodiments, the method further comprises adding a test compound wherein the test compound may alter (e.g., decreases, eliminates, or increases) the activity of the granzyme B. In embodiments, the substrate for the modified circularly-permuted thermostable luciferase biosensor may be luciferin or a luciferin derivative.

Described herein is a method of detecting the presence of granzyme B . For instance, a cell is contacted with a vector comprising a promoter, e.g., a regulatable promoter, and a nucleic acid sequence encoding a modified circularly-permuted thermostable luciferase biosensor of the invention. In one embodiment, a transfected cell is cultured under conditions in which the promoter induces transient expression of the modified circularly-permuted thermostable luciferase biosensor, and a detectable activity of the modified circularly-permuted thermostable luciferase biosensor is determined. An animal, e.g., a mouse, may be contacted with a vector comprising a promoter, e.g., a regulatable promoter, and a nucleic acid sequence encoding a modified circularly-permuted thermostable luciferase biosensor of the invention or a transfected cell expressing the modified circularly-permuted thermostable luciferase biosensor of the present invention. Detectable activity of the modified circularly-permuted thermostable luciferase biosensor is then determined.

The invention also provides methods of screening for agents ("test" agents) capable of modulating the the activity of granzyme B in a sample. "Modulation" refers to the capacity to either enhance or inhibit a functional property of biological activity or process (e.g., enzyme activity). Such enhancement or inhibition may be contingent on the occurrence of a specific event, such as activation of a signal transduction pathway, and/or may be manifest only in particular cell types. A "modulator" refers to an agent (naturally occurring or non-naturally occurring), such as, for example, a biological macromolecule (e.g., nucleic acid, protein, non-peptide, or organic molecule), small molecules, an extract made from biological materials such as bacteria, plants, fungi, or animal (particularly mammalian) cells or tissues, or any other agent. Modulators are evaluated for potential activity as inhibitors or activators (directly or indirectly) of a biological process or processes (e.g., agonist, partial antagonist, partial agonist, or antagonist) by inclusion in the screening assays described herein. The activities (or activity) of a modulator may be known, unknown or partially known. Such modulators can be screened using the methods of the invention. The term "test agent" or "test compound" refers to an agent or compound to be tested by one or more screening method(s) of the invention as a putative modulator. Usually, various predetermined concentrations are used for screening such as 0.01 µM, 0.1 µM, 1.0 µM, and 10.0 µM. Controls can include the measurement of a signal in the absence of the test agent or compound, comparison to an agent or compound known to modulate the target, or comparison to a sample (e.g., a cell, tissue, or organism) before, during, and/or after contacting with the test agent or compound.

Accordingly, a screening system useful for identifying agents or compounds which modulate the cleavage of recognition sequence present in a modified is provided circularly-permuted thermostable luciferase biosensor of the invention and detecting its activity is provided. This allows one to rapidly screen for granzyme B activity modulators. Utilization of the screening system described herein provides a sensitive and rapid means to identify agents or compounds which modulate (e.g., inhibit or activate) granzyme B. The modified circularly-permuted thermostable luciferase biosensors include an amino acid sequence that is a cleavage site for granzyme B Thus, the insertion comprises a peptide containing a cleavage recognition sequence for granzyme B. A cleavage recognition sequence for a granzyme B is a specific amino acid sequence recognized by granzyme B during proteolytic cleavage. Accordingly, the invention provides methods to determine the amount of granzyme B in a sample by contacting the sample with a modified circularly-permuted thermostable luciferase biosensor of the invention and measuring changes in luciferase activity. The modified circularly-permuted thermostable luciferase biosensor of the invention can be used for, among other things, monitoring the activity of a granzyme B granzyme B inside a cell or an animal that expresses the modified circularly-permuted thermostable luciferase biosensor.

The assays of the invention can be used to screen drugs to identify agents or compounds that alter the activity of granzyme B. In some embodiments, the assay is performed on a sample *in vitro* containing granzyme B. A sample containing a known amount of granzyme B is mixed with a modified circularly-permuted thermostable luciferase biosensor of the invention and with a test agent. The amount of the granzyme B activity in the sample is then determined as described above. The amount of activity per mole of granzyme B in the presence of the test agent is compared with the activity per mole of granzyme B in the absence of the test agent. A difference indicates that the test agent alters the activity of the protease. Accordingly, the alterations may be an increase in granzyme B activity resulting in a decrease in modified circularly-permuted thermostable luciferase biosensor activity or a decrease in granzyme B activity corresponding to an increase or maintenance of modified circularly-permuted thermostable luciferase biosensor activity.

In some embodiments, the ability of an agent to alter granzyme B activity is determined. In this assay, cells are conditioned or contacted with an agent or compound suspected of modulating protease activity. The cell or cells in the culture are lysed and granzyme B activity measured. For example, a lysed cell sample containing a known or unknown amount of granzyme B is mixed with a modified circularly-permuted thermostable luciferase biosensor of the invention. The amount of the granzyme B activity in the sample is then determined as above by determining the degree of modified circularly-permuted thermostable luciferase biosensor activity in a control or non-treated sample and the treated lysed cellular sample. The activity or inhibition can be calculated based on a per microgram or milligram protein in the sample. Accordingly, the modulation in granzyme B activity includes an increase in protease activity resulting in a decrease in modified circularly-permuted thermostable luciferase biosensor activity or a decrease in granzyme B activity corresponding to an increase or maintenance of modified circularly-permuted thermostable luciferase biosensor activity. Typically, the difference is calibrated against standard measurements to yield an absolute amount of granzyme B activity. A test agent that inhibits or blocks the activity or expression of granzyme B can be detected by increased modified circularly-permuted thermostable luciferase biosensor activity in treated cells compared to untreated controls.

In an *in vivo* assay, cells transfected, either transiently or stably, with an expression vector encoding a modified circularly-permuted thermostable luciferase biosensor are exposed to different amounts of the test agent or test compound, and the effect of the test agent or test compound on luciferase activity in a cell can be determined. Typically, the difference is calibrated against standard measurements to yield an absolute amount of granzyme B activity. A test agent that inhibits or blocks the activity or expression of the can be detected by increased modified circularly-permuted thermostable luciferase biosensor activity in treated cells compared to untreated controls.

In a whole animal assay, an animal, e.g., mouse, may be injected with cells that express a modified circularly-permuted thermostable luciferase biosensor, and the animal exposed to different amounts of a test agent or test compound. Cells that express a modified circularly-permuted thermostable luciferase biosensor of the invention may be implanted in an animal. The substrate for the modified circularly-permuted thermostable luciferase may be injected into the animal or into the cells of the animal. The effect of the test agent or test compound on luciferase activity in the animal can then be determined.

The disclosure also provides a method of immobilizing the modified circularly-permuted thermostable luciferase biosensor to a solid support, e.g., a particle, resin, column, solid surface (e.g., plate, slide, or well bottom), etc. The immobilized biosensor can then be used to detect the presence or activity of granzyme B. In some embodiments, the modified circularly-permuted thermostable luciferase biosensor of the invention, either in purified form or expressed in cell lysate, e.g., *E. coli* cell lysate, can be immobilized onto a solid support, e.g., resin or solid surface, and granzyme B detected. The granzyme B can be in a purified form or also be expressed in a cell lysate. Detectable activity of the modified circularly-permuted thermostable luciferase biosensor is then determined.

In some aspects, the disclosure provides compositions and methods for a non-radioactive assay for cell-mediated cytotoxicity. The disclosure provides compositions and methods to detect or determine cytotoxicity mediated by cytotoxic lymphocytes, e.g., cytotoxic T lymphocytes (CTLs) and cytokine-activated natural killer (NK) cells. The disclosure also provides novel granzyme B recognition sequences and bioluminescent biosensors for detecting granzyme B activity. The disclosure further includes methods of detecting granzyme B activity in cell-based assays using the bioluminescent biosensors described herein.

In some embodiments, the method comprises transfecting the bioluminescent protease biosensor of the invention into a target cell, co-incubating the transfected target cells with a cytotoxic lymphocyte, e.g., a NK cell, and detecting luminescence. If granzyme B is successfully transferred to the transfected target cell, it will cleave the biosensor via the recognition sequence, and luminescence will be generated. Examples of a target cell include a tumor cell, such as K562 cell, EL4 cell, or Jurkat cell, virus-infected cells, cells infected with intracellular bacterial or protozoal parasites, and allografts, such as transplanted kidney, heart, lungs,

In other embodiments, the method of the present invention can be used to test patient or patient-derived CTLs or NK cells to determine their cytotoxic function. In some embodiments, the method comprises transfecting the bioluminescent protease biosensor of the invention into a target cell, e.g., a tumor cell, co-incubating the transfected target cells with the patient or patient-derived CTLs or NK cells, and detecting luminescence. In some embodiments, the patient or patient-derived CTLs or NK cells have little or unknown cytotoxic function.

In other embodiments, the method of the present invention can be used to screen for inhibitors, e.g., siRNAs, or enhancers, e.g., small molecules, of the cytotoxic function of a cell. In some embodiments, the screening for inhibitors or enhancers can be done in a multi-well format, e.g., 96-, 384-, or 1536-wells.

Disclosed herein are novel granzyme B recognition sequence GRIEADSE (SEQ ID NO:80) and KSVGPDFG (SEQ ID NO:81).

The disclosure also provides protease biosensors for the detection of cell-mediated cytotoxicity. The protease biosensors, as described in U.S. Patent Application Serial No. 13/105,648, encode a biosensor comprising a circularly-permuted, thermostable luciferase and a peptide linker. The peptide linker comprises a sensor region capable of interacting with a molecule of interest.

The disclosure also provides methods for detecting granzyme B activity in cellular processes, e.g., beta-arrestin localization. In some embodiments, the granzyme B biosensor of the present invention would be used to determine the granzyme B activity in a cellular process.

The materials and composition for use in the assay of the invention are ideally suited for the preparation of a kit. Such a kit may comprise a carrier means containing one or more container means such as vials, tubes, and the like, each of the containers means comprising one of the separate elements to be used in the method. One of the containers comprises a modified circularly-permuted thermostable luciferase biosensor or polynucleotide (e.g., in the form of a vector) of the invention. A second container may contain a substrate for the modified circularly-permuted thermostable luciferase biosensor.

### EXAMPLES

### Example I: Generation of a Modified Thermostable Luciferase Biosensor with Increased Responsivity in Cells

The Caspase-3 BioSensor (CBS) is a thermostable *Photuris pennsylvanica* luciferase (TL), circularly-permuted (CP) at amino acid 358, with a caspase-3 recognition site, i.e., one containing the caspase-3 recognition site comprising amino acids DEVD, at the junction between the TL fragments. The specific CBS that was used as the starting template is termed TL-CP358-DEVD:DD. The amino acid sequence of this CBS can be represented as: M / *TL residues 358-544* / *S**DEVD**GSL* / *TL residues 4-354* / *V* (SEQ ID NO:6). The amino acid positions in the CP TL correlate to those of the non-CP TL sequence (provided in the attached appendix). Upon treatment with caspase-3, CBS is cleaved at the recognition site allowing the two TL fragments to form a more favorable, higher activity, conformation.

The utility of CBS is the differential activity before and after cleavage by caspase-3. The basal activity of CBS is defined as the activity at assay time (0), before caspase-3 has had time to cleave at the recognition site. The induced activity is defined as the activity at some later time (t) after CBS has been cleaved by caspase-3. The response or fold increase in activity, is the ratio of induced to basal activity. Substitutions in TL-CP358-DEVD:DD were generated to develop CBS variants with enhanced responsivity to induction using the error-prone, mutagenic PCR-based system GeneMorph II (Stratagene; Daugherty, PNAS USA 97(5):2029 (2000)), according to manufacturer's instructions.

The resulting library was expressed in *E. coli* and screened for luciferase activity with and without pre-treatment with recombinant caspase-3 (data not shown). CBS variants having the best signal and response characteristics were then evaluated in HEK293 cells by kinetic assay measuring the response to TNF-α-related apoptosis inducing ligand (TRAIL) treatment (Wiley, S.R. et al., Immunity 3:673 (1995); Niles, A.L. et al., Meth. Mol. Biol. 414:137 (2008)). TRAIL induces apoptosis via activation of the death receptor to form active caspase-8, which in turn activates procaspase 3 to produce caspase-3. The appearance of active caspase-3 should be accompanied by an increase in luminescence as the CBS variants are cleaved and activated. Briefly, HEK293 cells, plated at 15,000 cells/well in a 96-well plate, were transiently transfected using TransIT-LTI (Mirus Bio) with plasmid DNAs encoding various CBS variants with amino acid substitutions in TL-CP358-DEVD:DD. The same plasmids also carried a gene for constitutive expression of *Renilla* luciferase to act as a transfection control. Cells were pretreated with 2 mM luciferin for 2 hrs at 37°C. Cells were treated with 1 ug/mL TRAIL and assayed for 10 hrs at 37°C. Luminescence was monitored continuously over time (Luminometer: Varioskan Flash (Thermo) 1 sec integration time). Cells in replicate wells were lysed, at the time of TRAIL addition, i.e., time (0) and *Renilla* luciferase activity was measured. All biosensor data was then normalized for transfection efficiency using *Renilla* luciferase luminescence (Dual-GloAssay System; Promega Corporation).

Exemplary CBS variants include, but are not limited to those listed in Table 1. Table 1 lists the variants of TL-CP358-DEVD:DD, identified by clone name, showing improved response to TRAIL treatment. Improvements listed in Table 1 are normalized to the parental TL-CP358-DEVD:DD CBS. "BASAL" represents the normalized biosensor luminescence at TRAIL addition, i.e., time (0), "INDUCED" represents the normalized biosensor luminescence at roughly 10 hrs after TRAIL addition, and "RESPONSE" represents the fold-induction, i.e., the ratio of INDUCED to BASAL activity.

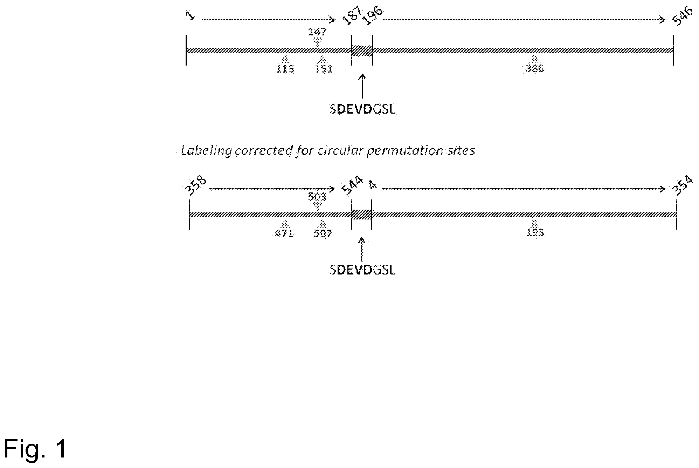

Standard sequencing techniques known in the art were used to identify the amino acid substitution in each clone (see Table 1). The amino acid position is based on parental TL, e.g., Pro at position 2 of the variants = TL 358; the residues to the N-terminus of the DEVD therefore represent TL residues 358-544(Gly); the residues to the C-terminus of the DEVD represent TL residues 4(Lys)-354(Gly) (See FIG. 1 for examples). Each amino acid substitution is indicated by the position corresponding to the amino acid position in the parental TL, not the TL-CP358-DEVD:DD sequence, whereby the first letter following the numerical position represents the corresponding amino acid in parental TL. If the amino acid is substituted with another amino acid, the second letter represents the amino acid substitution. If the amino acid is substituted with a stop codon, the substitution is indicated by "STOP."

**Table 1: Summary of the fold improvement in responsivity of CBS variants over the corresponding TL-CP358-DEVD:DD**

| | **IMPROVEMENT OVER TL-CP358-DEVD:DD** | | | | | | | | |
|---|---|---|---|---|---|---|---|---|---|
| **CLONE** | **BASAL** | **INDUCED** | **RESPONSE** | **mut#1** | **mut#2** | **mut#3** | **mut#4** | **mut#5** | **mut#6** |
| 01:E-12 | 0.35 | 0.71 | 2.04 | 021AD | | | | | |
| 01:G-12 | 0.00 | 0.00 | 2.11 | 044AD | | | | | |
| 01:G-03 | 0.47 | 0.59 | 1.26 | 128SP | | | | | |
| 01:E-11 | 0.4 | 0.6 | 1.3 | 193SP | | | | | |
| 09:A-11 | 0.69 | 1.17 | 1.69 | 273LQ | | | | | |
| 01:H-06 | 0.22 | 0.39 | 1.77 | 275SP | | | | | |
| 13:F-03 | 0.30 | 1.02 | 3.41 | 503DG | | | | | |
| 03:E-12 | 0.44 | 0.50 | 1.16 | 286LH | | | | | |
| 01:C-04 | 0.15 | 0.65 | 4.20 | 294LH | | | | | |
| 05:C-07 | 0.16 | 0.20 | 1.30 | 294LP | | | | | |
| 14:A-04 | 0.34 | 0.95 | 2.77 | 297SG | | | | | |
| 12:B-10 | 1.15 | 1.34 | 1.16 | 297SI | | | | | |
| 13:B-03 | 0.74 | 1.45 | 1.95 | 329RW | | | | | |
| 14:F-06 | 0.20 | 1.12 | 5.74 | 409AV | | | | | |
| 05:C-02 | 0.19 | 0.38 | 2.02 | 461PL | | | | | |
| 06:A-12 | 0.04 | 0.14 | 3.26 | 465DV | | | | | |
| 01:D-02 | 0.1 | 0.6 | 5.3 | 471IT | | | | | |
| 14:G-12 | 0.11 | 0.30 | 2.69 | 482AS | | | | | |
| 06:G-09 | 0.55 | 1.30 | 2.69 | 482AV | | | | | |
| 07:C-06 | 0.16 | 0.61 | 3.79 | 485VE | | | | | |
| 05:B-06 | 0.05 | 0.20 | 4.06 | 497VA | | | | | |
| 07:B-02 | 0.5 | 1.0 | 1.8 | 503SG | | | | | |
| 01:A-05 | 0.2 | 0.6 | 3.6 | 507TI | | | | | |
| 05:D-11 | 0.04 | 0.16 | 3.55 | 522PS | | | | | |
| 02:B-09 | 0.25 | 0.55 | 2.39 | 526TS | | | | | |
| 06:D-04 | 0.18 | 0.45 | 2.42 | 530DV | | | | | |
| 01:F-02 | 0.58 | 0.67 | 1.15 | 543NS | | | | | |
| 05:E-06 | 0.12 | 0.41 | 3.51 | 017EG | 513GA | | | | |
| 09:D-10 | 0.19 | 0.43 | 2.21 | 026FY | 530DG | | | | |
| 13:B-04 | 0.51 | 1.26 | 2.48 | 051LS | 193SP | | | | |
| 01:H-08 | 0.34 | 0.67 | 1.96 | 110EV | 304DA | | | | |
| 11:A-11 | 0.71 | 1.16 | 1.63 | 115HY | 5211V | | | | |
| 05:G-10 | 0.43 | 0.82 | 1.92 | 123RH | 303YD | | | | |
| 04:A-05 | 0.21 | 0.45 | 2.20 | 128SP | 523KQ | | | | |
| 12:D-08 | 330.89 | 764.74 | 2.31 | 192AT | 389LS | | | | |
| 05:D-06 | 0.55 | 1.03 | 1.87 | 193SP | 1141K | | | | |
| 15:E-10 | 0.06 | 0.17 | 2.73 | 196FY | 530DG | | | | |
| 11F-07 | 0.23 | 0.69 | 2.96 | 208MK | 466AG | | | | |
| 04:H-08 | 0.06 | 0.17 | 3.03 | 226FY | 509KT | | | | |
| 07:C-02 | 0.29 | 0.78 | 2.66 | 264MT | 303YN | | | | |
| 01:E-04 | 0.22 | 0.57 | 2.63 | 294LH | 304DE | | | | |
| 13:C-09 | 0.55 | 1.36 | 2.46 | 294LH | 308LI | | | | |
| 04:H-07 | 0.14 | 0.25 | 1.80 | 294LP | 510WR | | | | |
| 14:G-10 | 0.23 | 0.50 | 2.17 | 302KE | 530DV | | | | |
| 01:B-08 | 0.07 | 0.15 | 2.20 | 308LS | 343E(STOP) | | | | |
| 13:G-12 | 0.37 | 1.06 | 2.89 | 309KN | 331KI | | | | |
| 16:D-12 | 0.2 | 0.6 | 2.4 | 329RQ | 530DV | | | | |
| 05:A-07 | 0.29 | 0.61 | 2.10 | 353K(STOP) | 530DA | | | | |
| 09:E-03 | 0.54 | 1.26 | 2.33 | 364IM | 530DA | | | | |
| 18:C-05 | 0.07 | 0.29 | 4.24 | 374DY | 431FL | | | | |
| 13:A-05 | 0.09 | 0.18 | 2.67 | 374DY | 431FS | | | | |
| 08:A-12 | 0.24 | 0.75 | 3.13 | 385EG | 465DG | | | | |
| 01:C-11 | 0.01 | 0.10 | 8.50 | 420GR | 489GR | | | | |
| 04:A-08 | 0.01 | 0.03 | 3.92 | 468VI | 484VL | | | | |
| 04:G-10 | 0.17 | 0.53 | 3.14 | 484VI | 516KN | | | | |
| 04:D-01 | 0.66 | 0.79 | 1.21 | 543NS | 494EV | | | | |
| 04:E-11 | 0.14 | 0.42 | 3.07 | 081SC | 374DV | 517FC | | | |
| 01:H-11 | 0.03 | 0.07 | 2.15 | 101VA | 286LP | 364IL | | | |
| 05:C-06 | 0.21 | 0.41 | 2.01 | 119IN | 294LH | 542TP | | | |
| 04:B-09 | 0.10 | 0.16 | 1.66 | 128SP | 211HL | 287VA | | | |
| 11:D-05 | 0.20 | 0.46 | 2.25 | 137NY | 493NY | 507TI | | | |
| 10:D-04 | 0.17 | 0.41 | 2.33 | 196FY | 228NT | 530DG | | | |
| 11:B-03 | 0.04 | 0.19 | 4.57 | 208ML | 230IT | 273LP | | | |
| 07:B-04 | 0.24 | 0.61 | 2.54 | 295AV | 449AT | 537ML | | | |
| 04:C-03 | 0.2 | 0.7 | 2.9 | 523KI | 533VA | 536QR | | | |
| 10:D-11 | 0.51 | 1.31 | 2.58 | 005ND | 133QL | 228NT | 294LH | | |
| 15:D-11 | 0.27 | 1.00 | 3.64 | 021AS | 426DN | 428DG | 526TS | | |
| 11:A-06 | 0.23 | 0.85 | 3.73 | 039IT | 2141V | 348VA | 507TI | | |
| 14:H-06 | 0.16 | 0.69 | 4.24 | 186NI | 233TM | 427ND | 465DG | | |
| 05:F-11 | 0.94 | 1.50 | 1.58 | 103PS | 191VA | 306SP | 313ST | 473DV | |
| 11:B-10 | 0.53 | 1.19 | 2.25 | 126FC | 466AV | 471IM | 536QR | 543NK | |
| 10:D-03 | 0.34 | 1.06 | 3.09 | 023EG | 228NT | 309KE | 324EG | 4561V | 460HL |

### Example II: Evaluation of Specific Combinations of Mutations in Thermostable Luciferase Caspase-3 Biosensors

Additional CBS variants were generated using the oligo-based site-directed mutagenesis kit Quik Change (Stratagene; Kunkel, PNAS USA 82(2):488 (1985)), according to the manufacturer's instructions. The amino acid substitutions identified in those variants from Example I with the most improved response, specifically clones 12:B-10, 01 :A-05, 04:C-03, 01 :E-11, 16:D-12, 01 :D-02 and 07:B-02, were combined and evaluated in HEK293 cells as in Example I. The amino acid substitutions used to generate the additional CBS variants were 193SP, 297SI, 329RQ, 471IT, 503SG, 507TI, 523KI, 533VA, and 536QR corresponding to SEQ ID NO:2. Exemplary CBS variants include, but are not limited to, those listed in Table 2. Table 2 identifies the clone ("NEW #"), the amino acid substitutions found in the clone indicated by an X in the column which indicate the amino acid substitution, 193SP, 297SI, 329RQ, 471IT, 503SG, 507TI, 523KI, 533VA, and 536QR, the improvement in Basal, Induced and Response over the corresponding starting TL-CP358-DEVD:DD.

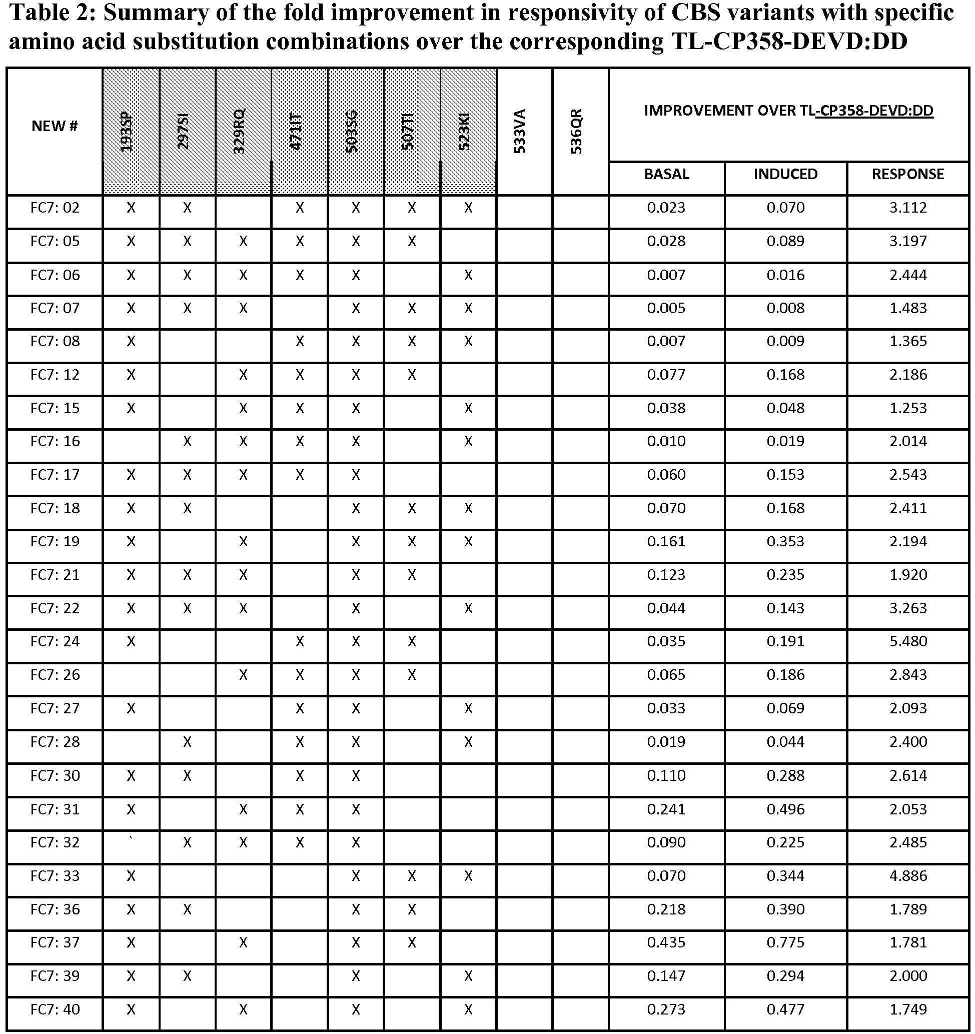

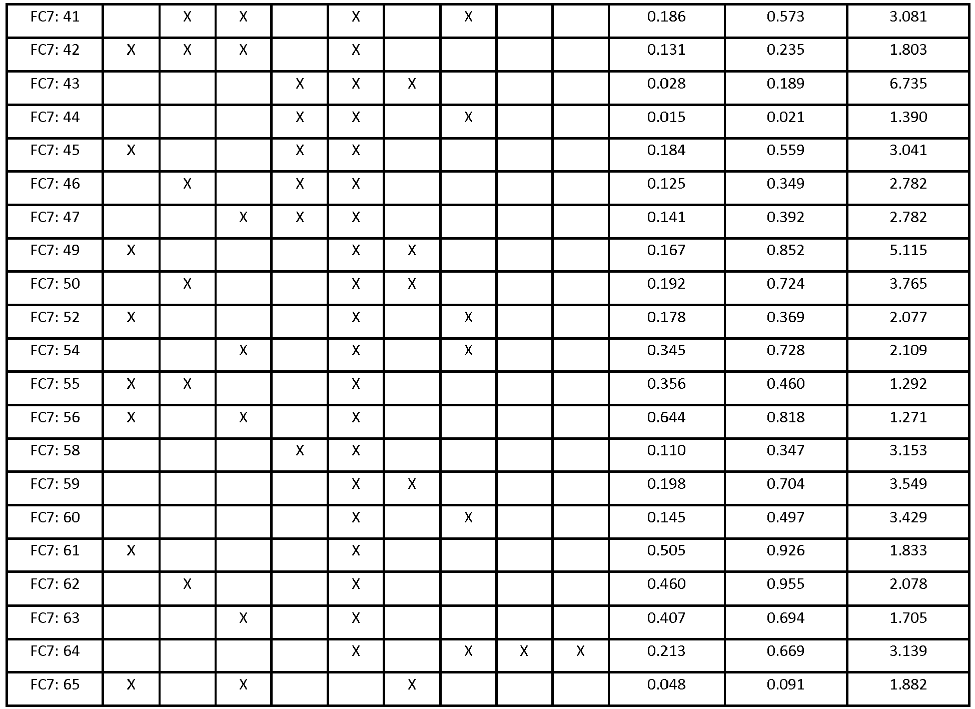

Many of the combinations of substitutions tested demonstrated increased responsivity as compared to the parental TL-CP358-DEVD:DD biosensor or the variants disclosed in Table 1. Four CBS variants, namely 01 :A-05, FC7:24, FC7:43 and FC7:49, were of particular interest, (see FIG. 1 and Table 3). FIG. 1 shows the position of the four amino acid substitutions, I471T, S503G, T507I, and S193P, incorporated into these variants in the parental TL-CP358-DEVD:DD sequence and the positions corrected for the circular permutation sites (see also Table 3). The top cartoon in FIG. 1 indicates the location of the substitutions based on sequential numbering of the primary amino acid sequence. The bottom cartoon in FIG. 1 indicates the codon designations based on the parental TL-CP358-DEVD:DD. The nucleotide changes are as follows: 471: ata>aca; 503: agt>ggt; 507: aca>ata; 193: tcg>ccg.

**Table 3: Summary of amino acid substitutions found in clones 01:1-05, FC7:24, FC7:43 and FC7:43**

| **Clone** | **Substitution(s)** | | | |
|---|---|---|---|---|
| 01:A-05 | T507I | | | |
| FC7:24 | I471T | S503G | T507I | S193P |
| FC7:43 | I471T | S503G | T507I | |
| FC7:49 | S503G | T507I | S193P | |

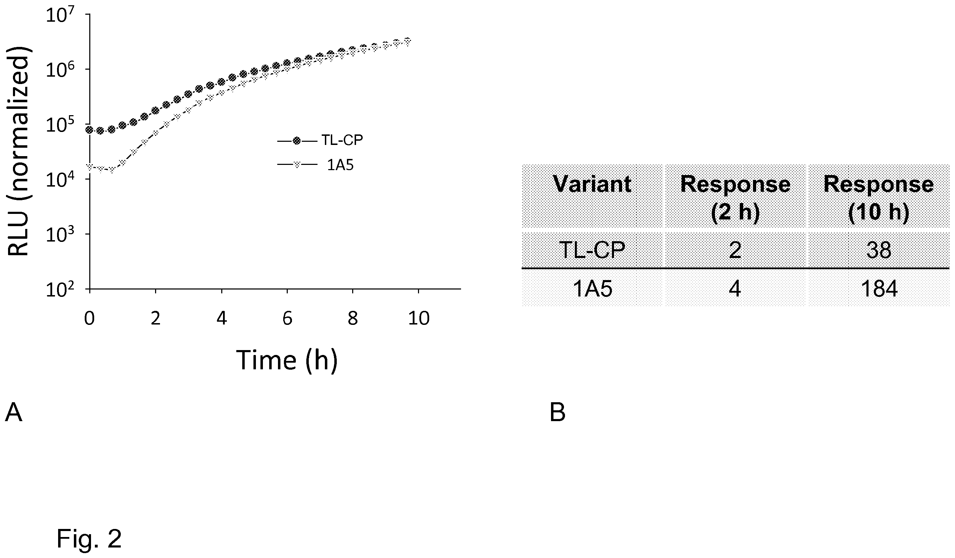

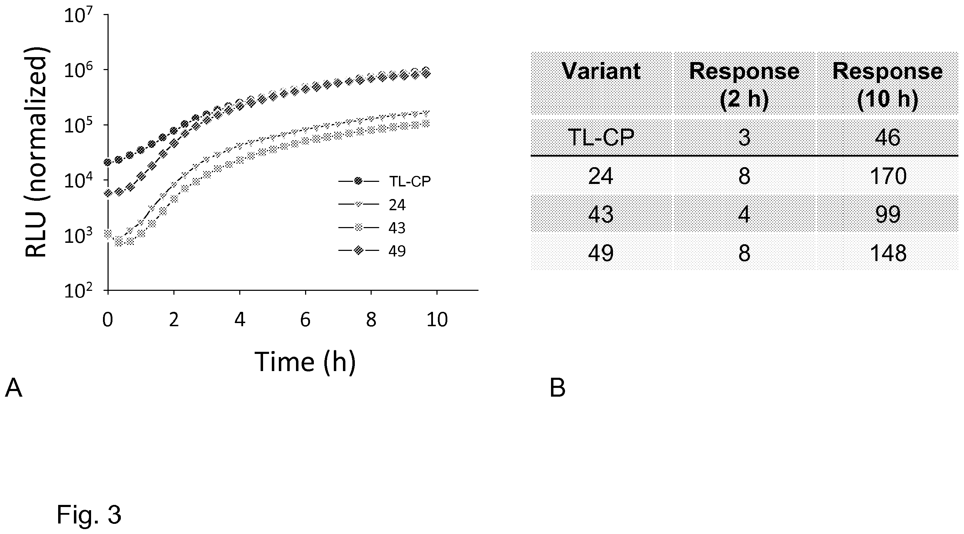

The response to TRAIL in live cells in the improved CBS variants 01 :A-05, FC7:24, FC7:43 and FC7:49 ("1A5", "24", "43", and "49", respectively) was compared to the parental TL-CP358-DEVD:DD ("TL-CP") in FIGS. 2A-B and 3A-B over a 10 hr time period. Variant 01 :A-05 had 2 times and about 4.8 times greater RESPONSE after 2 and 10 hrs TRAIL treatment, respectively, compared with TL-CP358-DEVD:DD (FIGS. 2A and 2B). After 2 hrs, variants FC7:24 and FC7:49 had about 2 times greater response than TL-CP358-DEVD:DD and variant 43 (FIGS. 3A and 3B). After 10 hrs, variants FC7:24 and FC7:49 had about 3.2-3.7 times greater response than TL-CP358-DEVD:DD (FIGS. 3A and 3B), while variant FC7:43 had about 2.2 times greater response. These data demonstrates that CBS biosensors can be generated to have improved response by incorporating one or more of these four amino acid substitutions, I471T, S503G, T507I, and S193P.

### Example III

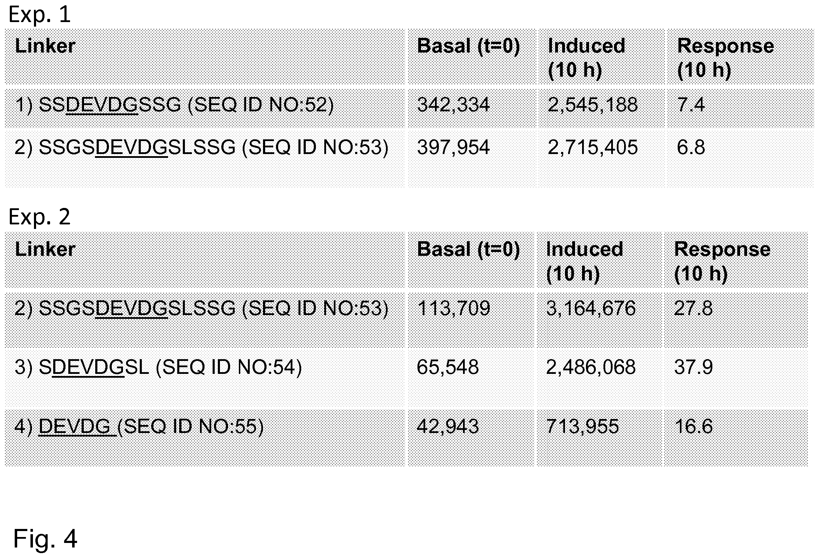

Additional CBS variants were generated to have different linker sequences, such as SSDEVDGSSG (SEQ ID NO:52), SSGSDEVDGSLSSG (SEQ ID NO:53), SDEVDGSL (SEQ ID NO:54), or DEVDG (SEQ ID NO:55). The CBS variants were evaluated in HEK293 cells as in Example I. Exemplary CBS variants include, but are not limited to, those listed in FIG. 4. All biosensor data was then normalized for transfection efficiency using *Renilla* luciferase luminescence as in Example I. FIG. 4 identifies the clone by the linker sequence it contains ("Linker") and shows the luminescence in RLUs at TRAIL addition, i.e., time (0), ("Basal (t=0)"), at roughly 10 hrs after TRAIL addition ("Induced (10 h)") and the fold-induction, i.e., the ration of Induced to Basal Activity ("Response (10 h)"). The common linker clone between the two experiments is #2 (i.e., SSGSDEVDGSLSSG). The difference in the numbers is typical variation between experiments. Linker #3 is the same linker found in the clone referred to as "TL-CP358-DEVD:DD."

### Example IV: Evaluation of the Mutant Thermostable Luciferase Biosensors to Detect Caspase-8

To evaluate whether the mutant thermostable luciferase biosensors can be used to detect Caspase-8 activity in cells, biosensors were generated that contained the Caspase-8 cleavage site, LETDG (SEQ ID NO:15). Two different biosensors, TL-CP358-Caspase-8 and TL-CP233-Caspase-8 were used. As controls, the firefly (*Photinus pyralis*; Ppy) luciferase biosensors FF-CP234-Caspase-8 (M/Ppy residues 234-544/LETDG /Ppy residues 4-230/V), FF-CP359-Caspase-8 (M/Ppy residues 359-544/LETDG /Ppy residues 4-355/V), and TL-CP358-DEVD. Table 4 provides sequence details of the biosensors.

**Table 4**

| **Construct** | **Caspase Cleavage site with linker** | **Luciferase** | **Fragments** |
|---|---|---|---|
| TL-CP358-Caspase 8 (SEQ ID NOs:59 and 60) | GSSLETDSSG (SEQ ID NO:76) | TL Ppe | 358-543 and 4-354 |
| TL-CP233-Caspase 8 (SEQ ID NOs:57 and 58 ) | GSSLETDSSG (SEQ ID NO:76) | TL Ppe | 233-543 and 4-232 |
| FF-CP234-caspase-8 (SEQ ID NOs:21 and 22) | GSSLETDSSG (SEQ ID NO:76) | Ppy | 234-544 and 4-233 |
| FF-CP359-caspase-8 (SEQ ID NOs:19 and 20) | GSSLETDSSG (SEQ ID NO:76) | Ppy | 359-544 and 4-355 |
| TL-CP358-DEVD (SEQ ID NOs:5 and 6) | GSSDEVDSSG (SEQ ID NO:77) | TL Ppe | 358-543 and 4-354 |

All biosensors were transfected into HeLa cells. Cells were plated at a (10,000/well) into a 96-well tissue culture plate. Biosensor DNA was prepared for transfection into the cells as described in Table 4. Thirty 10 µL reactions were set up for each biosensor. TransIT^{®} LTI (LTI; Mirus) transfection master mix was prepared by mixing 1650 µL DMEM with 49.5 µL LTI. The master mix was incubated for 15 min at room temperature. 300 µL of the master mix was then added to each biosensor DNA (enough for 30 reactions; 0.1 µg/reaction) and incubated for another 15 min at room temperature (Table 5). 10 µL of the biosensor DNA-transfection master mix solution was added to the cells in the appropriate wells. The cells were then incubated overnight at 37°C, 5% CO₂.

**Table 5**

| | **FF-CP234-caspase-8** | **FF-CP359-caspase-8** | **TL-CP233-caspase-8** | **TL-CP358-caspase-8** |
|---|---|---|---|---|
| concentration DNA | 0.309 | 0.363 | 0.327 | 0.348 |
| amount for 30 reactions (0.1 µg/well) | 9.71 | 8.26 | 9.17 | 8.62 |
| volume of DMEM per 30 reactions (10 µL) | 300 | 300 | 300 | 300 |
| µL of Mirus LT1 per 30 reactions | 9 | 9 | 9 | 9 |

After overnight incubation, the media was removed from the cells and replaced with CO₂ Independent Media (Invitrogen Cat. No. 18045088) with 2 mM Luciferin EF (Promega Cat. No. E6551). Cells were pre-equilibrated with Luciferin EF for 2 hrs in a Varioskan luminometer with bioluminescence readings taken every 20 min. Following incubation, the cells were either induced with 1 µg/mL TRAIL in CO₂ Independent Media+10% Fetal Bovine Serum (FBS) or no compound (control; media + 10% FBS only). The cells were again incubated at 37°C in a Varioskan luminometer for 500+ min with bioluminescence measured every 20 min.

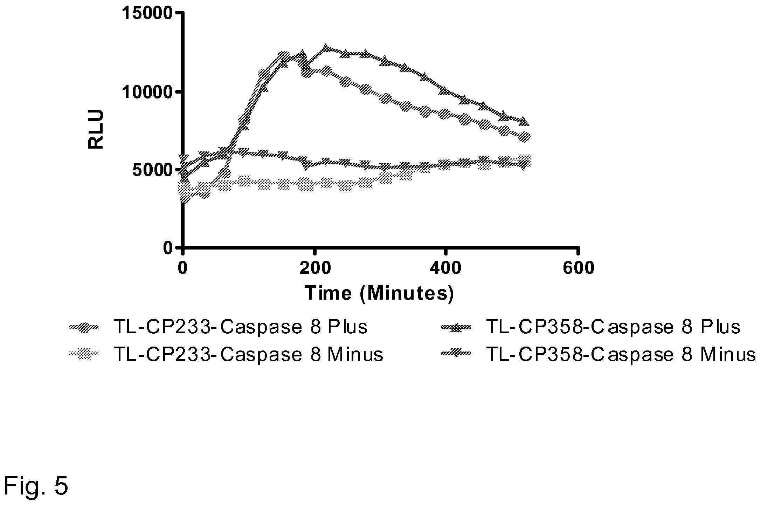

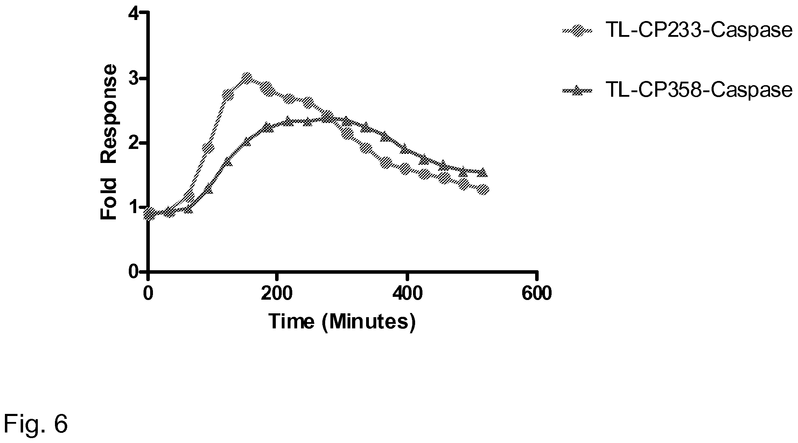

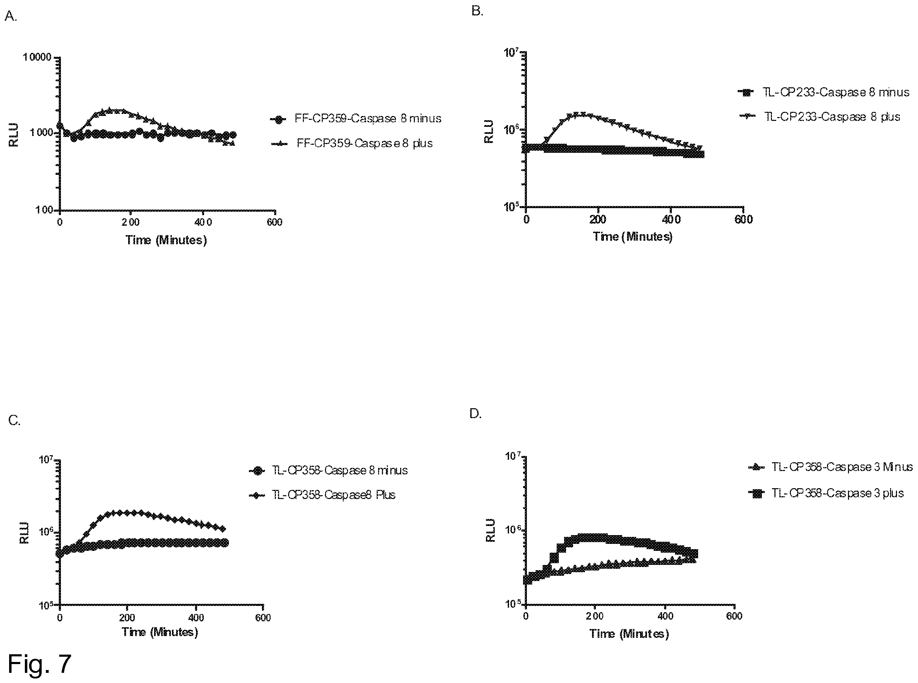

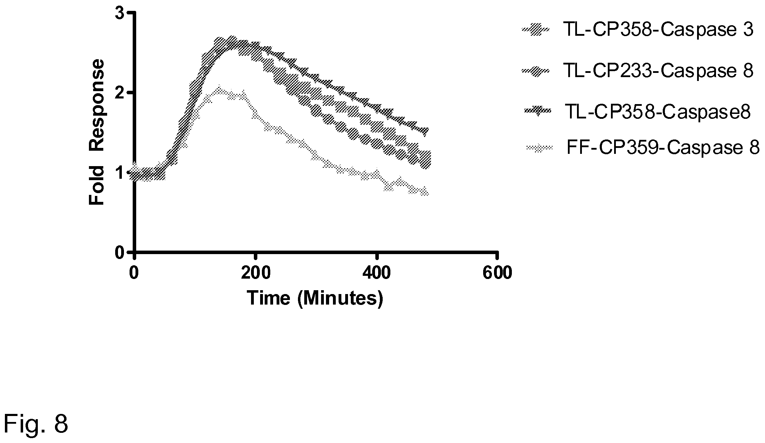

FIGS. 5-8 demonstrate at TL-CP233-Caspase 8 and TL-CP358-Caspase 8 biosensors can detect Caspase 8 activation by TRAIL. FIGS. 5 and 7 identify the kinetic profiles of Caspase 8 activation by TRAIL over time at 37°C. FIGS. 6 and 8 identify the fold response of Caspase 8 activation by TRAIL over time. Fold induction of activation was calculated by dividing the signal of samples with TRAIL over the signals without trail at a given time point. FIGS. 7 and 8 show Caspase 3 induction by Trail as measured by TL-CP358-DEVD as well as Caspase 8 induction.

### Example V: Activation of TEV Protease Mutant Thermostable Luciferase Biosensor

To evaluate whether the mutant thermostable luciferase biosensors can detect TEV protease activity in cells, the TL-CP233 biosensor, TL-CP233-TEV, containing the TEV protease cleavage site GSS-ENLYFQS-SSG (SEQ ID NO:78) was generated. TL-CP233-TEV has an amino acid sequence that can be represented as: M/TL residues 233-544/ GSS-ENLYFQS-SSG TL residues 4-233/V (SEQ ID NOs:61 and 62). As controls, the firefly (*Photinus pyralis*; Ppy) luciferase biosensors FF-CP235-TEV (M/Ppy residues 234-544/ GSS-ENLYFQS-SSG /Ppy residues 4-233/V; SEQ ID NOs:63 and 64), FF-CP269-TEV (M/Ppy residues 269-544/ GSS-ENLYFQS-SSG /Ppy residues 4-268/V; SEQ ID NOs:65 and 66), and FF-CP359-TEV (M/Ppy residues 359-544/ GSS-ENLYFQS-SSG /Ppy residues 4-355/V; SEQ ID NOs:67 and 68) were used. For all transfections, TEV protease (Genbank accession no. BFB754) constitutively expressed from a CMV promoter was transfected (pF9a - BFB754). This construct also co-expresses *Renilla* luciferase for use as a transfection efficiency control.

Each of the biosensors and TEV protease constructs were transfected in Chinese Hamster Ovary (CHO) cells. Cells were plated at (15,000 cells per well) into a 96-well tissue culture plate. The transfection solution was prepared according to Table 6. Each sensor was co-transfected with either the TEV protease or a carrier vector (pF9a-null).

**Table 6**

| **DNA** | **Amount for 60 wells of a 96-well plate (600 µL of media plus 18 µL Mirus LT1 plus DNA)** | | | | | | |
|---|---|---|---|---|---|---|---|
| **concentration** | 0.202 | 0.177 | 0.38 | 0.342 | 0.261 | 0.214 | 0.214 |
| **construct** | **pF9a-TEV protease** | **pF9a Null** | **FF-CP233-TEV** | **FF-CP268-TEV** | **FF-CP358-TEV** | **TL-CP233-TEV** | **Read Through** |
| **Amount per Tfx (µg)** | 1.2 | 1.2 | 5.8 | 5.8 | 5.8 | 5.8 | 5.8 |
| **Amount per Tfx (µL)** | 5.9 | 6.8 | 15.3 | 17.0 | 22.2 | 27.1 | 27.1 |

Cells were incubated overnight for 24 hrs at 37°C, 5% CO₂. After overnight incubation, cells were equilibrated with media and 5 mM Luciferin EF for 2 hrs. Bioluminescence was then measured at 37°C in a Varioskan luminometer. Results were normalized to *Renilla* to control for transfection efficiencies.

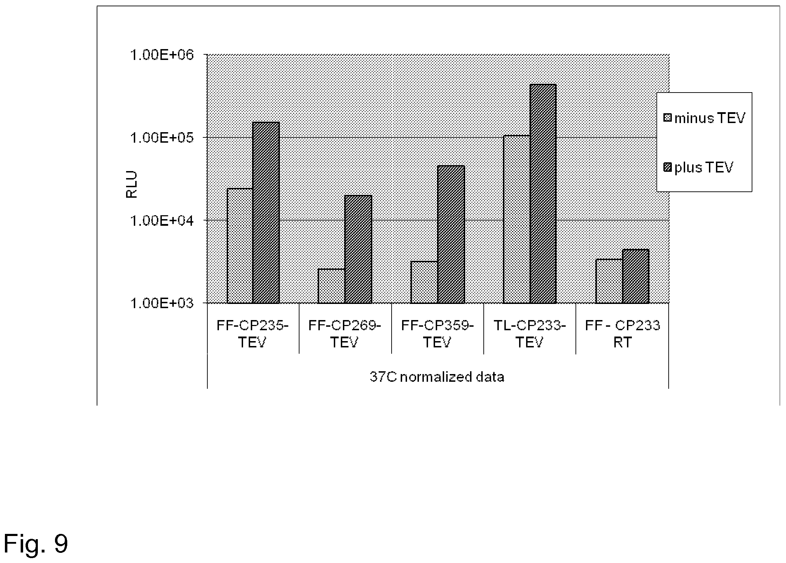

The Biosensor without the TEV recognition sequence (FF-CP233-Read through; FF-CP233-RT) is not activated by TEV protease while the other biosensors were activated by TEV protease (FIG. 9).

### Example VI: Molecular Imaging of Apoptosis in Glioma cells

To demonstrate that the mutant thermostable biosensors can be used to detect cell death in cells, the thermostable caspase 3 biosensors, TL-CP233-Caspase 3 ("233"; SEQ ID NOs:17 and 18), TL-CP358-Caspase 3 ("358V2"; SEQ ID NOs:5 and 6) and the mutant thermostable caspase 3 biosensors 1A5 ("358V3"; SEQ ID NOs:7 and 8), 24 ("358V4"; SEQ ID NOs:9 and 10), 43 ("358V5"; SEQ ID NOs:11 and 12) and 49 ("358V6"; SEQ ID NOs:13 and 14) were stably expressed in the glioma cell line D54-MG, the cells treated with TRAIL and bioluminescence measured to detect caspase 3 activity.

To derive cells stably expressing the thermostable biosensors, D54-MG cells were transfected with the biosensors. The biosensors were subcloned into pEF vector containing a neomycin resistance gene (Invitrogen) via PCR amplification and inserted into the multiple cloning site at the SalI and EcoRI restriction sites. Transfections were performed in 6-well tissue culture dishes using 3 µL Fugene 6 transfection reagent (Roche) and 1 µg plasmid DNA. Cells were placed in RPMI media (Gibco) containing 10% FBS (Gibco), Pen/Strep Glutamine (100X; Gibco) and 200 µg/mL geneticin (G418) for 48 hrs. Single clones were selected approximately 10 days after transfection using standard techniques known in the art. Briefly, the media was removed from the cells, and the cells were gently washed with PBS. Round filter papers were soaked in trypsin and placed on a single colony. The filter paper, which contained the attached cells, was removed and placed into a 24-well tissue culture dish. Each individual clone was tested approximately 2-3 weeks after selection for reporter expression by Western blotting using a luciferase antibody (Promega; Cat. No. G7451) and bioluminescence (100 µg/mL D-Luciferin reconstituted in PBS was add directly to the media and detected). Clones with similar bioluminescent activity (highest fold induction) and reporter expression (determined by Western blot) were selected for use in detecting cell death.

To detect cell death, the stable D54-MG cells were seeded at 10,000 cells/well into a 96-well assay plate and allowed to incubate for 24 hrs at 37°C, 5% CO₂. After overnight incubation, the cells were treated with 200ng/mL TRAIL and 100 µg/mL D-luciferin (Promega). Live cell bioluminescence was imaged at 2, 4, and 6 hrs. Photon counts were taken at the different time points pre- and post-TRAIL treatment using the Envision luminometer (Perkin Elmer). Reporter expression and TRAIL-induced apoptosis was further detected by Western blotting against luciferase and Caspase-3.

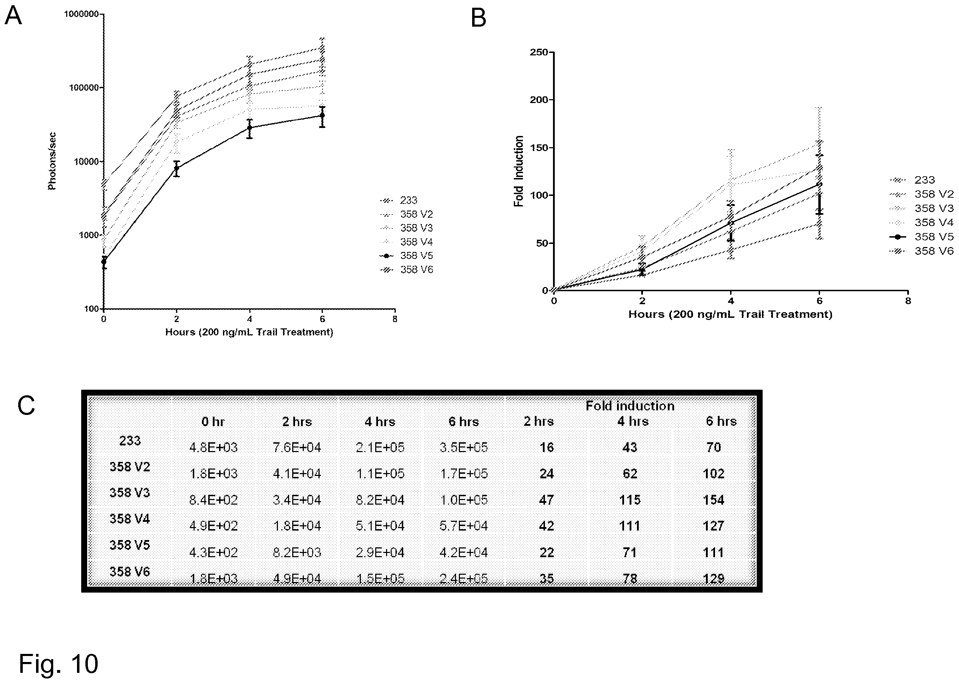

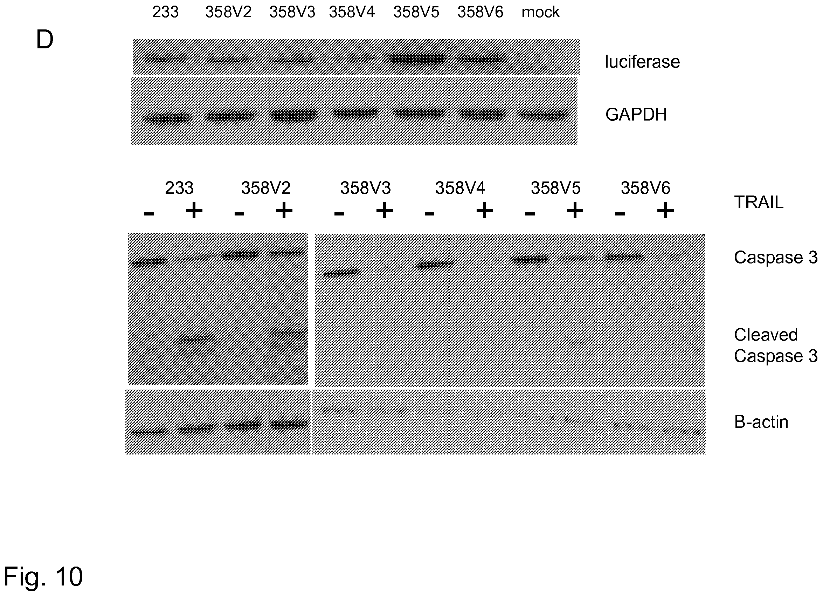

FIG. 10 demonstrates that upon treatment with TRAIL, D54-MG cells stably expressing the various thermostable biosensors resulted in a 100-200 fold induction in bioluminescent activity. D54-MG cells expressing different versions of the thermostable biosensors were untreated or treated with 200 ng/mL TRAIL and imaged at indicated time points; photon counts/sec were recorded at the indicated time points (FIG. 10A). The fold induction of D54-MG cells expressing different versions of the thermostable biosensors untreated or treated with 200 ng/mL TRAIL were calculated by normalizing the values (photons/sec) to baseline (time 0 hr) (FIG. 10B). The average photon counts/sec at baseline, 2 hrs, 4 hrs and 6 hrs post treatment in addition to fold changes achieved with different biosensor versions are depicted in FIG. 10C. FIG. 10D shows the detection of reporter expression and TRAIL induced apoptosis by Western blotting against luciferase and Caspase-3.

### Example VII: Mutant thermostable biosensors use

To demonstrate the use of the mutant thermostable biosensors to detect cell death *in vivo*, D54-MG cells stably expressing either TL-CP233-Caspase 3 ("233"; SEQ ID NOs: 17 and 18), TL-CP358-Caspase 3 ("358V2"; SEQ ID NOs:5 and 6), 1A5 ("358V3"; "3-S"; SEQ ID NOs:7 and 8), 43 ("358V5"; "5-R"; SEQ ID NOs:11 and 12) or 49 ("358V6"; "6-A"; SEQ ID NOs:13 and 14) were implanted into nude mice.

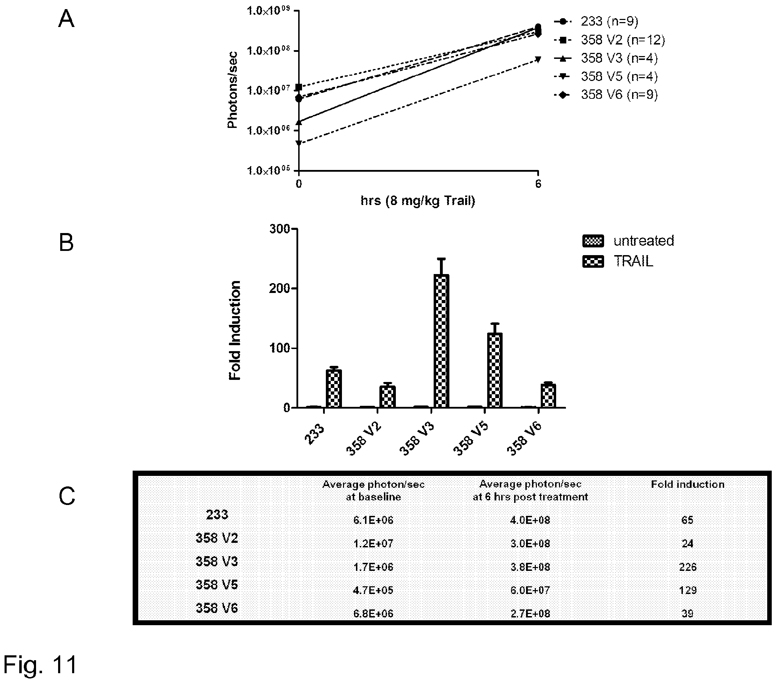

To establish a flank xenograft mouse model, 2 × 10⁶ D54-MG cells stably expressing one of the biosensors listed above (as described in Example VI) were implanted subcutaneously into nude mice. Treatment with 8 mg/kg TRAIL started when tumors reached ~100 mm³ as assayed by electronic digital caliper measurement. For *in vivo* bioluminescence detection, mice were anesthetized using 2% isofluorane/air mixture and injected intraperitoneally with a single dose (150 mg/kg) D-luciferin. Photon counts/sec were acquired before and 6 hrs post-TRAIL treatment (FIG. 11A) using IVIS imaging system (Caliper Life Sciences). Fold induction (FIG. 11B) was calculated by normalizing post treatment values to pre-treatment values per mouse.

The data demonstrates that the mutant thermostable biosensors are extremely sensitive as 100 fold bioluminescence activation upon TRAIL treatment was seen in the mouse xenograft model. D54-MG reporter xenografted nude mice were treated with 8 mg/kg of TRAIL. Photon counts/sec were acquired pre- and post-treatment (FIG. 11). The fold induction was calculated by normalizing post treatment values to pre treatment values per mouse (FIG. 11B). FIG. 11C shows a table depicting the average photon counts/sec at baseline and 6 hrs post treatment in addition to fold changes achieved with different biosensor versions.

### Example VIII: Imaging of Cell Death in Breast Bone Metastasis

To demonstrate the use of the thermostable caspase-3 biosensors to detect cell death in animals, 100,000 MDA-MB231/1833 cells ("1833"; breast cancer cell line) stably expressing TL-CP233-Caspase-3 biosensor (derived as described in Example VI for glioma cells) were implanted into the tibia of nude mice. Tumor growth was followed by MRI and TRAIL treatment was initiated when the tumor reached 5-15 mm³.

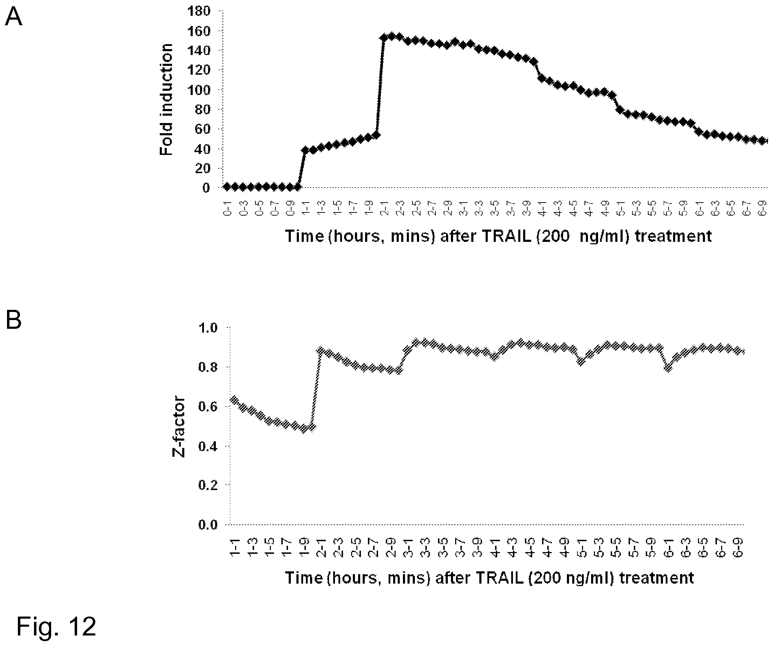

For *in vivo* bioluminescence detection, mice were anesthetized using 2% isofluorane/air mixture and injected intraperitoneally with a single dose (150 mg/kg) D-luciferin. Photon counts/sec were acquired before and 6 hrs post-TRAIL treatment or as indicated in FIG. 12A-D using IVIS imaging system (Caliper Life Sciences). Fold induction (FIG. 11B) was calculated by normalizing post treatment values to pre-treatment values per mouse.

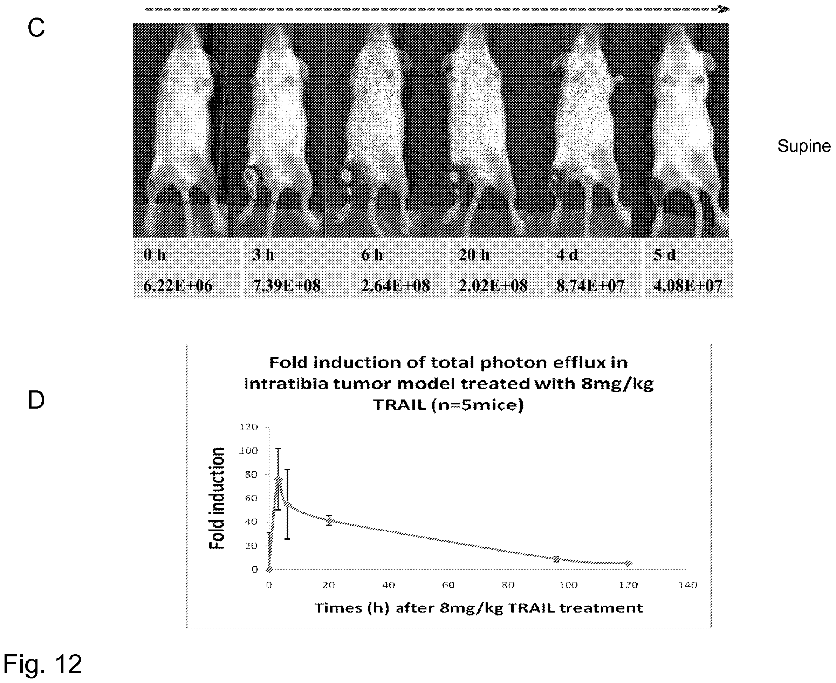

In FIG. 12A, intratibial implanted MDA-MB231/1833 cells stably expressing TL-CP233-Caspase-3 were treated with TRAIL (200 ng/mL) and imaged every hour for 10 consecutive min. Fold induction was calculated by normalizing data to pre-treatment value. In FIG. 12B, Z factors were calculated as described in Zhang et al (Biomol Screen. 4:67-73. 1999) for every time point, and an average Z factor of 0.82 sufficed assay suitability for high-throughput screening. In FIG. 12C, representative images taken at the indicated time points of intratibial implanted TL-CP233-Caspase-3 stably expressing MDA-MB231/1833 cells with the photons/sec. In FIG. 12D, fold induction of xenografted animals tested treated with TRAIL. This data highlights the usefulness of the thermostable biosensor for imaging cell death dynamically and over time in mouse models.

### Example IX: Utility of the Thermostable Caspase Biosensor in High-Throughput Screening

To demonstrate the utility of the thermostable biosensors for high-throughput screening (HTS), the MDA-MB231/1833 ("1833") cells stably expressing TL-CP233-Caspase-3 from Example VIII were used to screen compounds in the NIH Clinical Collection Biofocus and TimTec Kinase Inhibitor libraries.

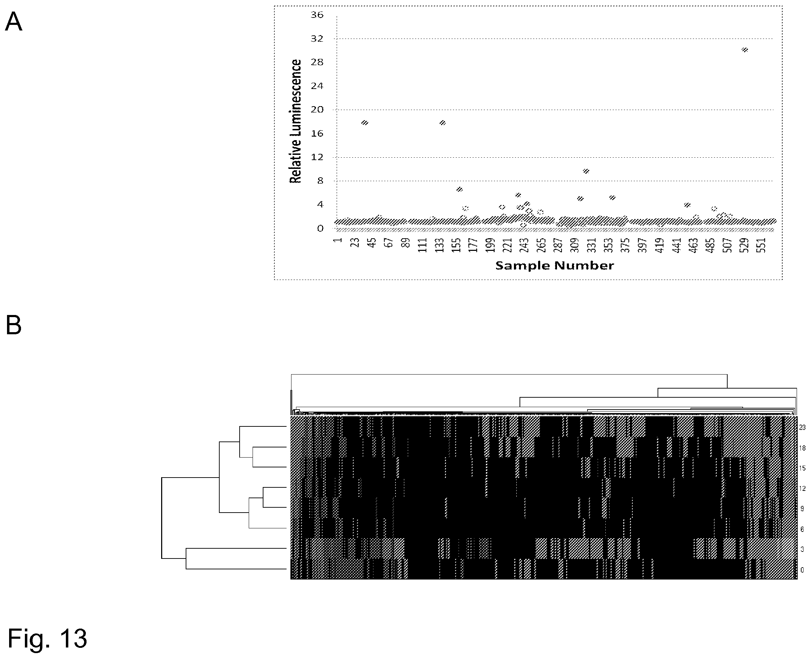

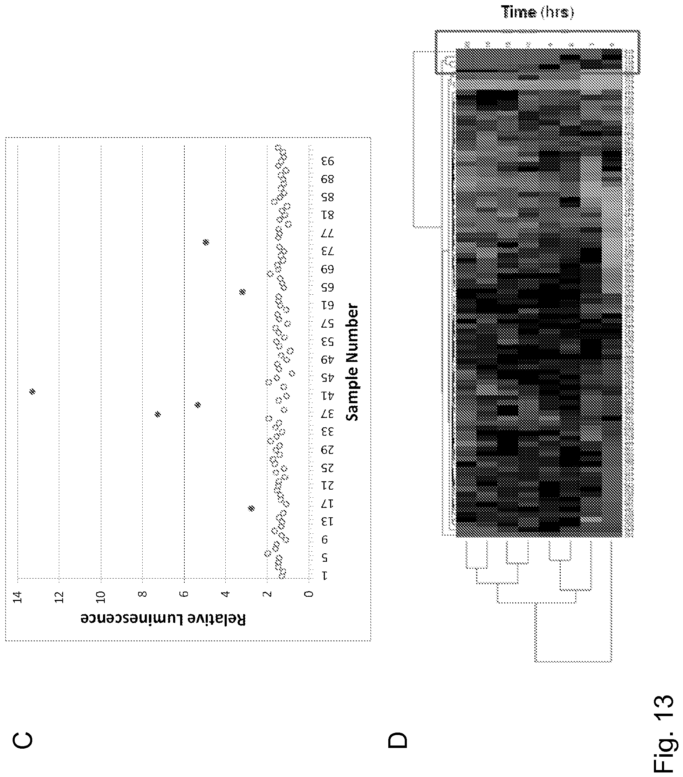

TL-CP233-Caspase-3 MDA-MB231/1833 cells were seeded at 10,000 cells/well in a 96-well plate. Forty-eight hrs post-seeding, the media was changed to CO₂ Independent Media containing 1% GloSensor cAMP Reagent (Promega; Cat. No. E1290) and incubated for 0-23 hrs with compound at a final concentration of 10 µM. A total of 483 compounds in the NIH Clinical Collection and 80 kinase inhibitors from the TimTec collection were tested. The addition of media and compound library was performed using a Titertek Multidrop Microplate Dispensor (ThermoFisher Scientific). Relative luminescence was calculated by normalizing values of compound treated wells to untreated wells. (FIGS. 13A and 13C). FIG. 13A shows the relative luminescence upon compound treatment (max) from compounds in the NIH Clinical Collection Biofocus Library. FIG. 13C shows the relative luminescence upon compound treatment (max) from compounds in the TimTec Kinase Inhibitor Library. Maximum values reaching above 4 were considered significant. Heat maps were generated using bioinformatics toolbox of Matlab Software and show correlation of biosensor activation over time. (FIGS. 13B and 13D). The Z-factor was calculated as previously described in Example VIII.

Due to the ability of repeated imaging of the thermostable biosensor, dynamics of apoptosis in response to various drugs could be imaged. This allowed for the identification of interesting death inducing compounds in the otherwise chemoresistant 1833 breast cancer cell line.

### Example X: Purification of MMP-2 Sensor

The matrix metalloproteinases (MMP) are a homologous group of zinc enzymes that participate in the breakdown of the major protein components of the extracellular matrix. Five major MMP have been identified in humans and implicated in connective tissue turnover and destruction. These include the fibroblast- type and neutrophil-type interstitial collagenases that hydrolyze the type I, II, and III collagens that make up the majority of the matrix. Fibroblast collagenase also hydrolyzes native type VII and X collagens. The MMP are sometimes referred to by a numerical code in which the fibroblast-type and neutrophil-type collagenases are designated MMP-1 and MMP-8, respectively. A 72-kDa gelatinase (MMP-2) is produced by proliferating fibroblasts and tumor cells, while a distinct 92-kDa gelatinase (MMP-9) is produced by neutrophils, macrophages, and certain transformed cells.

The MMP-2 sensor (SEQ ID NOs:69 and 70) used herein contains the 1A5 variant backbone and the human MMP-2 recognition site, PLGMWSR (SEQ ID NO:75). In addition, the MMP-2 sensor contains two purification tags: a GST tag on the N-terminus of the sensor that is separated from the sensor region by a TEV protease site (for removal of the GST tag from the purified MMP-2 sensor) and a 5x HQ (HQHQHQHQHQ; SEQ ID NO:79) tag on the C-terminus of the sensor.

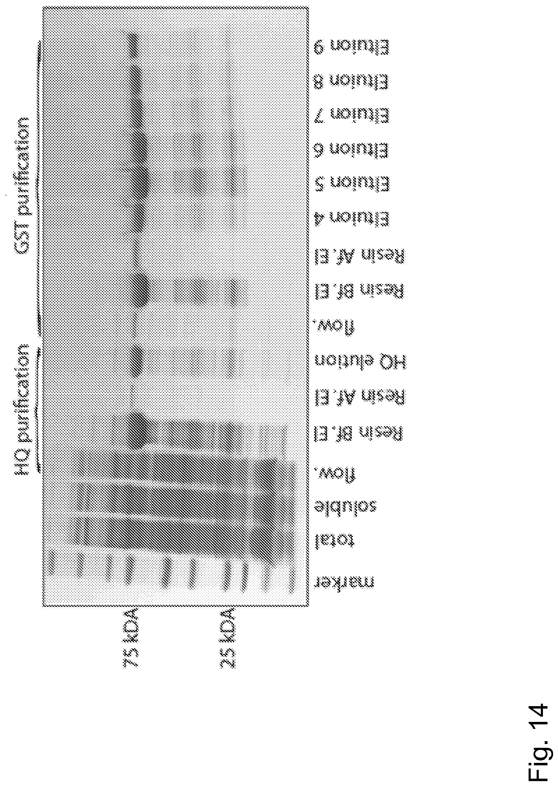

Purification of the MMP-2 sensor was performed as follows using His and GST purification.
1. 2-5 mL cultures of *E. coli* KRX cells (Promega) containing the MMP-2 sensor were grown in LB/ampicillin with shaking at 37°C.
2. Each culture was diluted 1:100 in 1L LB with 0.05% rhamnose and 0.05% glucose.
3. Incubated at 25°C for 18-20 hrs.
4. Cells were harvested by centrifugation at 5000g for 5 min (split 1L into 2-500 mL aliquots), cell paste weight was determined, and placed at -20°C overnight.
5. One of the 2 cell pastes was resuspended with 30 mL lysis buffer (8.5 mL/g cell paste; 50 mM NaH₂PO₄, 300 mM NaCl, 10 mM Imidazole and pH to 8.0 with NaOH). 1 mg/mL lysozyme was added, and the resuspension was incubated on ice, with inverting occasionally, for 30 min.
6. The lysis solution was sonicated at power 6.0 for 2 min (5 sec on, 5 sec off). 100 µl of the sample was saved as "total" sample.
7. The lysis solution was spun at 16,000g for 20 min. 100 µl of the sample was saved as "soluble" sample.
8. 1 mL 50% Ni-NTA resin (Qiagen; pre-washed in lysis buffer) per 6 mL lysate (5 mLs total) was added and mixed at 4°C for 1 hr.
9. Sample was spun at 700 rpm on a tabletop centrifuge for 2 min. 100 µl of the sample was saved as "flowthrough" sample with the supernatant discarded.
10. The resin was washed in 40 mL lysis buffer, mixed at 4°C for 5 min, spun at 700 rpm on a tabletop centrifuge for 2 min, and the supernatant was discarded.
11. The resin was then washed in 40 mL wash buffer (lysis buffer with 20 mM Imidazole), spun at 700 rpm on a tabletop centrifuge for 2 min, and the supernatant was discarded.
12. 10 mL of wash buffer was added and mixed, and the supernatant was added to an empty column.
13. The column was washed with 50 mL wash buffer and 100 µL resin was removed and saved.
14. The sensor was eluted from the column with 10 mL elution buffer (lysis buffer with 250 mM Imidazole) with 0.5 mL fractions collected and directly assayed using the Bradford Assay.
15. 100 µL of the resin was removed and saved, the elution fractions were combined, and the combined fraction was diluted to 10 mL in lysis buffer.
16. The combined fraction was dialyzed (1 hr with 1L twice) in GST binding/wash buffer (1XPBS).
17. The dialyzed protein was added to 5 mL glutathione-sepharose resin slurry (GE Cat #17-0756-01) prewashed in GST binding/wash buffer) and was incubated for 1 hr at 4°C.
18. The resin mixture was spun at 700 rpm in a tabletop centrifuge for 2 min, 100 µL was saved as "flowthrough", and the supernatant was discarded.
19. The resin was added to an empty column, washed with 50 mL GST binding/wash buffer, and 100 µL was removed.
20. The protein was eluted with elution buffer (IX PBS buffer with 10 mM reduced Glutathione). 0.5 mL fractions were collected and directly assayed using the Bradford assay. 100 µL of resin was removed and saved.
21. The saved fractions ("total", "soluble", "flowthrough"(his), His resin before elution, His resin after elution, after dialysis sample, flowthrough (GST), GST resin before elution, GST resin after elution and GST fractions) were analyzed on an SDS-PAGE gel (FIG. 14).
22. The GST fractions were combined and dialyzed in storage buffer (50 mM HEPES pH 7.5, 150 mM NaCl, 1 mM DTT, 50% glycerol).

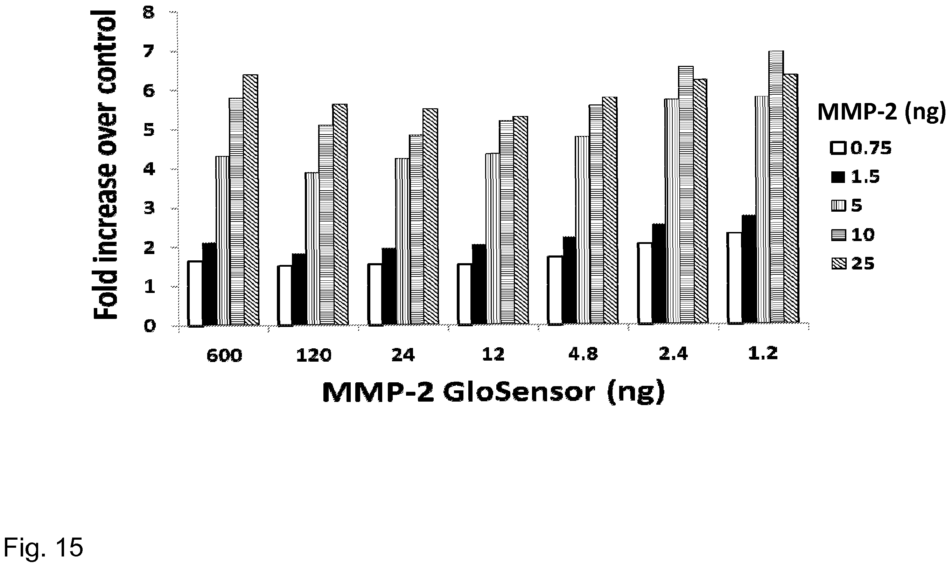

To demonstrate that the purified MMP-2 sensor can detect MMP-2, purified MMP-2 sensor (1.3 mg/mL) was diluted in buffer (50 mM Tris-HCl pH 7.5, 150 mM NaCl, 10 mM CaCl₂, and 0.05% Brij35) as described in FIG. 15. Activated MMP-2 (10 ng/µL; Anaspec) was added to the diluted MMP-2 sensor as described in FIG. 15. Total volume of the final reaction mixture was 50 µL. The mixture was incubated at 37°C for 1 hr. Bright-Glo Assay Reagent (Promega) was prepared according to the manufacturer's instructions, and 50 µL added to the mixture. Luminescence was read immediately on a GloMax MultiPlus luminometer.

FIG. 15 presents the fold increase over control (background from MMP-2 sensor). The results demonstrate that a purified MMP-2 sensor can be used to detect as little as 0.75 ng protein.

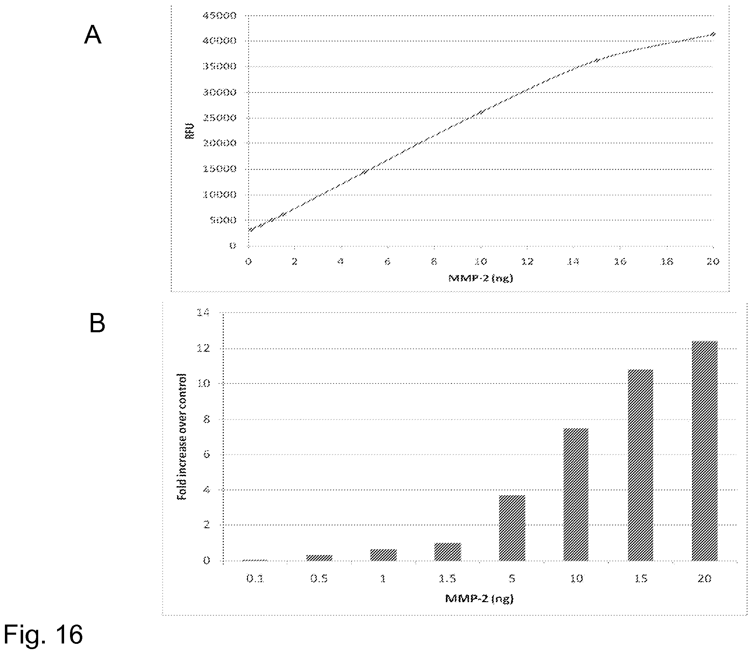

To further demonstrate the sensitivity of the purified MMP-2 sensor, the fluorogenic SensoLyte 520 MMP-2 Assay (Anaspec) was also used to detect MMP-2 protein. The assay was performed according to the manufacturer's instructions. Fluorescence was detected on a Tecan fluorometer at Ex490nm/Em520nm. FIGS. 16A-B report detection of 1.5 ng MMP-2 protein using the SensoLyte assay.

### Example XI: Cell-Free Expression of CBS

To demonstrate that the protease biosensor can effectively and efficiently be cleaved by exogenous protease in a cell-free environment, the CBS variant 1A5 was expressed in wheat germ extract and used to detect Caspase-3.

The CBS variant 1A5 was cloned into the vector pFN19K HaloTag^{®} (Promega Cat. No. G1841) to generate a CBS-HaloTag^{®} (CBS-HT) fusion protein (SEQ ID NOs:71 and 72). 20 µl (8 µg) of the CBS-HT vector was added to 30 µl TnT^{®} SP6 High-Yield Wheat Germ Expression System (Promega Cat. No. L3261) and incubated at 25°C for 90 min.

For the CBS-HT caspase-3 cleavage reaction, one volume of the expression reaction was incubated with an equal volume of either an *E. coli* lysate containing caspase-3 or C(3) Lysis Buffer (0.8X FastBreak (Promega Cat. No. V857A), 10 mM DTT, 0.1% CHAPS, 0.8 mg/mL Lysozyme, 3U/µL RQ1 DNase (Promega Cat. No. M610A)) and incubated for 60 min at room temperature. The *E. coli* lysate containing caspase-3 was prepared from KRX cells overexpressing recombinant caspase-3. Briefly, KRX cells were transformed with pTS1k:caspase-3(T/S). A starter culture (50 mL, LB Broth) was inoculated from a single colony and grown for 17-22 hrs at 37°C with shaking (275 rpm). The starter culture was diluted (1:50) into fresh media and growth was continued for an additional 3 hrs. The incubation temperature was then lowered to 25°C and, after 15 min, expression of caspase-3 was initiated by addition of rhamnose (0.2% final concentration). After 2 hr, cells were collected by centrifugation, re-suspended in 50 mL C(3) Lysis Buffer and incubated at ambient temperature (i.e. 22-24°C) for 10 min. The lysate was clarified by centrifugation (20,000 × g for 20 min at 4°C) and used as the Caspase-3 source.

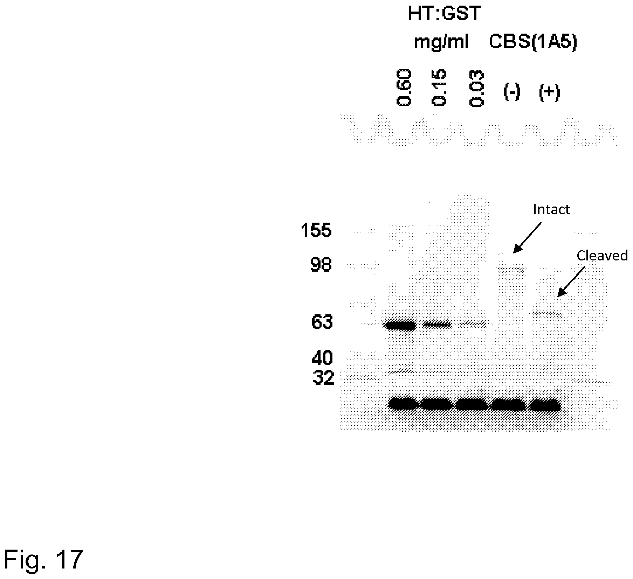

Cleavage of CBS-HT by Caspase-3 was detected in two different ways: by SDS-PAGE analysis and luminescence detection. For SDS-PAGE analysis, cleavage reaction samples were first labeled with the fluorescent marker CA-TAM (chloro alkane-TAMRA ligand (Promega Cat. No. G825A). 20 µl of the sample was added to 20 µL CA-TAM (diluted 1:100 in buffer (1× PBS, 0.05% IGEPAL)) and incubated for 30 min at room temperature. To this sample, 40 µL SDS-PAGE Loading Buffer (120 mM Tris Buffer (pH7.4), 1% SDS, 25.2% Glycerol, 1.5 mM Bromophenol Blue, 100 mM DTT) was added. The resulting solution was incubated at 65°C for 30 min. 10 µL was loaded onto an SDS-PAGE gel. As a control, 0.60, 0.15 and 0.03 mg/mL HT:GST (HaloTag^{®}-GST (Promega Cat. No. G449A) fusion was also loaded onto the gel (FIG. 17). After electrophoresis, CA-TAM labeled species were detected by fluorescent imaging (ex:532, Em:580; FIG. 17). For detection via luminescence, 40 µL of the cleavage reaction samples were added to 60 µL buffer (50 mM HEPES (pH 7.5) and 100 µL Bright-Glo assay reagent. Luminescence was detected as previously described (Table 7).

**Table 7**

| | **WG(HY)** | | |
|---|---|---|---|
| **MIN.** | **BASAL** | **INDUCED** | **RESPONSE** |
| 5 | 10,494 | 4,626,173 | 441 |

### Example XII: Immobilization of CBS

To demonstrate that the protease biosensor when expressed in a cell-free environment maintains activity when immobilized on a solid support, the CBS-HT fusion expressed in wheat germ extract (Example XI) was immobilized to a solid support (resin and plate) and used to detect Caspase-3.

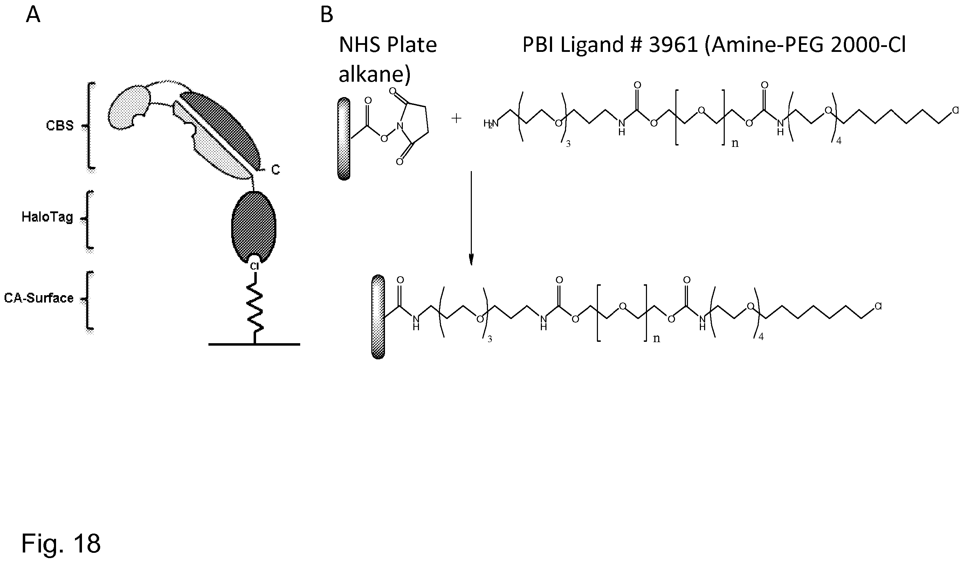

For immobilization to a resin (FIG. 18A), HaloLink resin (25% slurry, Promega Cat. No. G1912) was first equilibrated with HTPB Buffer (50 mM HEPES (pH 7.5), 150 mM NaCl, 1 mM DTT and 0.5 mM EDTA). Resin from one volume of slurry was collected by centrifugation (5 min at 1000 × g), and the storage buffer was removed. The resin pellet was re-suspended in 2 volumes of HTPB and mixed. This process was repeated for a total of three HTPB washes. 100 µL of the CBS-HT fusion from Example XI (cell free expression reaction) was mixed with 25 µL washed resin (50% slurry in HTPB) and incubated overnight with mixing at 4°C. Incubation was continued at ambient temperature for 2 hrs. After incubation, the resin was washed to remove un-bound CBS. The resin was split into two 50 µl aliquots, and each aliquot was washed 3 times with HTPB. The final resin pellets were re-suspended in 50 µL HTPB.

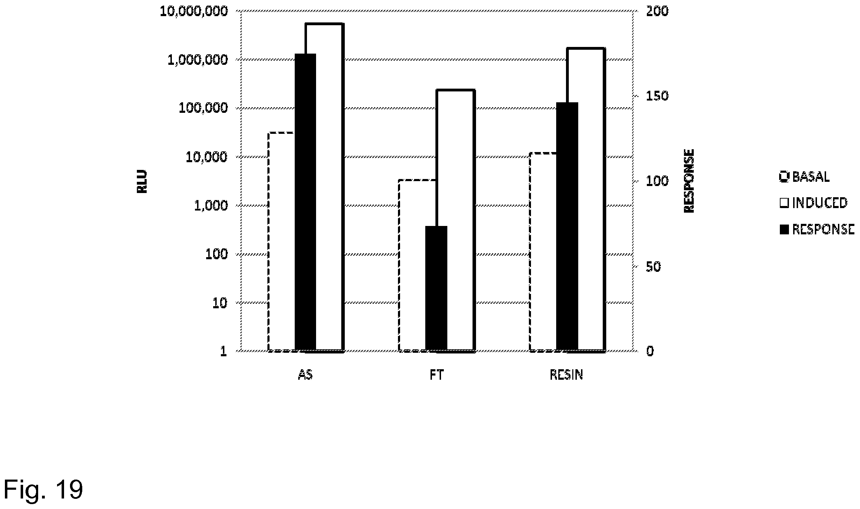

For the CBS caspase-3 cleavage reaction, 20 µl of the washed resin was mixed with 20 µL of either *E. coli* lysate containing caspase-3 or C(3) Lysis Buffer (as in Example XI) and incubated for 30 min at ambient temperature. 60 µl HEPES pH 7.5 was added to the samples, followed by the addition of 100 µl Bright-Glo Assay Reagent. Luminescence was detected as previously described (Table 8 and FIG. 19).

**Table 8**

| | **BASAL** | **INDUCED** | **RESPONSE** |
|---|---|---|---|
| **BLANK** | 10 | 70 | 7 |
| **AS** | 29,553 | 5,162,108 | 175 |
| **FT** | 3,170 | 232,117 | 73 |
| **RESIN** | 11,219 | 1,635,259 | 146 |

For immobilization to a plate (FIG. 18B), a microtiter plate was prepared containing HaloTag^{®} ligand for immobilizing the CBS-HT fusion protein. Briefly, bicarbonate buffer pH 8.5 (100 µL), containing amine-PEG 2000-Cl alkane HaloTag^{®} ligand (0.25 mM (final concentration; PBI 3961 and methoxy-PEG-NH₂ (0.75 mM final concentration) were added to wells of a NHS microtiter plate and incubated for 1 hr at room temperature. The wells were then washed 3 times with PBS containing 0.05% Tween-20. After washing, 50 mM ethanolamine was added, and the plates were incubated for 30 min at room temperature and washed again 3 times with PBS containing 0.05% Tween-20. The plate was then stored at 4°C with PBS containing 0.05% Tween-20 in each well. For the assay, the wells were washed 3 times with 200 µl HPTB. 50 µl CBS-HT cell-free expression reaction (Example XI) was added to the wells, and incubated overnight at 4°C. Following incubation, the plate was washed 3 times with 200 µl PBSI (1×PBS with 0.05% IGEPAL). 100 µL *E. coli* lysate containing caspase-3 or C(3) lysis buffer (described above) were added and the plate was incubated for 60 min with mixing at room temperature. The wells were then washed with 100 µl PBSI. 100 µL HEPES pH 7.5 and 100 µl Bright-Glo Assay Reagent were added and luminescence was detected as previously described (Table 9).

**Table 9**

| **MIN.** | **BASAL** | **INDUCED** | **RESPONSE** |
|---|---|---|---|
| 0 | 66 | 1,226 | 19 |
| 1 | 16 | 1,636 | 102 |
| 2 | 106 | 2,267 | 21 |
| 3 | 66 | 2,147 | 33 |
| 4 | 126 | 2,297 | 18 |
| 5 | 126 | 2,857 | 23 |
| 6 | 76 | 3,107 | 41 |
| 7 | 56 | 3,377 | 60 |
| 8 | 136 | 3,037 | 22 |
| 9 | 116 | 3,667 | 32 |

### Example XIII: CBS Expression in E. coli

To demonstrate that the protease biosensor can be expressed and function in *E. coli*, the CBS variant 1A5 was expressed in *E. coli* and used to detect Caspase-3.

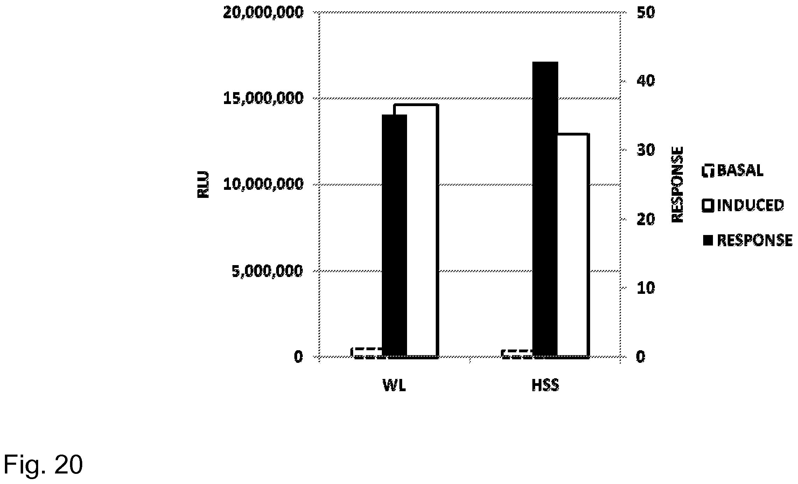

The CBS variant 1A5 was cloned into a bacterial expression vector (pFNA:HQ(5x):CBS:HT(7); SEQ ID NOs:73 and 74) containing HaloTag^{®} (C-terminal to the CBS) and a 5x HQ tag (N-terminal to CBS). The fusion protein was expressed in *E. coli* as follows: *E. coli* (KRX) was transformed with the vector. A starter culture (50 mL, LB Broth) was inoculated from a single colony and grown for 17-22 hrs at 37°C with shaking (275 rpm). The starter culture was diluted (1:50) into induction media (500 mL, LB Broth with 0.05% glucose and 0.02% rhamnose) and growth was continued for another 17-22 hrs at 25°C with shaking (275 rpm). The culture was divided into two 250 mL aliquots, and cells were collected by centrifugation (5,000 × g for 20 min at 4°C). One cell pellet was re-suspended in Lysis Buffer (25 mL, 50 mM HEPES (pH 7.5), 0.2X FastBreak, 2 mM DTT, 0.05% CHAPS, 50 mM Arginine, 50 mM Glutamic acid, 0.2 mg/mL Lysozyme, 10U/mL RQ1 DNase, and Protease Inhibitors (Beckton/Dickenson Cat. No. 544779)) and incubated on ice for 30 min. After incubation, the sample was sonicated (Misonix Sonicator-3000, 4 min total, 5 sec on, 5 sec rest, Power Setting 5). The crude lysate was clarified by centrifugation (20,000 × g for 20 min at 4°C), and the supernatant (cleared lysate) was used as the CBS source. For the caspase-3 cleavage reaction, 20 µl of the cleared lysate was mixed with 20 µL *E. coli* lysate expressing caspase-3 (Example XII) and incubated at room temperature for 30 min. 60 µL HEPES pH 7.5 was added, followed by the addition of 100 µL Bright-Glo Assay Reagent. Luminescence was detected as previously described above (FIG. 20).

### Example XIV: Purification of CBS from E. coli

To demonstrate the ability to purify a functional protease biosensor from *E. coli*, the CBS expressed in Example XIII was purified using HisLink (Promega Cat. No.V8821)) column chromatography according to the manufacturer's instructions. Briefly, 25 mL of cleared lysate was made 0.5 M in NaCl (final concentration) and applied to 2 mL of settled HisLink resin that had been equilibrated with Binding Buffer (100 mM HEPES (pH7.5), 10 mM Imidazole, 500 mM NaCl). The resin was washed with 12 mL of Binding Buffer followed by washing with Elution Buffer (100 mM HEPES (pH 7.5), 1000 mM Imidazole). 1.75 mL fractions were collected.

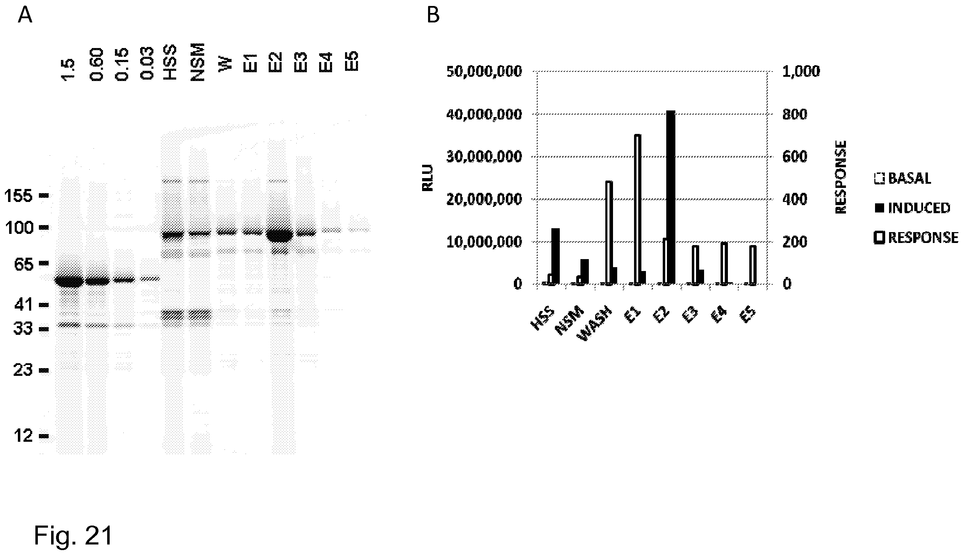

For SDS-PAGE gel analysis, samples were labeled with CA-TAM and analyzed as described previously (FIG. 21A). For caspase-3 cleavage reaction, 20 µl of each sample was mixed with 20 µL *E. coli* lysate expressing Caspase-3 (Example XII) and incubated at room temperature for 30 min. 60 µL HEPES pH 7.5 was added, followed by the addition of 100 µL Bright-Glo Assay Reagent. Luminescence was detected as previously described above (Table 10 and FIG. 21B).

**Table 10**

| | **HSS** | **NSM** | **WASH** | **E1** | **E2** | **E3** | **E4** | **E5** |
|---|---|---|---|---|---|---|---|---|
| **BASAL** | 301,304 | 176,259 | 7,734 | 4,221 | 193,268 | 18,454 | 1,130 | 270 |
| **INDUCED** | 12,875,742 | 5,822,003 | 3,706,541 | 2,951,899 | 40,913,496 | 3,287,059 | 215,580 | 48,068 |
| **RESPONSE** | 43 | 33 | 479 | 699 | 212 | 178 | 191 | 178 |

### Example XV: Immobilization of E. coli Expressed CBS

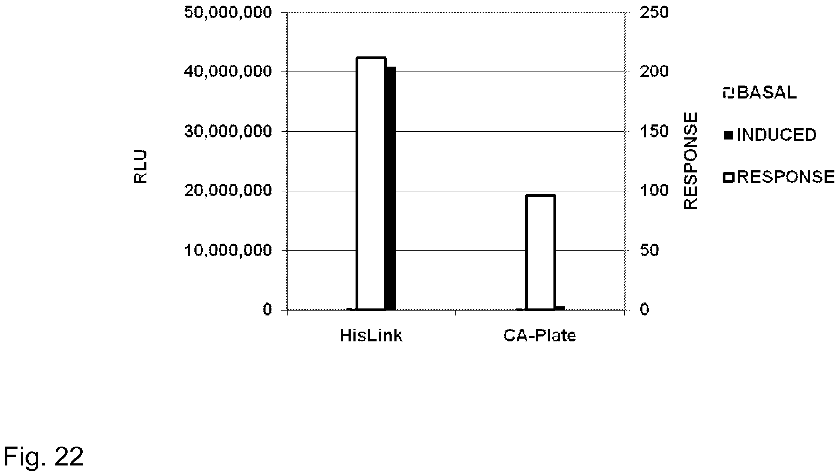

To demonstrate that the protease biosensor expressed in *E. coli* can be immobilized to a solid support while maintaining the ability to detect protease, purified HQ:CBS:HT (5x HQ tag:CBS: HaloTag) was immobilized on HaloLink resin and HaloLink plates and assayed for activation by Caspase-3. For immobilization on HaloLink resin, 100 µL of purified HQ:CBS:HT was added to 30 µL of settled HaloLink resin (pre-equilibrated with HTPB as described previously) and incubated for 2 hrs at ambient temperature. The resin was washed 3 times with 300 µL HTPB, and the final resin pellet was re-suspended in 300 µL HTPB. 50 µL washed resin was added to 50 µL *E. coli* Lysate containing Caspase-3 or 50 µL C(3) Lysis Buffer and incubated for 30 min at ambient temperature. 100 µL of Bright-Glo Assay Reagent was added and luminescence was detected as previously described (Table 11 and FIG. 22).

For immobilization on HaloLink plates, 100 µL of purified HQ:CBS:HT was added to a microtiter plate containing immobilized HaloTag^{®} ligand (Example XII). The plate was incubated at room temperature with mixing for 2 hrs. The plate was then washed 3 times in IX PBSI (IX PBS with 0.05% IGEPAL) and incubated with 100 µL of *E. coli* Lysate containing Caspase-3 (prepared as described previously) for 30 min at room temperature. 100 µL Bright-Glo Assay Reagent was added, and luminescence detected as previously described (Table 11 and FIG. 22).

**Table 11**

| | **HisLink** | **HaloLink-Plate** |
|---|---|---|
| **BASAL** | 193,268 | 6,880 |
| **INDUCED** | 40,913,496 | 661,597 |
| **RESPONSE** | 212 | 96 |

### Example XVI - Kinetic study of NK92 cells with different target cells

To determine whether the granzyme B biosensors of the present invention can be used to detect cell-mediated cytotoxicity, protease biosensors containing 1 of 3 granzyme B recognition sites or 1 of 4 control recognition sites were generated.

Granzyme B cleavage site design:
1. Search for protease sites for known granzyme B target proteins.
2. Create the corresponding non-cleavable control sequences as well.

Site B (BID): GR***IEAD***SE (SEQ ID NO:80) and negative control: ***IEAA*** (SEQ ID NO:83) (grB and caspase 10)

Site C (Casp3): G***IETD***SG (SEQ ID NO:82) and negative control: ***IETA*** (SEQ ID NO:84) (grB and caspase 8)

Site D (DDPK): KS***VGPD***FG (SEQ ID NO:81) and negative control: ***VGPA*** (SEQ ID NO:85) (grB specific)

Constructs were expressed in the target cells, K562 cells, and co-incubated with NK effector cells. Luminescence was detected. K562 target cells were transduced with one of the following vectors:
i) pGloSensor^{™}-30F-GRIEADSE: Granzyme B sensor B ("B+");
ii) pGloSensor^{™}-30F-IEAA: Negative control sensor B ("B-");
iii) pGloSensor^{™}-30F-GIETSDG: Granzyme B sensor C ("C+");
iv) pGloSensor^{™}-30F-IETA: Negative control sensor C ("C-");
v) pGloSensor^{™}-30F-KSVGPDFG: Granzyme B sensor D ("D+");
vi) pGloSensor^{™}-30F-IVGPA: Negative control sensor D ("D-"); or
vii) pGloSensor^{™}-30F-DEVD: Positive control sensor ("DEVD").

Cells expressing the granzyme B sensors or control sensors were selected using G418. Once selected, the granzyme B sensor or control sensor target cells were incubated with NK92 effectors cells. Cells were then co-incubated, a luciferase detection reagent added, and luminescence detected on a Veritas luminometer. The DEVD construct was used as a positive control as an apoptosis-substrate for activated caspase 3/7 as it will activate with all inducers of apoptosis.

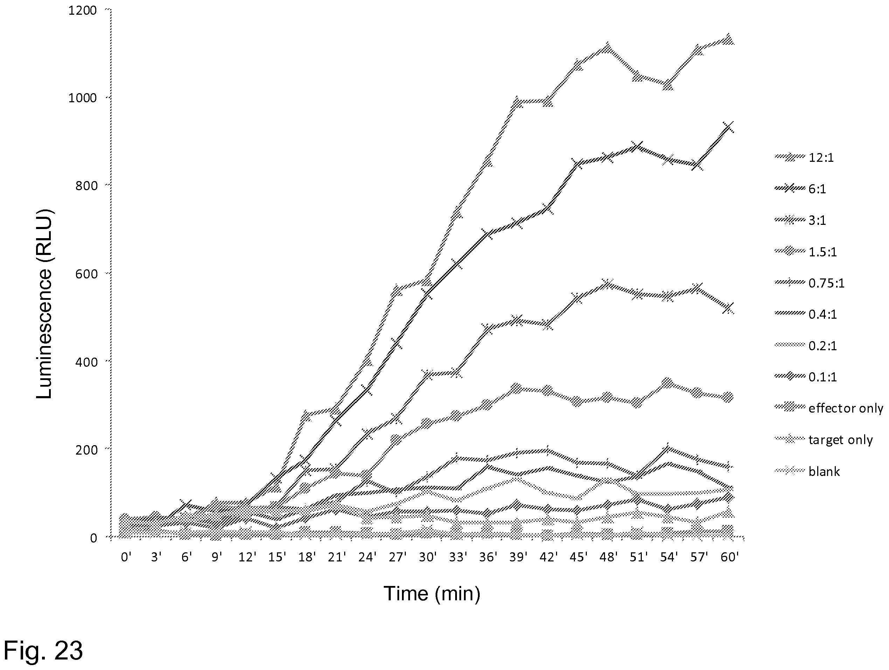

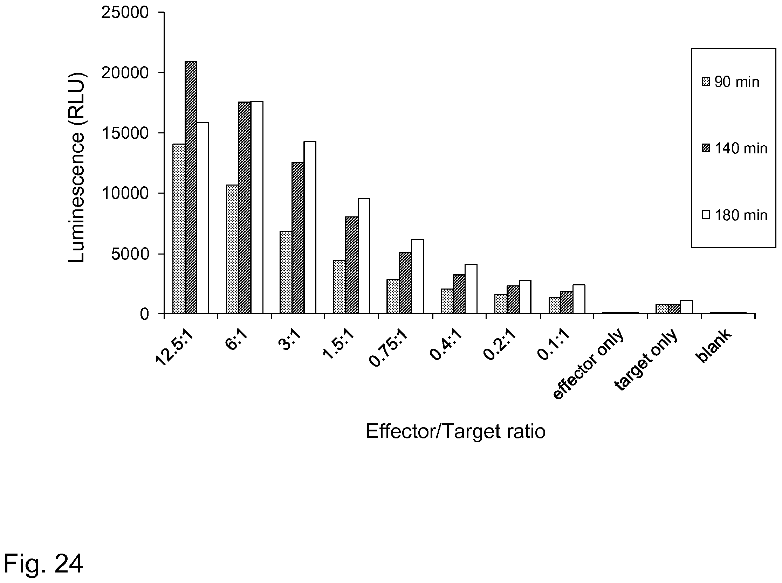

To establish the capacity for a positive control to work with the control effector cell line NK92, K562 cells were transfected with the caspase 3/7 cleaved firefly ("DEVD") biosensor construct. A stable transfectant was obtained by limiting dilution in G418. After incubation with luciferin (2%) for 30 min, the K562 cells were added to wells of a 96-well round bottom, white plate at 30,000 cells/well. NK92 cells were then added to the wells at decreasing Effector/Target (E/T) ratios (from 50:1 to 0.1:1). After centrifugation of the plate at 200 × g for 5 min, luminescence was measured at 37°C every 3 min for a total of 60 min on a Biotek luminometer. FIG. 23 shows an increase of the luminescent signal 6 min after the incubation and a signal plateau at about 40-45 min. An increase of the luminescent signal can be seen in E/T ratios as low as 0.75:1. Control K562 target alone had a low basal signal. NK92 and K562 cells expressing the DEVD biosensor construct were co-incubated at 37°C in a 5% CO₂ incubator for 90 min, 140 min, and 180 min, respectively. The plates were cooled at room temperature for 10 min, and luminescence measured on a Veritas plate reader. FIG. 24 shows an induction of luminescent signal in all different E/T ratios as compared with the target alone. Longer incubation (180 min) at 37°C resulted in higher luminescent signal. FIGS. 23 and 24 show the capacity for the positive control to work with the control effector cell line, NK92.

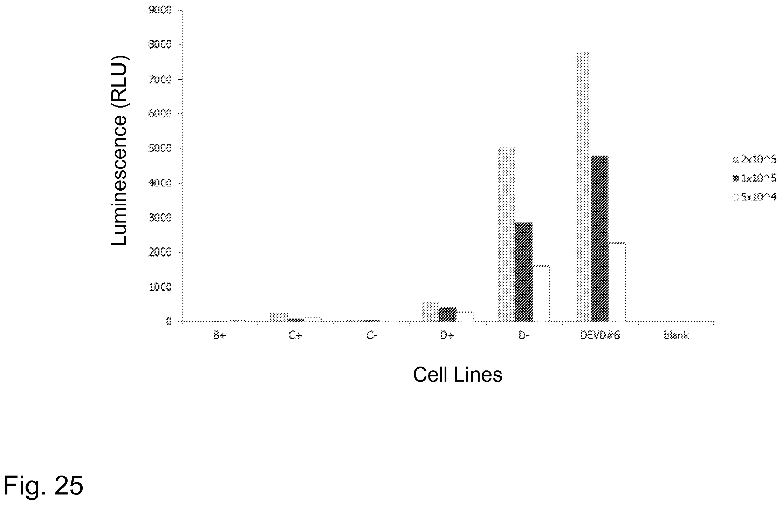

The granzyme B biosensors, B+, C+, C-, D+, and D-, were transfected into K562 cells and selected using G418 selection. The caspase 3/7 biosensor transfected cells (K562-DEVD clone #6; "K562-DEVD #6") were treated the same way as described above and served as a comparison. Cells were harvested and resuspended in 2% luciferin substrate and aliquoted into wells of a 96-well round bottom, white plate at 2×10⁵, 1×10⁵, and 5×10⁴ cells per well, respectively. Plates were incubated at 37°C for 30 min and cooled at room temperature for 10 min. Luminescence was read on a Veritas plate reader. FIG. 25 shows that K562-DEVD #6 and K562-D- (a negative control for K562-D+ construct cells) target cells have a higher basal luminescence (i.e., no effectors) than other granzyme B constructs target cells. FIG. 25 also shows that the co-incubation of target cells expressing the granzyme B sensors with the NK92 effector cells lead to the generation of luminescence when NK cell signaling occurs. In addition, the data demonstrated that the luminescence generated was easily detected in as little as 30,000 target cells/well with a 6:1 ratio of effector to target cells in a 96-well plate.

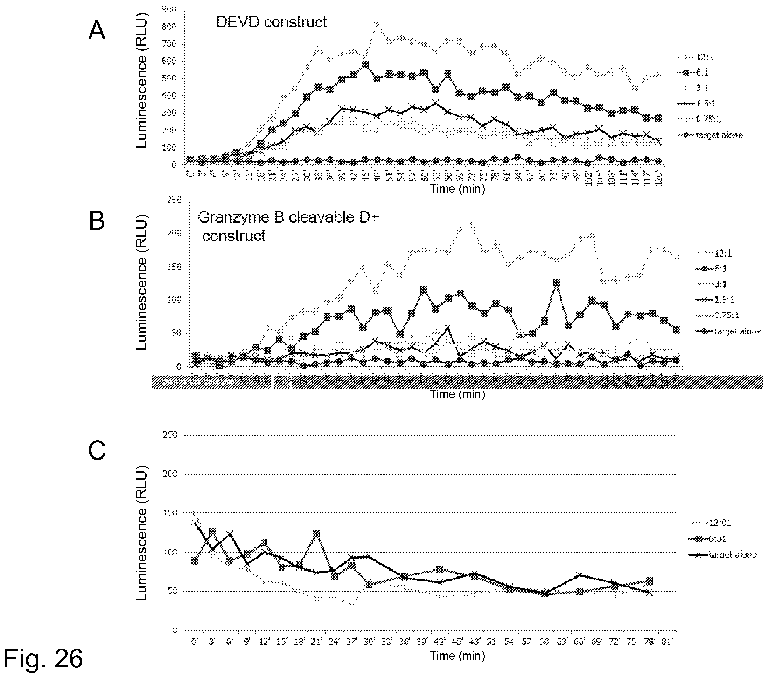

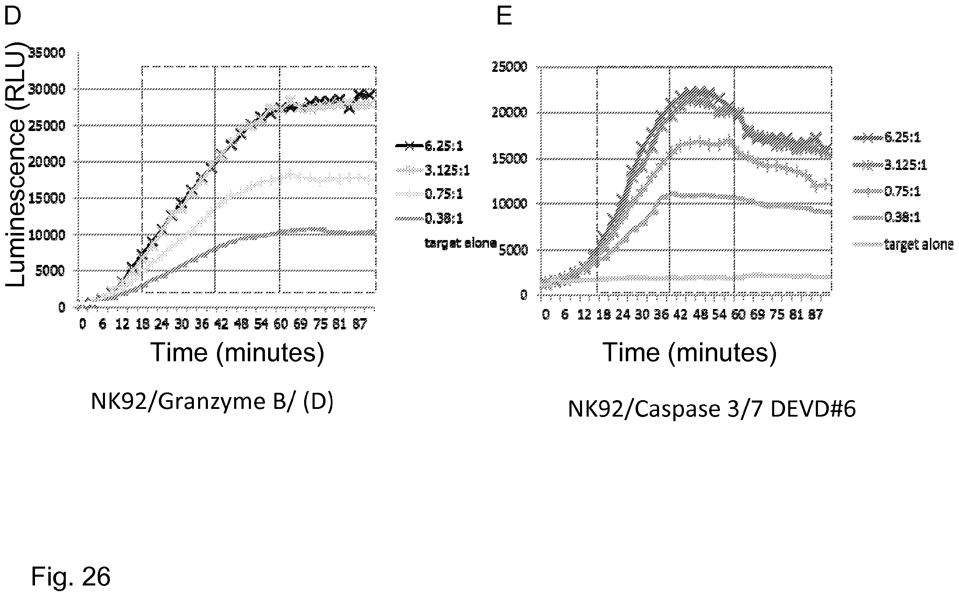

The K562-DEVD #6, granzyme B cleavable (D+) construct, and the granzyme B negative control (D-) construct target cells were examined. Transfected K562 target cells (K562-DEVD clone #6 in FIGS. 26A and 26E, K562-D+ parental line in FIGS. 26B and 26D, K562-D-parental line in FIG. 26C) were incubated with 2% luciferin substrate at 37°C in a 5% CO₂ incubator for 30 min. Cells were aliquot into wells of a 96-well round bottom, white plate at 30,000 cells/well. A titration of effector NK92 cells was then added to the wells at various Effector/Target (E/T) ratios (from 12:1 to 0.75:1). The plate was centrifuged at 200 × g for 5 min and incubated in a Biotek luminometer at 37°C. Luminescence was measured every 3 min for a total of 120 minutes. FIGS. 26A-C show induction of luminescent signal in K562-DEVD #6 (FIG. 26A) and K562-D+ target cells (FIG. 26B) when effector NK92 cells were added, but no induction of luminescent signal in K562-D- target cells (FIG. 26C). FIGS. 26D and 26E show an increase in luminescent signal 6 min after incubation and a signal plateau at about 40-45 min, similar to the results shown in FIG. 23. Also, FIGS. 26D and 26E show that an increase of the luminescent signal can be seen in E/T ratio as low as 0.75:1 and the control. K562 target cells alone had low basal signal.

### Example XVII

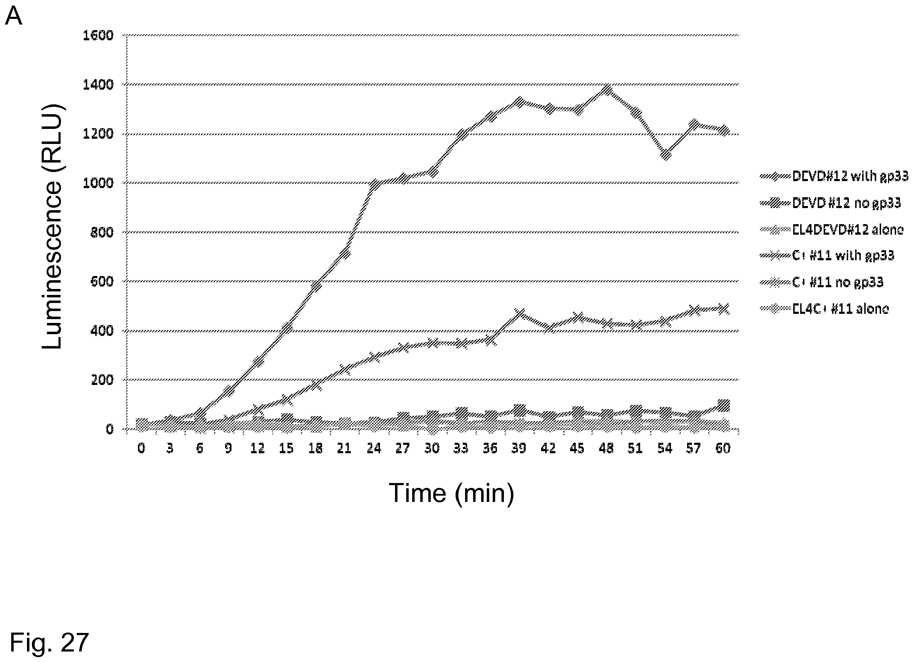

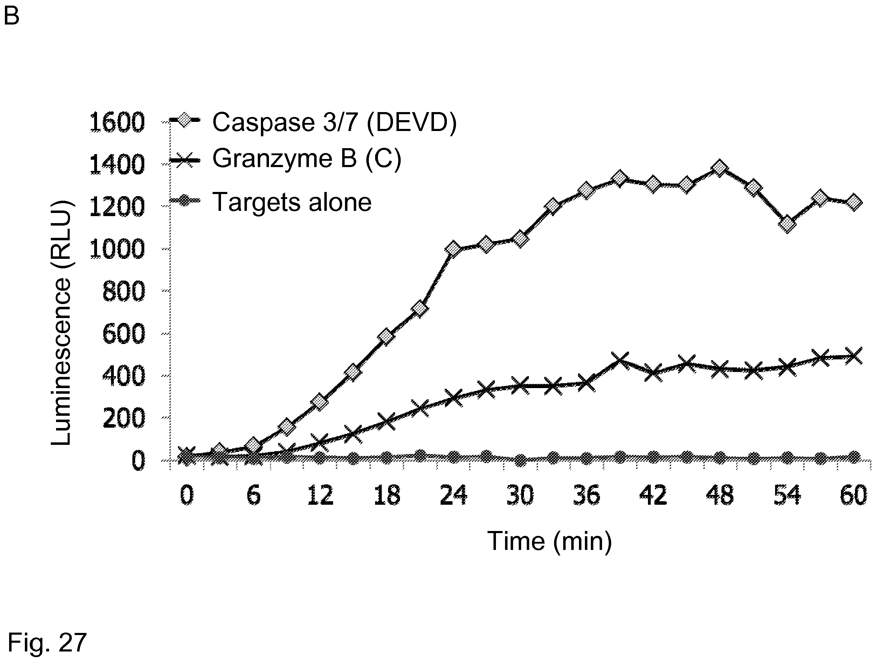

The granzyme B biosensors described above were tested with EL4 target cells (ATCC Manassas, VA) using mouse cytotoxic T cells (CTLs) as effectors to determine if the sensors work with different target cells and effector cells. Splenocytes from T-cell receptor (TCR) transgenic P14 mice (available through A.T.C.C.) were stimulated *in vitro* with Lymphocytic Choriomeningitis (LCMV) gp33 peptide for 3 (FIG. 27A) or 2 (FIG. 27B) days followed by expansion in IL-2 for 2 or 3 days, respectively. The CTL effector cells were isolated and added to plates containing 20,000 per well of EL4 target cells preincubated with 2% GloSensor cAMP reagent in an effector/target ratio of 12:1. The plate was centrifuged at 200 × g for 5 min, and luminescence measured on a Biotek luminometer at 37°C for 60 min. As shown in FIGS. 27A and 27B, activated CTL cells from P14 mice triggered caspase 3/7 (DEVD) and granzyme B (C+) biosensors in murine EL4 cells through antigen specific recognition.

### Example XVIII

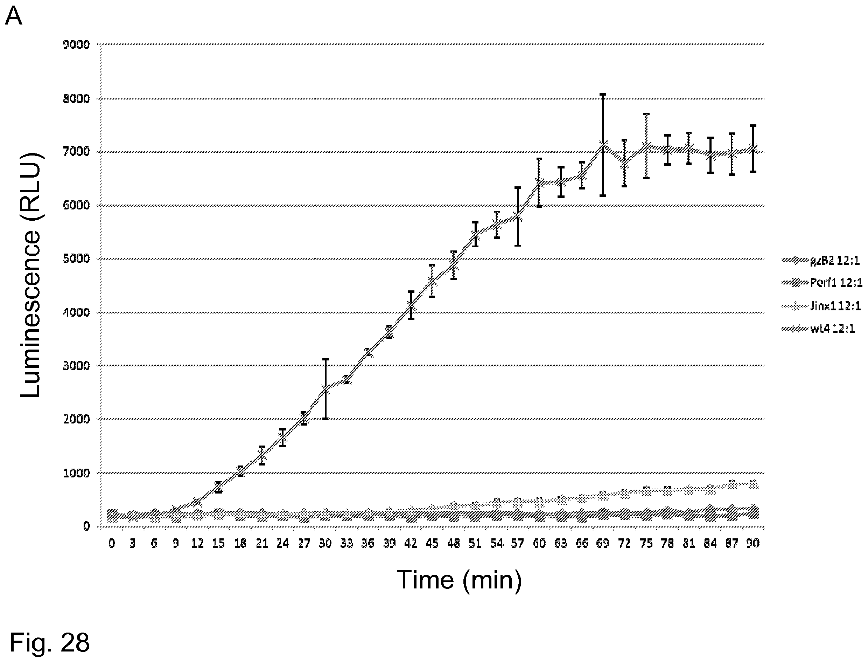

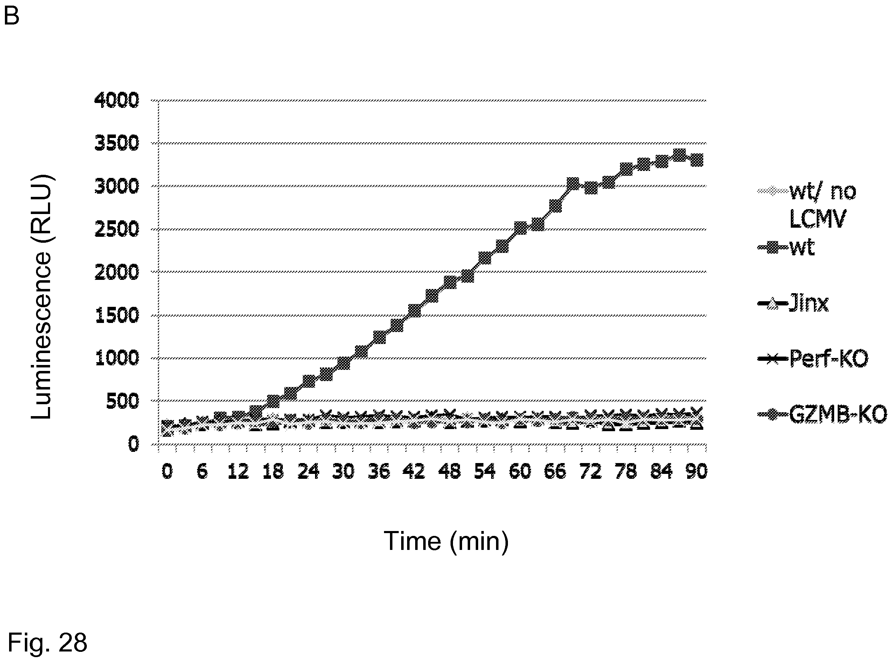

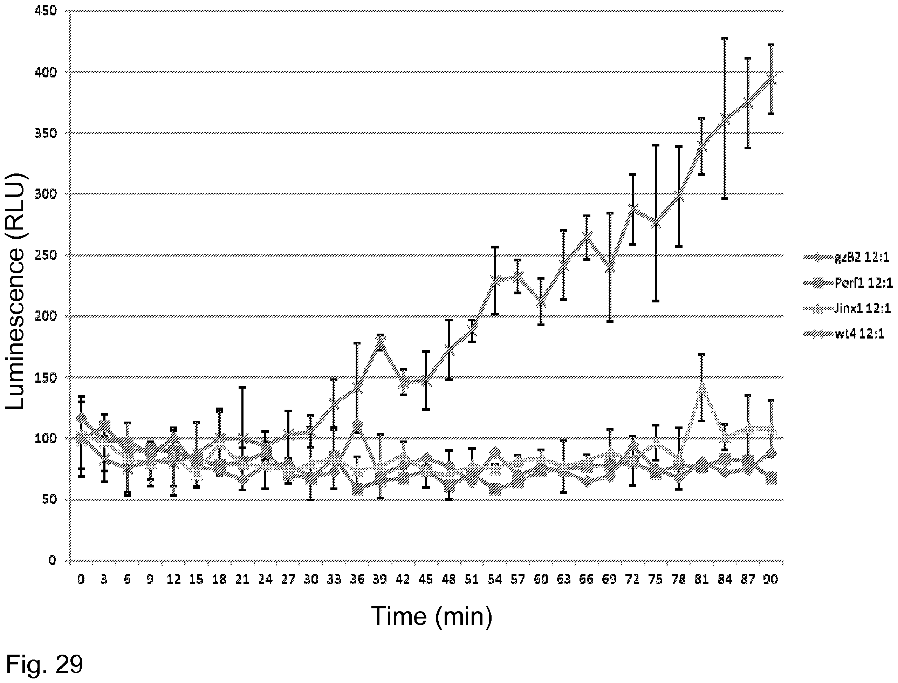

The granzyme B sensors described above were tested to determine if the sensors were granzyme and/or perforin dependent. Granzyme B knockout, perforin knockout and Jinx mice were used and are described in Lykens et al., Blood 118:618-626 (2011). The mice (three granzyme B knockout mice, three perforin knockout mice, three Jinx mice, and five wild type mice) were infected with LCMV (see Lykens et al., Blood 118:618-626 (2011)). On day 7 after the infection, splenocytes were harvested and co-incubated with gp33-pulsed EL4 target cells transfected as described above with the caspase 3/7 biosensor or the granzyme B biosensor at a 12:1 ratio. The gp33 peptide is the dominant LCMV epitope and used to present antigen to effector T cells. Luminescence was measured at 37°C with a Glomax luminometer for 90 min. FIGS. 28A and 28B show that caspase 3/7 biosensor is dependent on delivery of granzyme B into the target cells and on perforin. FIG. 29 shows that the granzyme B biosensor (C+) is dependent on delivery of granzyme B into the target cells.

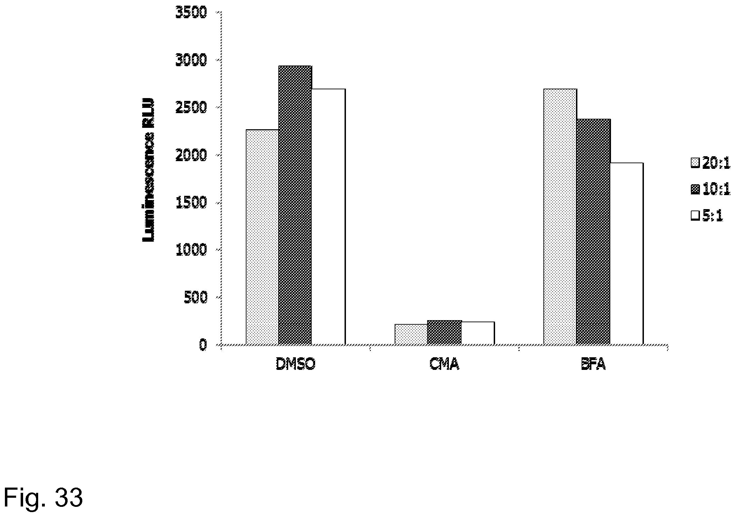

NK92 and K562 cells expressing granzyme B sensor (D+) were used to determine if inhibition of perforin-dependent killing blocks the granzyme B signal. NK92 cells were incubated with concanamycin A (CMA), Brefeldin (BFA), or control (DMSO). The luminescence signal was measured on Veritas luminometer following a 60 min co-incubation at varying E/T ratios at 37°C. FIG. 33 shows the secretory granule/perforin dependence of the signal generated.

### Example XIX

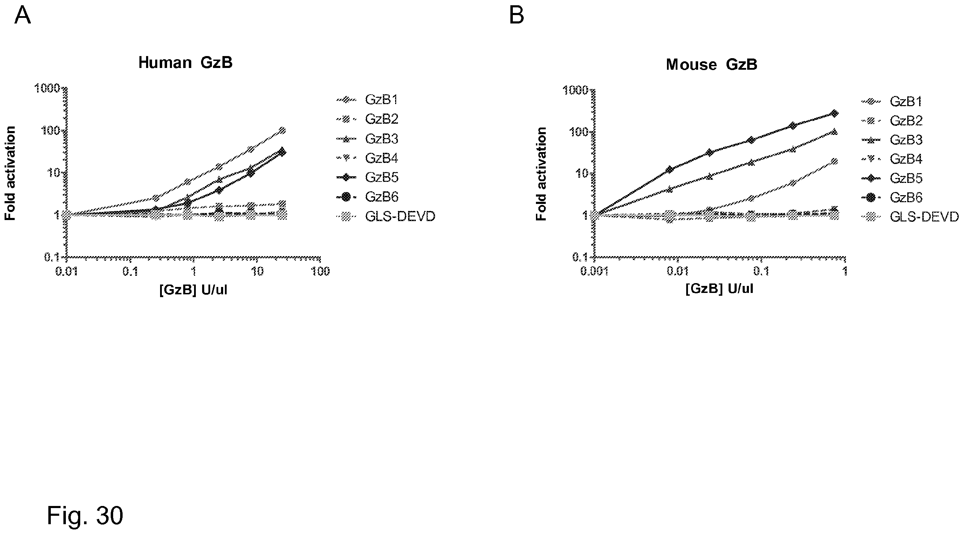

To confirm activation and specificity, biosensor constructs encoding GRIEADSE ("GzB1"), KSVGPDFG ("GzB5"), GIETDSG ("GzB3") or DEVDG ("GLS-DEVD") recognition and cleavage sites, together with non-cleavable control constructs encoding GRIEAASE ("GzB2"), KSVPPAFG ("GzB6"), GIETASG ("GzB4"), were expressed *in vitro* and treated with mouse or human granzyme B (see Table 12). Biosensor constructs were transcribed and translated in wheat germ extract (Promega cat # L4140) plus Fluorotect (Promega cat # L5001) following the manufacturer's recommended protocol for two hours at 30°C. Human granzyme B (Enzo BML-SE238) or mouse granzyme B (Sigma G9278) were serially diluted in assay buffer (100 mM HEPES, pH = 7.4; 200 mM NaCl; 0.2% CHAPS; 2 mM EDTA; 20% glycerol, 1 mM DTT). Serially diluted enzymes were mixed 1:1 with cell-free expression extract, and samples were allowed to incubate at 37 °C for 1 hour. After 1 hour, 10 µL of reaction mixture was transferred in triplicate to a new plate, followed by injection of 100 µL of Luciferase Assay Reagent (Promega cat # E1500) and measurement of luminescence on a GloMax Multi Plus luminometer (Promega; 0.5 second integration time). FIGS. 30A and 30B show that constructs with granzyme B cleavage sites showed a dose dependent increase in luminescence with increasing concentrations of enzyme, but control constructs lacking the P1 aspartic acid residue or containing the caspase-3/7 cleavage site (DEVDG) showed no dose dependent increase in luminescence.

**Table 12**

| **Vector Name** | **Biosensor Constructs** |
|---|---|
| pGLSGZB1 | pF9aAg3-V3P-CP358/GRIEADSE |
| pGLSGZB2 | pF9aAg3-V3P-CP358/GRIEAASE [pGLSGZB1 control] |
| pGLSGZB3 | pF9aAg3-V3P-CP358/GIETDSG |
| pGLSGZB4 | pF9aAg3-V3P-CP358/GIETASG [pGLSGZB3 control] |
| pGLSGZB5 | pF9aAg3-V3P-CP358/KSVGPDFG |
| pGLSGZB6 | pF9aAg3-V3P-CP358/KSVGPDFG [pGLSGZB5 control] |

### Example XX

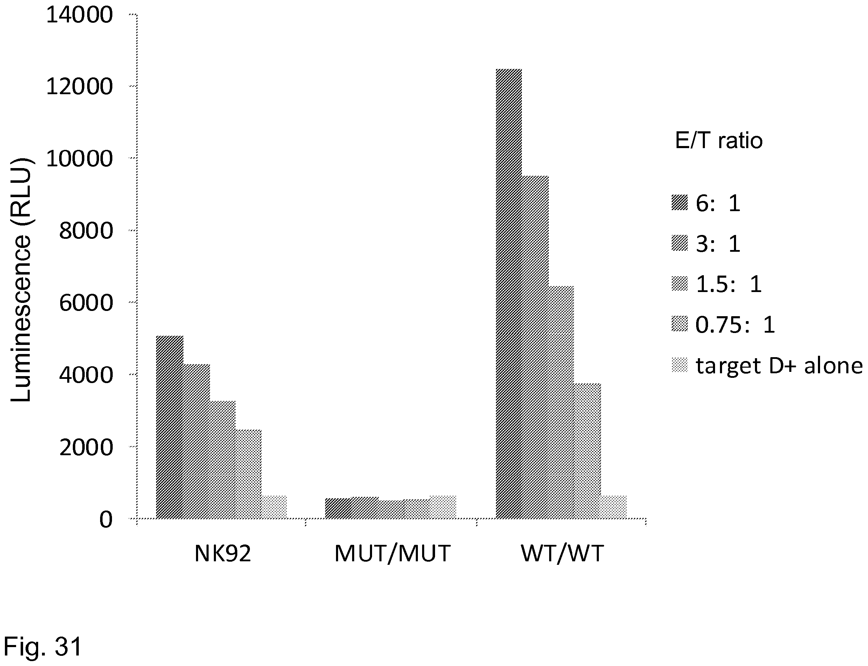

Herpes Saimiri Virus (HSV)-transformed, IL-2 activated NK cells from a healthy control patient (WT/WT) and a patient with a biallelic perforin mutation (Mut/Mut) were co-incubated at various E/T ratios with K562 target cells containing the granzyme B biosensor B+, C+, C-, D+, and D-, and were selected with G418. Cells were harvested and resuspended in 2% luciferin substrate and aliquoted into wells of a 96-well bottom plate at 2×10⁵, 1×10⁵, and 5×10⁴ cells per well, respectively. Plates were incubated at 37°C for 30 min and cooled at room temperature for 10 min. Luminescence was read on a Veritas luminometer plate reader. FIG. 31 shows granzyme B delivery induced luminescence in IL-2 activated NK cell lines from healthy control (WT/WT) and patient with biallelic perforin mutations (MUT/MUT). FIG. 31 shows the perforin dependence of the luminescent signal generated.

### Example XXI

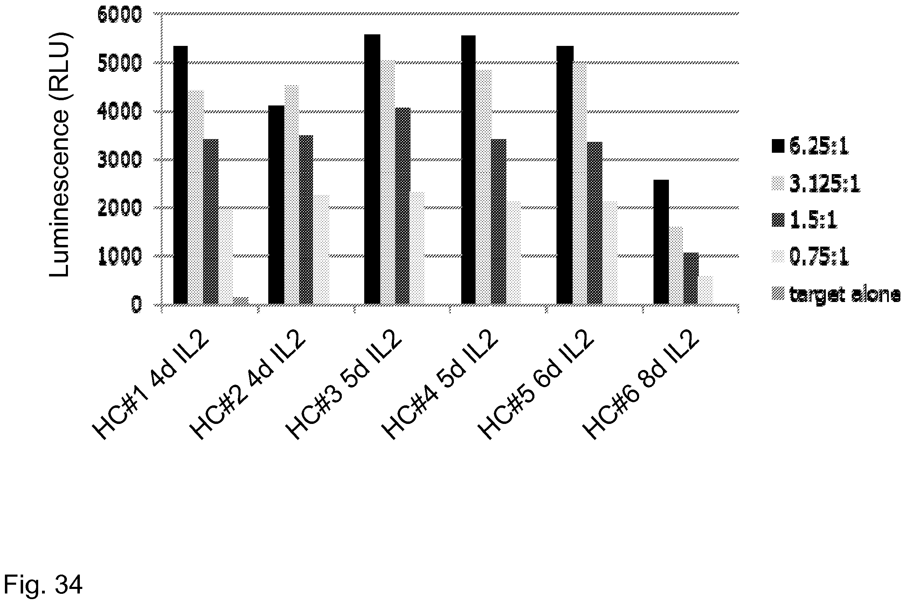

PBMC cells from healthy donors (n=5) were cultured with high dose IL-2 (1000 U/mL) for 4-8 days. At 4, 5, 6, and 8 days, the cells were co-incubated at various E/T ratios with K562 target cells transfected with the granzyme B biosensor D+ for 60 min at 37°C. Luminescence was measured on a Veritas luminometer as described above. FIG. 34 shows that NK cells are able to activate luminescence from a granzyme B biosensor after prolonged exposure to IL-2.

### Example XXII

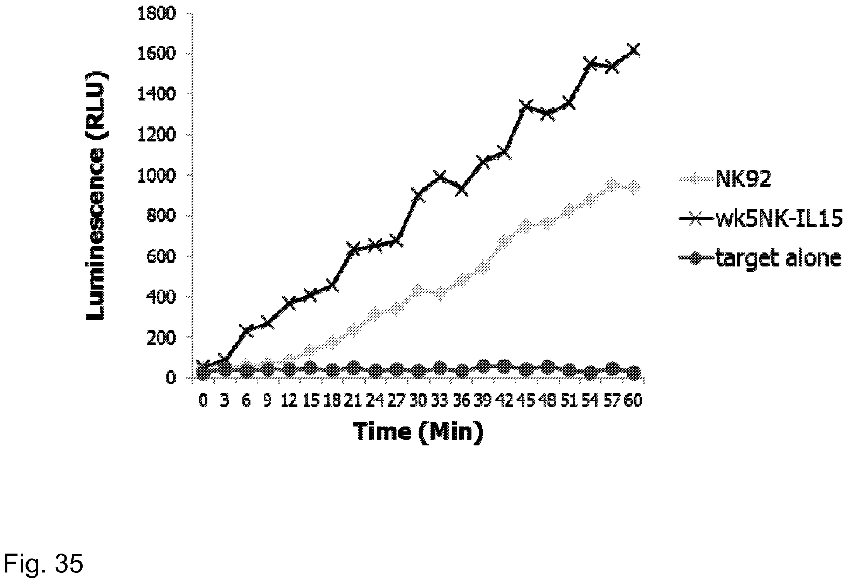

Human CD34 cells were isolated from umbilical cord blood (healthy donor) and cultured on stromal cells (MS5) in IL-15 and stem cell factor (SCF) for 5 weeks before testing cytotoxicity against K562 cells expressing granzyme B biosensor D+. Luminescence was measured as described above on a Biotek luminometer at 37°C. FIG. 35 shows that umbilical cord-derived, IL-15 stimulated cells are capable of releasing and delivering granzyme B to target cells containing a granzyme B biosensor and activating luminescence from the biosensor.

### Example XXIII

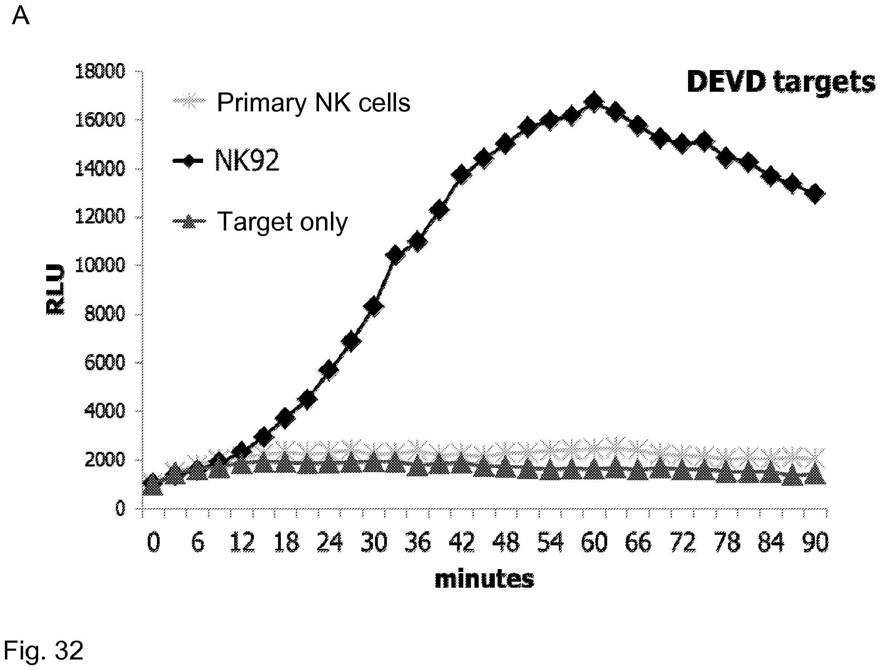

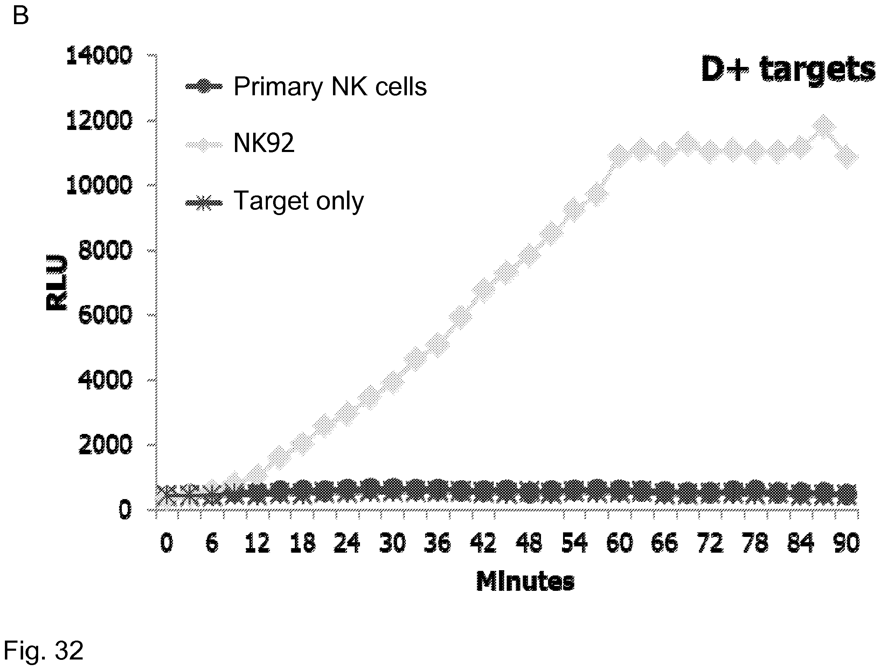

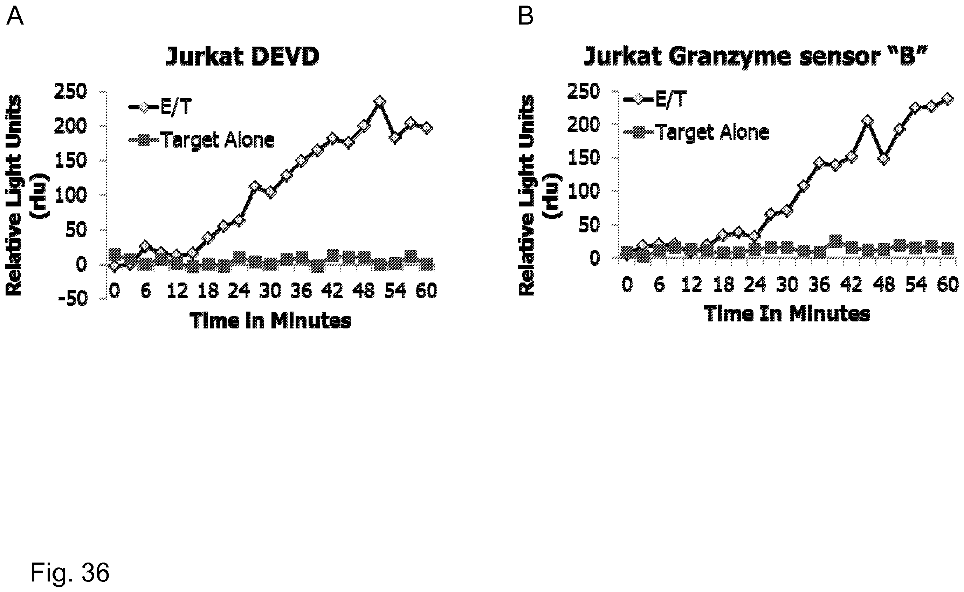

FIGS. 32A-B show the use of caspase 3/7 biosensor and the granzyme B biosensor with primary NK and NK92 cells. FIGS. 36A-B show the use of caspase 3/7 biosensor and the granzyme B biosensor in Jurkat target cells.

## Claims

1. A polynucleotide encoding a circularly-permuted thermostable luciferase comprising a peptide linker linking the original N- and C-termini of the non-permuted thermostable luciferase, wherein the peptide linker comprises a granzyme B recognition site, wherein the granzyme B recognition site is GRIEADSE (SEQ ID NO:80), KSVGPDFG (SEQ ID NO:81), or GIETDSG (SEQ ID NO:82), wherein the circularly-permuted thermostable luciferase has increased luminescence following cleavage by granzyme B when compared to an uncleaved circularly-permuted thermostable luciferase;
wherein the modified circularly-permuted thermostable luciferase has a substitution of at least one amino acid at positions 5, 17, 21, 23, 26, 39, 44, 51, 81, 101, 103, 110, 114, 115, 119, 123, 126, 128, 133, 137, 186, 191, 192, 193, 196, 208, 211, 214, 226, 228, 230, 233, 264, 273, 275, 286, 287, 294, 295, 297, 302, 303, 304, 306, 308, 309, 313, 324, 329, 331, 343, 348, 353, 364, 374, 385, 389, 409, 420, 426, 427, 428, 431, 449, 456, 460, 461, 465, 466, 468, 471, 473, 482, 484, 485, 489, 493, 494, 497, 503, 507, 509, 510, 513, 516, 517, 521, 522, 523, 526, 530, 533, 536, 537, 542, or 543 corresponding to SEQ ID NO: 2;
wherein the modified circularly-permuted thermostable luciferase has an enhanced response after interaction of the modified circularly-permuted thermostable luciferase with granzyme B relative to the parental circularly-permuted thermostable luciferase corresponding to SEQ ID NO: 2 in the presence of granzyme B.

2. The polynucleotide of claim 1, wherein the granzyme B recognition site is flanked by peptide linkers.

3. A vector comprising the polynucleotide of claim 1 or 2.

4. A cell comprising the polynucleotide of claim 1 or 2 or the vector of claim 3.

5. A kit comprising the polynucleotide of claim 1 or 2 or the vector of claim 3.

6. A circularly-permuted thermostable luciferase biosensor encoded by the polynucleotide of claim 1 or 2.

7. A method for detecting at least one of the presence or amount of active granzyme B in a cell or sample, the method comprising:
a) contacting the cell or sample with: (i) the polynucleotide of any one of claims 1-2, the vector of claim 3, or the biosensor of claim 6, and (ii) a substrate for the circularly-permuted thermostable luciferase biosensor; and
b) detecting luminescence in the cell or sample, wherein the granzyme B is capable of cleaving the peptide linker in the circularly-permuted thermostable luciferase biosensor, thereby detecting the presence or amount of active granzyme B.

8. A method for detecting at least one of the presence or amount of active granzyme B in a cell or sample, the method comprising:
a) contacting the cell or sample with:
i) the polynucleotide of any one of claims 1-2, the vector of claim 3, or the biosensor of claim 6;
ii) a substrate for the circularly-permuted thermostable luciferase biosensor; and
iii) a cytotoxic lymphocyte; and
b) detecting luminescence in the cell or sample, wherein the granzyme B is capable of cleaving the peptide linker of the circularly-permuted thermostable luciferase biosensor, thereby detecting the presence or amount of active granzyme B released from cytotoxic lymphocytes.

9. The method of claim 8, wherein the cytotoxic lymphocytes are patient or patient-derived cytotoxic T lymphocytes or cytokine-activated natural killer cells; and/or the detection of active granzyme B determines the cytotoxic function of the cytotoxic lymphocyte.

10. The method of any one of claims 8 to 9, wherein the detection of active granzyme B indicates cell-mediated cytotoxicity.

11. A method of screening for one or more modulators of granzyme B activity, the method comprising:
a) contacting a cell or sample with:
i) the polynucleotide of any one of claims 1-2, the vector of claim 3, or the biosensor of claim 6;
ii) one or more test agents; and
iii) a substrate for the circularly-permuted thermostable luciferase biosensor; and
b) detecting luminescence in the cell or sample, wherein the granzyme B is capable of cleaving the peptide linker of the circularly-permuted thermostable luciferase biosensor, thereby identifying modulators of granzyme B.

12. The method of claim 11, wherein the modulator is an inhibitor of granzyme B or an enhancer of granzyme B.

13. A method of screening for one or more modulators of a cytotoxic function of a cell, the method comprising:
a) contacting a target cell population with:
i) the polynucleotide of any one of claims 1-2, the vector of claim 3, or the biosensor of claim 6; and
ii) a substrate for the circularly-permuted thermostable luciferase biosensor;
b) contacting the target cell population and/or cells mediating cytotoxicity with one or more test agents; and
c) detecting luminescence in the target cell population, wherein the granzyme B released from the cells mediating cytotoxicity is capable of cleaving the peptide linker of the circularly-permuted thermostable luciferase biosensor in the target cell population, thereby identifying modulators of the cytotoxic function.

14. A method of detecting granzyme B activity in a cellular process, the method comprising:
a) contacting a cell or sample with:
i) one or more test agents;
ii) the polynucleotide of any one of claims 1-2, the vector of claim 3, or the biosensor of claim 6; and
iii) a substrate for the circularly-permuted thermostable luciferase biosensor; and
b) detecting luminescence in the cell or sample, wherein the granzyme B is capable of cleaving the peptide linker in the circularly-permuted thermostable luciferase biosensor, thereby detecting granzyme B activity.

15. A method of screening for one or more modulators of cytotoxic function, the method comprising:
a) contacting a target cell population with:
i) the polynucleotide of any one of claims 1-2, the vector of claim 3, or the biosensor of claim 6; and
ii) a substrate for the circularly-permuted thermostable luciferase biosensor; and
b) contacting the target cell population and/or cells mediating cytotoxicity with one or more test agents; and
c) detecting luminescence in the target cell population, wherein granzyme B released from the cells mediating cytotoxicity cleaves and activates the circularly-permuted thermostable luciferase biosensor in the target cell population, thereby identifying modulators of the cytotoxic function.

## Patentansprüche

1. Polynukleotid, welches eine kreisförmig permutierte thermostabile Luciferase codiert, umfassend einen Peptidlinker, der die ursprünglichen N- und C-Termini der nicht-permutierten thermostabilen Luciferase verknüpft, wobei der Peptidlinker eine Granzyme B-Erkennungsstelle umfasst, wobei die Granzyme B-Erkennungsstelle GRIEADSE (SEQ ID NO: 80), KSVGPDFG (SEQ ID NO: 81) oder GIETDSG (SEQ ID NO: 82) ist, wobei die kreisförmig permutierte thermostabile Luciferase nach Spaltung durch Granzyme B eine im Vergleich zu einer nicht-gespaltenen kreisförmig permutierten thermostabilen Luciferase gesteigerte Lumineszenz aufweist;
wobei die modifizierte kreisförmig permutierte thermostabile Luciferase eine Substitution von wenigstens einer Aminosäure an den Positionen 5, 17, 21, 23, 26, 39, 44, 51, 81, 101, 103, 110, 114, 115, 119, 123, 126, 128, 133, 137, 186, 191, 192, 193, 196, 208, 211, 214, 226, 228, 230, 233, 264, 273, 275, 286, 287, 294, 295, 297, 302, 303, 304, 306, 308, 309, 313, 324, 329, 331, 343, 348, 353, 364, 374, 385, 389, 409, 420, 426, 427, 428, 431, 449, 456, 460, 461, 465, 466, 468, 471, 473, 482, 484, 485, 489, 493, 494, 497, 503, 507, 509, 510, 513, 516, 517, 521, 522, 523, 526, 530, 533, 536, 537, 542 oder 543 gemäß SEQ ID NO: 2 aufweist;
wobei die modifizierte kreisförmig permutierte thermostabile Luciferase nach Interaktion der modifizierten kreisförmig permutierten thermostabilen Luciferase mit Granzyme B eine relativ zur elterlichen kreisförmig permutierten thermostabilen Luciferase entsprechend SEQ ID NO: 2 in Gegenwart von Granzyme B verstärkte Reaktion aufweist.

2. Polynukleotid nach Anspruch 1, wobei die Granzyme B-Erkennungsstelle durch Peptidlinker flankiert ist.

3. Vektor, welcher das Polynukleotid nach Anspruch 1 oder 2 umfasst.

4. Zelle, welche das Polynukleotid nach Anspruch 1 oder 2 oder den Vektor nach Anspruch 3 umfasst.

5. Kit, welches das Polynukleotid nach Anspruch 1 oder 2 oder den Vektor nach Anspruch 3 umfasst.

6. Kreisförmig-permutierte-thermostabile-Luciferase-Biosensor, welcher durch das Polynukleotid nach Anspruch 1 oder 2 codiert wird.

7. Verfahren zum Detektieren wenigstens eines vom Vorliegen von oder der Menge an aktivem Granzyme B in einer Zelle oder Probe, wobei das Verfahren umfasst:
a) Inkontaktbringen der Zelle oder Probe mit: (i) dem Polynukleotid nach irgendeinem der Ansprüche 1-2, dem Vektor nach Anspruch 3 oder dem Biosensor nach Anspruch 6 und (ii) einem Substrat für den kreisförmig-permutierte-thermostabile-Luciferase-Biosensor; und
b) Detektieren von Lumineszenz in der Zelle oder Probe, wobei das Granzyme B zum Spalten des Peptidlinkers in dem kreisförmig-permutierte-thermostabile-Luciferase-Biosensor in der Lage ist, und dadurch Detektieren des Vorliegens von oder der Menge an aktivem Granzyme B.

8. Verfahren zum Detektieren wenigstens eines vom Vorliegen von oder der Menge an aktivem Granzyme B in einer Zelle oder Probe, wobei das Verfahren umfasst:
a) Inkontaktbringen der Zelle oder Probe mit:
i) dem Polynukleotid nach irgendeinem der Ansprüche 1-2, dem Vektor nach Anspruch 3 oder dem Biosensor nach Anspruch 6;
ii) einem Substrat für den kreisförmig-permutiertethermostabile-Luciferase-Biosensor; und
iii) einem zytotoxischen Lymphozyten; und
b) Detektieren von Lumineszenz in der Zelle oder Probe, wobei das Granzyme B zum Spalten des Peptidlinkers des kreisförmig-permutierte-thermostabile-Luciferase-Biosensors in der Lage ist, und dadurch Detektieren des Vorliegens von oder der Menge an aktivem Granzyme B, das aus zytotoxischen Lymphozyten freigesetzt wird.

9. Verfahren nach Anspruch 8, wobei die zytotoxischen Lymphozyten zytotoxische T-Lymphozyten oder zytokinaktivierte natürliche Killerzellen vom Patienten oder vom Patienten abgeleitet sind; und/oder die Detektion von aktivem Granzyme B die zytotoxische Funktion des zytotoxischen Lymphozyten bestimmt.

10. Verfahren nach irgendeinem der Ansprüche 8 bis 9, wobei die Detektion von aktivem Granzyme B auf zellvermittelte Zytotoxizität hinweist.

11. Verfahren zum Screenen hinsichtlich eines oder mehrerer Modulatoren von Granzyme B-Aktivität, wobei das Verfahren umfasst:
a) Inkontaktbringen einer Zelle oder Probe mit:
i) dem Polynukleotid nach irgendeinem der Ansprüche 1-2, dem Vektor nach Anspruch 3 oder dem Biosensor nach Anspruch 6;
ii) einem oder mehreren Testagenzien; und
iii) einem Substrat für den kreisförmig-permutiertethermostabile-Luciferase-Biosensor; und
b) Detektieren von Lumineszenz in der Zelle oder Probe, wobei das Granzyme B zum Spalten des Peptidlinkers des kreisförmig-permutierte-thermostabile-Luciferase-Biosensors in der Lage ist, und dadurch Identifizieren von Modulatoren von Granzyme B.

12. Verfahren nach Anspruch 11, wobei der Modulator ein Inhibitor von Granzyme B oder ein Enhancer von Granzyme B ist.

13. Verfahren zum Screenen hinsichtlich eines oder mehrerer Modulatoren einer zytotoxischen Funktion einer Zelle, wobei das Verfahren umfasst:
a) Inkontaktbringen einer Zielzellpopulation mit:
i) dem Polynukleotid nach irgendeinem der Ansprüche 1-2, dem Vektor nach Anspruch 3 oder dem Biosensor nach Anspruch 6; und
ii) einem Substrat für den kreisförmig-permutiertethermostabile-Luciferase-Biosensor;
b) Inkontaktbringen der Zielzellpopulation und/oder von Zytotoxizität vermittelnden Zellen mit einem oder mehreren Testagenzien; und
c) Detektieren von Lumineszenz in der Zielzellpopulation, wobei das aus den Zytotoxizität vermittelnden Zellen freigesetzte Granzyme B zum Spalten des Peptidlinkers des kreisförmigpermutierte-thermostabile-Luciferase-Biosensors in der Zielzellpopulation in der Lage ist, und dadurch Identifizieren von Modulatoren der zytotoxischen Funktion.

14. Verfahren zum Detektieren von Granzyme B-Aktivität in einem zellulären Prozess, wobei das Verfahren umfasst:
a) Inkontaktbringen einer Zelle oder Probe mit:
i) einem oder mehreren Testagenzien;
ii) dem Polynukleotid nach irgendeinem der Ansprüche 1-2, dem Vektor nach Anspruch 3 oder dem Biosensor nach Anspruch 6; und
iii) einem Substrat für den kreisförmig-permutiertethermostabile-Luciferase-Biosensor; und
b) Detektieren von Lumineszenz in der Zelle oder Probe, wobei das Granzyme B zum Spalten des Peptidlinkers in dem kreisförmig-permutierte-thermostabile-Luciferase-Biosensor in der Lage ist, und dadurch Detektieren von Granzyme B-Aktivität.

15. Verfahren zum Screenen hinsichtlich eines oder mehrerer Modulatoren der zytotoxischen Funktion, wobei das Verfahren umfasst:
a) Inkontaktbringen einer Zielzellpopulation mit:
i) dem Polynukleotid nach irgendeinem der Ansprüche 1-2, dem Vektor nach Anspruch 3 oder dem Biosensor nach Anspruch 6; und
ii) einem Substrat für den kreisförmig-permutiertethermostabile-Luciferase-Biosensor; und
b) Inkontaktbringen der Zielzellpopulation und/oder von Zytotoxizität vermittelnden Zellen mit einem oder mehreren Testagenzien; und
c) Detektieren von Lumineszenz in der
Zielzellpopulation, wobei aus den Zytotoxizität vermittelnden Zellen freigesetztes Granzyme B den kreisförmig-permutierte-thermostabile-Luciferase-Biosensor in der Zielzellpopulation spaltet und aktiviert, und dadurch Identifizieren von Modulatoren der zytotoxischen Funktion.

## Revendications

1. Polynucléotide codant pour une luciférase thermostable à permutation circulaire comprenant un lieur peptidique liant les terminaisons N et C d'origine de la luciférase thermostable sans permutation, dans lequel le lieur peptidique comprend un site de reconnaissance de granzyme B, dans lequel le site de reconnaissance de granzyme B est GRIEADSE (SEQ ID N° : 80), KSVGPDFG (SEQ ID N° : 81) ou GIETDSG (SEQ ID N° : 82), dans lequel la luciférase thermostable à permutation circulaire présente une luminescence accrue après clivage par le granzyme B par comparaison avec une luciférase thermostable à permutation circulaire non clivée ;
dans lequel la luciférase thermostable à perméation circulaire modifiée présente une substitution d'au moins un acide aminé aux positions 5, 17, 21, 23, 26, 39, 44, 51, 81, 101, 103, 110, 114, 115, 119, 123, 126, 128, 133, 137, 186, 191, 192, 193, 196, 208, 211, 214, 226, 228, 230, 233, 264, 273, 275, 286, 287, 294, 295, 297, 302, 303, 304, 306, 308, 309, 313, 324, 329, 331, 343, 348, 353, 364, 374, 385, 389, 409, 420, 426, 427, 428, 431, 449, 456, 460, 461, 465, 466, 468, 471, 473, 482, 484, 485, 489, 493, 494, 497, 503, 507, 509, 510, 513, 516, 517, 521, 522, 523, 526, 530 , 533, 536, 537, 542 ou 543 correspondant à SEQ ID N° : 2 ;
dans lequel la luciférase thermostable à permutation circulaire modifiée présente une réponse améliorée après interaction de la luciférase thermostable à permutation circulaire modifiée avec le granzyme B par rapport à la luciférase thermostable à permutation circulaire parente correspondant à SEQ ID N° : 2 en présence de granzyme B.

2. Polynucléotide selon la revendication 1, dans lequel le site de reconnaissance de granzyme B est flanqué de lieurs peptidiques.

3. Vecteur comprenant le polynucléotide selon la revendication 1 ou 2.

4. Cellule comprenant le polynucléotide selon la revendication 1 ou 2 ou le vecteur selon la revendication 3.

5. Kit comprenant le polynucléotide selon la revendication 1 ou 2 ou le vecteur selon la revendication 3.

6. Biocapteur de luciférase thermostable à perméation circulaire codé par le polynucléotide selon la revendication 1 ou 2.

7. Procédé de détection d'au moins l'une de la présence ou de la quantité de granzyme B actif dans une cellule ou un échantillon, le procédé comprenant les étapes consistant à :
a) mettre en contact la cellule ou l'échantillon avec :
(i) le polynucléotide selon l'une quelconque des revendications 1-2, le vecteur selon la revendication 3, ou le biocapteur selon la revendication 6, et
(ii) un substrat pour le biocapteur de luciférase thermostable à permutation circulaire ; et
b) détecter une luminescence dans la cellule ou l'échantillon, dans lequel le granzyme B est apte à cliver le lieur peptidique dans le biocapteur de luciférase thermostable à permutation circulaire, permettant ainsi de détecter la présence ou la quantité de granzyme B actif.

8. Procédé pour détecter au moins une de la présence ou de la quantité de granzyme B actif dans une cellule ou un échantillon, le procédé comprenant les étapes consistant à :
a) mettre en contact la cellule ou l'échantillon avec :
i) le polynucléotide selon l'une quelconque des revendications 1-2, le vecteur selon la revendication 3 ou le biocapteur selon la revendication 6 ;
ii) un substrat pour le biocapteur de luciférase thermostable à permutation circulaire ; et
iii) un lymphocyte cytotoxique ; et
b) détecter une luminescence dans la cellule ou l'échantillon, dans lequel le granzyme B est apte à cliver le lieur peptidique du biocapteur de luciférase thermostable à permutation circulaire, permettant ainsi de détecter la présence ou la quantité de granzyme B actif libérée à partir de lymphocytes cytotoxiques.

9. Procédé selon la revendication 8, dans lequel les lymphocytes cytotoxiques sont des lymphocytes T cytotoxiques de patients ou dérivés de patients ou des cellules tueuses naturelles activées par cytokine ; et/ou la détection de granzyme B actif détermine la fonction cytotoxique du lymphocyte cytotoxique.

10. Procédé selon l'une quelconque des revendications 8 à 9, dans lequel la détection de granzyme B actif indique une cytotoxicité à médiation cellulaire.

11. Procédé de criblage d'un ou de plusieurs modulateurs d'activité de granzyme B, le procédé comprenant les étapes consistant à :
a) mettre en contact une cellule ou un échantillon avec :
i) le polynucléotide selon l'une quelconque des revendications 1-2, le vecteur selon la revendication 3 ou le biocapteur selon la revendication 6 ;
ii) un ou plusieurs agents de test ; et
iii) un substrat pour le biocapteur de luciférase thermostable à permutation circulaire ; et
b) détecter une luminescence dans la cellule ou l'échantillon, dans lequel le granzyme B est apte à cliver le lieur peptidique du biocapteur de luciférase thermostable à permutation circulaire, permettant ainsi d'identifier des modulateurs de granzyme B.

12. Procédé selon la revendication 11, dans lequel le modulateur est un inhibiteur de granzyme B ou un activateur de granzyme B.

13. Procédé de criblage d'un ou de plusieurs modulateurs d'une fonction cytotoxique d'une cellule, le procédé comprenant les étapes consistant à :
a) mettre en contact une population cellulaire cible avec :
i) le polynucléotide selon l'une quelconque des revendications 1-2, le vecteur selon la revendication 3, ou le biocapteur selon la revendication 6 ; et
ii) un substrat pour le biocapteur de luciférase thermostable à permutation circulaire ;
b) mettre en contact la population cellulaire cible et/ou des cellules médiatrices de cytotoxicité avec un ou plusieurs agents de test ; et
c) détecter une luminescence dans la population cellulaire cible, dans lequel le granzyme B libéré à partir des cellules médiatrices de cytotoxicité est apte à cliver le lieur peptidique du biocapteur de luciférase thermostable à perméation circulaire dans la population cellulaire cible, permettant ainsi d'identifier des modulateurs de la fonction cytotoxique.

14. Procédé de détection d'activité de granzyme B dans un processus cellulaire, le procédé comprenant les étapes consistant à :
a) mettre en contact une cellule ou un échantillon avec :
i) un ou plusieurs agents de test ;
ii) le polynucléotide selon l'une quelconque des revendications 1-2, le vecteur selon la revendication 3 ou le biocapteur selon la revendication 6 ; et
iii) un substrat pour le biocapteur de luciférase thermostable à permutation circulaire ; et
b) détecter une luminescence dans la cellule ou l'échantillon, dans lequel le granzyme B est apte à cliver le lieur peptidique dans le biocapteur de luciférase thermostable à permutation circulaire, permettant ainsi de détecter une activité de granzyme B.

15. Procédé de criblage d'un ou de plusieurs modulateurs de fonction cytotoxique, le procédé comprenant les étapes consistant à :
a) mettre en contact une population cellulaire cible avec :
i) le polynucléotide selon l'une quelconque des revendications 1-2, le vecteur selon la revendication 3 ou le biocapteur selon la revendication 6 ; et
ii) un substrat pour le biocapteur de luciférase thermostable à permutation circulaire ; et
b) mettre en contact la population cellulaire cible et/ou des cellules médiatrices de cytotoxicité avec un ou plusieurs agents de test ; et
c) détecter une luminescence dans la population cellulaire cible, dans lequel le granzyme B libéré à partir des cellules médiatrices de cytotoxicité clive et active le biocapteur de luciférase thermostable à permutation circulaire dans la population cellulaire cible, permettant ainsi d'identifier des modulateurs de la fonction cytotoxique.


### SEQUENCE LISTING

<110> PROMEGA CORPORATION
<120> MUTANT PROTEASE BIOSENSORS WITH ENHANCED DETECTION CHARACTERISTICS
<130> 209 734
<150> 13/105,648 <151> 2011-05-11
<150> 61/333,706 <151> 2010-05-11
<150> 61/470,845 <151> 2011-04-01
<150> 61/558,796 <151> 2011-11-11
<160> 85
<170> PatentIn version 3.3
<210> 1
   <211> 1635
   <212> DNA
   <213> Photuris pennsylvanica
<400> 1
   atggaagata aaaatatttt atatggacct gaaccatttc atcccttggc tgatgggacg      60
   gctggagaac agatgtttta cgcattatct cggtatgcag atatttcagg atgcattgca     120
   ttgacaaatg ctcatacaaa agaaaatgtt ttatatgaag aatttttaaa attgtcgtgt     180
   cgtttagcgg aaagttttaa aaagtatgga ttaaaacaaa acgacacaat agcggtgtgt     240
   agtgaaaatg gtttgcaatt tttccttcct ttaattgcat cattgtatct tggaataatt     300
   gcagcacctg ttagtgataa atacattgaa cgtgaattaa tacacagtct tggtattgta     360
   aaaccacgca taattttttg ttccaagaat acttttcaaa aagtactgaa tgtaaaatct      420
   aaattaaaat atgtagaaac tattattata ttagacttaa atgaagactt aggaggttat     480
   caatgcctca acaactttat ttctcaaaat tccgatatta atcttgacgt aaagaaattt     540
   aaaccaaatt cttttaatcg agacgatcag gttgcgttgg taatgttttc ttctggtaca     600
   actggtgttt ctaagggagt catgctaact cacaagaata ttgttgcacg attttctcat     660
   tgcaaagatc ctacttttgg taacgcaatt aatccaacga cagcaatttt aacagtaata     720
   ccattccacc atggttttgg tatgactacc acattaggat actttacttg tggattccga     780
   gttgctctaa tgcacacgtt tgaagaaaaa ctatttttac aatcattaca agattataaa     840
   gtggaaagta ctttacttgt accaacatta atggcatttt ttccaaaaag tgcgttagtt     900
   gaaaagtacg atttatcgca cttaaaagaa attgcatctg gtggcgcacc tttatcaaaa     960
   gaaattgggg agatggtgaa aaaacggttt aaattaaact ttgtcaggca agggtatgga    1020
   ttaacagaaa ccacttcggc tgttttaatt acaccggaca ctgacgtcag accgggatca   1080
   actggtaaaa tagtaccatt tcacgctgtt aaagttgtgg atcctacaac aggaaaaatt   1140
   ttggggccaa atgaaactgg agaattgtat tttaaaggcg acatgataat gaaaagttat   1200
   tataataatg aagaagctac taaagcaatt attaacaaag acggatggtt gcgctctggt   1260
   gatattgctt attatgacaa tgatggccat ttttatattg tggacaggct gaagtcatta   1320
   attaaatata aaggttatca ggttgcacct gctgaaattg agggaatact cttacaacac   1380
   ccgtatattg ttgatgccgg cgttactggt ataccggatg aagccgcggg cgagcttcca   1440
   gctgcaggtg ttgtagtaca gactggaaaa tatctaaacg aacaaatcgt acaaaatttt   1500
   gtttccagtc aagtttcaac agccaaatgg ctacgtggtg gggtgaaatt tttggatgaa   1560
   attcccaaag gatcaactgg aaaaattgac agaaaagtgt taagacaaat gtttgaaaaa   1620
   cacaaatcta agctg                                                    1635
<210> 2
   <211> 545
   <212> PRT
   <213> Photuris pennsylvanica
<400> 2
<210> 3
   <211> 1632
   <212> DNA
   <213> Artificial
<220>
   <223> Synthetic
<400> 3
   atggcagata agaatatttt atatgggccc gaaccatttt atcccttgga agatgggacg      60
   gctggagaac agatgtttga cgcattatct cgttatgcag ctattccggg ctgcatagca     120
   ttgacaaatg ctcatacaaa agaaaatgtt ttatatgaag agtttctgaa actgtcgtgt     180
   cgtttagcgg aaagttttaa aaagtatgga ttaaaacaaa acgacacaat agcggtgtgt     240
   agcgaaaata gtctgcaatt tttccttcct gtaattgcat cattgtatct tggaataatt     300
   gtggcacctg ttaacgataa atacattgaa cgtgaattaa tacacagtct tggtattgta     360
   aaaccacgca tagttttttg ctccaagaat acttttcaaa aagtactgaa tgtaaaatct      420
   aaattaaaat ctattgaaac tattattata ttagacttaa atgaagactt aggaggttat     480
   caatgcctca acaactttat ttctcaaaat tccgatagta atctggacgt aaaaaaattt     540
   aaaccctatt cttttaatcg agacgatcag gttgcgtcga ttatgttttc ttctggtaca     600
   actggtctgc cgaagggagt catgctaact cacaagaata ttgttgcacg attttctatt     660
   gcaaaagatc ctacttttgg taacgcaatt aatcccacgt cagcaatttt aacggtaata     720
   cctttccacc atggttttgg tatgatgacc acattaggat actttacttg tggattccga     780
   gttgttctaa tgcacacgtt tgaagaaaaa ctatttctac aatcattaca agattataaa     840
   gtggaaagta ctttacttgt accaacatta atggcatttc ttgcaaaaag tgcattagtt     900
   gaaaagtacg atttatcgca cttaaaagaa attgcatctg gtggcgcacc tttatcaaaa     960
   gaaattgggg agatggtgaa aaaacggttt aaattaaact ttgtcaggca agggtatgga    1020
   ttaacagaaa ccacttcggc tgttttaatt acaccgaaag gtgacgccaa accgggatca    1080
   actggtaaaa tagtaccatt acacgctgtt aaagttgtcg atcctacaac aggaaaaatt    1140
   ttggggccaa atgaacctgg agaattgtat tttaaaggcc cgatgataat gaagggttat    1200
   tataataatg aagaagctac taaagcaatt attgataatg acggatggtt gcgctctggt    1260
   gatattgctt attatgacaa tgatggccat ttttatattg tggacaggct gaagtcactg     1320
   attaaatata aaggttatca ggttgcacct gctgaaattg agggaatact cttacaacat    1380
   ccgtatattg ttgatgccgg cgttactggt ataccggatg aagccgcggg cgagcttcca    1440
   gctgcaggtg ttgtagtaca gactggaaaa tatctaaacg aacaaatcgt acaagattat    1500
   gttgccagtc aagtttcaac agccaaatgg ctacgtggtg gggtgaaatt tttggatgaa     1560
   attcccaaag gatcaactgg aaaaattgac agaaaagtgt taagacaaat gttagaaaaa    1620
   cacaccaatg gg                                                        1632
<210> 4
   <211> 544
   <212> PRT
   <213> Artificial
<220>
   <223> Synthetic
<400> 4
   Met Ala Asp Lys Asn Ile Leu Tyr Gly Pro Glu Pro Phe Tyr Pro Leu
   1 5 10 15
   Glu Asp Gly Thr Ala Gly Glu Gln Met Phe Asp Ala Leu Ser Arg Tyr
   20 25 30
   Ala Ala Ile Pro Gly Cys Ile Ala Leu Thr Asn Ala His Thr Lys Glu
   35 40 45
   Asn Val Leu Tyr Glu Glu Phe Leu Lys Leu Ser Cys Arg Leu Ala Glu
   50 55 60
   Ser Phe Lys Lys Tyr Gly Leu Lys Gln Asn Asp Thr Ile Ala Val Cys
   65 70 75 80
   Ser Glu Asn Ser Leu Gln Phe Phe Leu Pro Val Ile Ala Ser Leu Tyr
   85 90 95
   Leu Gly Ile Ile Val Ala Pro Val Asn Asp Lys Tyr Ile Glu Arg Glu
   100 105 110
   Leu Ile His Ser Leu Gly Ile Val Lys Pro Arg Ile Val Phe Cys Ser
   115 120 125
   Lys Asn Thr Phe Gln Lys Val Leu Asn Val Lys Ser Lys Leu Lys Ser
   130 135 140
   Ile Glu Thr Ile Ile Ile Leu Asp Leu Asn Glu Asp Leu Gly Gly Tyr
   145 150 155 160
   Gln Cys Leu Asn Asn Phe Ile Ser Gln Asn Ser Asp Ser Asn Leu Asp
   165 170 175
   Val Lys Lys Phe Lys Pro Tyr Ser Phe Asn Arg Asp Asp Gln Val Ala
   180 185 190
   Ser Ile Met Phe Ser Ser Gly Thr Thr Gly Leu Pro Lys Gly Val Met
   195 200 205
   Leu Thr His Lys Asn Ile Val Ala Arg Phe Ser Ile Ala Lys Asp Pro
   210 215 220
   Thr Phe Gly Asn Ala Ile Asn Pro Thr Ser Ala Ile Leu Thr Val Ile
   225 230 235 240
   Pro Phe His His Gly Phe Gly Met Met Thr Thr Leu Gly Tyr Phe Thr
   245 250 255
   Cys Gly Phe Arg Val Val Leu Met His Thr Phe Glu Glu Lys Leu Phe
   260 265 270
   Leu Gln Ser Leu Gln Asp Tyr Lys Val Glu Ser Thr Leu Leu Val Pro
   275 280 285
   Thr Leu Met Ala Phe Leu Ala Lys Ser Ala Leu Val Glu Lys Tyr Asp
   290 295 300
   Leu Ser His Leu Lys Glu Ile Ala Ser Gly Gly Ala Pro Leu Ser Lys
   305 310 315 320
   Glu Ile Gly Glu Met Val Lys Lys Arg Phe Lys Leu Asn Phe Val Arg
   325 330 335
   Gln Gly Tyr Gly Leu Thr Glu Thr Thr Ser Ala Val Leu Ile Thr Pro
   340 345 350
   Lys Gly Asp Ala Lys Pro Gly Ser Thr Gly Lys Ile Val Pro Leu His
   355 360 365
   Ala Val Lys Val Val Asp Pro Thr Thr Gly Lys Ile Leu Gly Pro Asn
   370 375 380
   Glu Pro Gly Glu Leu Tyr Phe Lys Gly Pro Met Ile Met Lys Gly Tyr
   385 390 395 400
   Tyr Asn Asn Glu Glu Ala Thr Lys Ala Ile Ile Asp Asn Asp Gly Trp
   405 410 415
   Leu Arg Ser Gly Asp Ile Ala Tyr Tyr Asp Asn Asp Gly His Phe Tyr
   420 425 430
   Ile Val Asp Arg Leu Lys Ser Leu Ile Lys Tyr Lys Gly Tyr Gln Val
   435 440 445
   Ala Pro Ala Glu Ile Glu Gly Ile Leu Leu Gln His Pro Tyr Ile Val
   450 455 460
   Asp Ala Gly Val Thr Gly Ile Pro Asp Glu Ala Ala Gly Glu Leu Pro
   465 470 475 480
   Ala Ala Gly Val Val Val Gln Thr Gly Lys Tyr Leu Asn Glu Gln Ile
   485 490 495
   Val Gln Asp Tyr Val Ala Ser Gln Val Ser Thr Ala Lys Trp Leu Arg
   500 505 510
   Gly Gly Val Lys Phe Leu Asp Glu Ile Pro Lys Gly Ser Thr Gly Lys
   515 520 525
   Ile Asp Arg Lys Val Leu Arg Gln Met Leu Glu Lys His Thr Asn Gly
       530                 535                 540
<210> 5
   <211> 1644
   <212> DNA
   <213> Artificial
<220>
   <223> Synthetic
<400> 5
   atgccgggat caactggtaa aatagtacca ttacacgctg ttaaagttgt cgatcctaca 60
   acaggaaaaa ttttggggcc aaatgaacct ggagaattgt attttaaagg cccgatgata 120
   atgaagggtt attataataa tgaagaagct actaaagcaa ttattgataa tgacggatgg 180
   ttgcgctctg gtgatattgc ttattatgac aatgatggcc atttttatat tgtggacagg 240
   ctgaagtcac tgattaaata taaaggttat caggttgcac ctgctgaaat tgagggaata 300
   ctcttacaac atccgtatat tgttgatgcc ggcgttactg gtataccgga tgaagccgcg 360
   ggcgagcttc cagctgcagg tgttgtagta cagactggaa aatatctaaa cgaacaaatc 420
   gtacaagatt atgttgccag tcaagtttca acagccaaat ggctacgtgg tggggtgaaa 480
   tttttggatg aaattcccaa aggatcaact ggaaaaattg acagaaaagt gttaagacaa 540
   atgttagaaa aacacaccaa tggatccgac gaggtggacg gaagcttgaa aaacatcctg 600
   tatggtccgg aaccgttcta cccactggaa gatggtaccg ctggtgaaca gatgtttgac 660
   gcattatctc gttatgcagc tattccgggc tgcatagcat tgacaaatgc tcatacaaaa 720
   gaaaatgttt tatatgaaga gtttctgaaa ctgtcgtgtc gtttagcgga aagttttaaa 780
   aagtatggat taaaacaaaa cgacacaata gcggtgtgta gcgaaaatag tctgcaattt 840
   ttccttcctg taattgcatc attgtatctt ggaataattg tggcacctgt taacgataaa 900
   tacattgaac gtgaattaat acacagtctt ggtattgtaa aaccacgcat agttttttgc 960
   tccaagaata cttttcaaaa agtactgaat gtaaaatcta aattaaaatc tattgaaact 1020
   attattatat tagacttaaa tgaagactta ggaggttatc aatgcctcaa caactttatt 1080
   tctcaaaatt ccgatagtaa tctggacgta aaaaaattta aaccctattc ttttaatcga 1140
   gacgatcagg ttgcgtcgat tatgttttct tctggtacaa ctggtctgcc gaagggagtc 1200
   atgctaactc acaagaatat tgttgcacga ttttctattg caaaagatcc tacttttggt 1260
   aacgcaatta atcccacgtc agcaatttta acggtaatac ctttccacca tggttttggt 1320
   atgatgacca cattaggata ctttacttgt ggattccgag ttgttctaat gcacacgttt 1380
   gaagaaaaac tatttctaca atcattacaa gattataaag tggaaagtac tttacttgta 1440
   ccaacattaa tggcatttct tgcaaaaagt gcattagttg aaaagtacga tttatcgcac 1500
   ttaaaagaaa ttgcatctgg tggcgcacct ttatcaaaag aaattgggga gatggtgaaa 1560
   aaacggttta aattaaactt tgtcaggcaa gggtatggat taacagaaac cacttcggct 1620
   gttttaatta caccgaaagg tgtt 1644
<210> 6
   <211> 548
   <212> PRT
   <213> Artificial
<220>
   <223> Synthetic
<400> 6
   Met Pro Gly Ser Thr Gly Lys Ile Val Pro Leu His Ala Val Lys Val
   1               5                   10                  15
   Val Asp Pro Thr Thr Gly Lys Ile Leu Gly Pro Asn Glu Pro Gly Glu
               20                  25                  30
   Leu Tyr Phe Lys Gly Pro Met Ile Met Lys Gly Tyr Tyr Asn Asn Glu
           35                  40                  45
   Glu Ala Thr Lys Ala Ile Ile Asp Asn Asp Gly Trp Leu Arg Ser Gly
       50                  55                  60
   Asp Ile Ala Tyr Tyr Asp Asn Asp Gly His Phe Tyr Ile Val Asp Arg
   65                  70                  75                  80
   Leu Lys Ser Leu Ile Lys Tyr Lys Gly Tyr Gln Val Ala Pro Ala Glu
                   85                  90                  95
   Ile Glu Gly Ile Leu Leu Gln His Pro Tyr Ile Val Asp Ala Gly Val
               100                 105                 110
   Thr Gly Ile Pro Asp Glu Ala Ala Gly Glu Leu Pro Ala Ala Gly Val
           115                 120                 125
   Val Val Gln Thr Gly Lys Tyr Leu Asn Glu Gln Ile Val Gln Asp Tyr
       130                 135                 140
   Val Ala Ser Gln Val Ser Thr Ala Lys Trp Leu Arg Gly Gly Val Lys
   145                 150                 155                 160
   Phe Leu Asp Glu Ile Pro Lys Gly Ser Thr Gly Lys Ile Asp Arg Lys
                   165                 170                 175
   Val Leu Arg Gln Met Leu Glu Lys His Thr Asn Gly Ser Asp Glu Val
               180                 185                 190
   Asp Gly Ser Leu Lys Asn Ile Leu Tyr Gly Pro Glu Pro Phe Tyr Pro
           195                 200                 205
   Leu Glu Asp Gly Thr Ala Gly Glu Gln Met Phe Asp Ala Leu Ser Arg
       210                 215                 220
   Tyr Ala Ala Ile Pro Gly Cys Ile Ala Leu Thr Asn Ala His Thr Lys
   225                 230                 235                 240
   Glu Asn Val Leu Tyr Glu Glu Phe Leu Lys Leu Ser Cys Arg Leu Ala
                   245                 250                 255
   Glu Ser Phe Lys Lys Tyr Gly Leu Lys Gln Asn Asp Thr Ile Ala Val
               260                 265                 270
   Cys Ser Glu Asn Ser Leu Gln Phe Phe Leu Pro Val Ile Ala Ser Leu
           275                 280                 285
   Tyr Leu Gly Ile Ile Val Ala Pro Val Asn Asp Lys Tyr Ile Glu Arg
       290                 295                 300
   Glu Leu Ile His Ser Leu Gly Ile Val Lys Pro Arg Ile Val Phe Cys
   305                 310                 315                 320
   Ser Lys Asn Thr Phe Gln Lys Val Leu Asn Val Lys Ser Lys Leu Lys
                   325                 330                 335
   Ser Ile Glu Thr Ile Ile Ile Leu Asp Leu Asn Glu Asp Leu Gly Gly
               340                 345                 350
   Tyr Gln Cys Leu Asn Asn Phe Ile Ser Gln Asn Ser Asp Ser Asn Leu
           355                 360                 365
   Asp Val Lys Lys Phe Lys Pro Tyr Ser Phe Asn Arg Asp Asp Gln Val
       370                 375                 380
   Ala Ser Ile Met Phe Ser Ser Gly Thr Thr Gly Leu Pro Lys Gly Val
   385                 390                 395                 400
   Met Leu Thr His Lys Asn Ile Val Ala Arg Phe Ser Ile Ala Lys Asp
                   405                 410                 415
   Pro Thr Phe Gly Asn Ala Ile Asn Pro Thr Ser Ala Ile Leu Thr Val
               420                 425                 430
   Ile Pro Phe His His Gly Phe Gly Met Met Thr Thr Leu Gly Tyr Phe
           435                 440                 445
   Thr Cys Gly Phe Arg Val Val Leu Met His Thr Phe Glu Glu Lys Leu
       450                 455                 460
   Phe Leu Gln Ser Leu Gln Asp Tyr Lys Val Glu Ser Thr Leu Leu Val
   465                 470                 475                 480
   Pro Thr Leu Met Ala Phe Leu Ala Lys Ser Ala Leu Val Glu Lys Tyr
                   485                 490                 495
   Asp Leu Ser His Leu Lys Glu Ile Ala Ser Gly Gly Ala Pro Leu Ser
               500                 505                 510
   Lys Glu Ile Gly Glu Met Val Lys Lys Arg Phe Lys Leu Asn Phe Val
           515                 520                 525
   Arg Gln Gly Tyr Gly Leu Thr Glu Thr Thr Ser Ala Val Leu Ile Thr
       530                 535                 540
   Pro Lys Gly Val
   545
<210> 7
   <211> 1644
   <212> DNA
   <213> Artificial
<220>
   <223> Synthetic
<400> 7
   atgccgggat caactggtaa aatagtacca ttacacgctg ttaaagttgt cgatcctaca     60
   acaggaaaaa ttttggggcc aaatgaacct ggagaattgt attttaaagg cccgatgata    120
   atgaagggtt attataataa tgaagaagct actaaagcaa ttattgataa tgacggatgg    180
   ttgcgctctg gtgatattgc ttattatgac aatgatggcc atttttatat tgtggacagg    240
   ctgaagtcac tgattaaata taaaggttat caggttgcac ctgctgaaat tgagggaata    300
   ctcttacaac atccgtatat tgttgatgcc ggcgttactg gtataccgga tgaagccgcg    360
   ggcgagcttc cagctgcagg tgttgtagta cagactggaa aatatctaaa cgaacaaatc    420
   gtacaagatt atgttgccag tcaagtttca atagccaaat ggctacgtgg tggggtgaaa    480
   tttttggatg aaattcccaa aggatcaact ggaaaaattg acagaaaagt gttaagacaa    540
   atgttagaaa aacacaccaa tggatccgac gaggtggacg gaagcttgaa aaacatcctg    600
   tatggtccgg aaccgttcta cccactggaa gatggtaccg ctggtgaaca gatgtttgac    660
   gcattatctc gttatgcagc tattccgggc tgcatagcat tgacaaatgc tcatacaaaa    720
   gaaaatgttt tatatgaaga gtttctgaaa ctgtcgtgtc gtttagcgga aagttttaaa    780
   aagtatggat taaaacaaaa cgacacaata gcggtgtgta gcgaaaatag tctgcaattt    840
   ttccttcctg taattgcatc attgtatctt ggaataattg tggcacctgt taacgataaa    900
   tacattgaac gtgaattaat acacagtctt ggtattgtaa aaccacgcat agttttttgc     960
   tccaagaata cttttcaaaa agtactgaat gtaaaatcta aattaaaatc tattgaaact   1020
   attattatat tagacttaaa tgaagactta ggaggttatc aatgcctcaa caactttatt   1080
   tctcaaaatt ccgatagtaa tctggacgta aaaaaattta aaccctattc ttttaatcga   1140
   gacgatcagg ttgcgtcgat tatgttttct tctggtacaa ctggtctgcc gaagggagtc   1200
   atgctaactc acaagaatat tgttgcacga ttttctattg caaaagatcc tacttttggt   1260
   aacgcaatta atcccacgtc agcaatttta acggtaatac ctttccacca tggttttggt   1320
   atgatgacca cattaggata ctttacttgt ggattccgag ttgttctaat gcacacgttt   1380
   gaagaaaaac tatttctaca atcattacaa gattataaag tggaaagtac tttacttgta   1440
   ccaacattaa tggcatttct tgcaaaaagt gcattagttg aaaagtacga tttatcgcac   1500
   ttaaaagaaa ttgcatctgg tggcgcacct ttatcaaaag aaattgggga gatggtgaaa   1560
   aaacggttta aattaaactt tgtcaggcaa gggtatggat taacagaaac cacttcggct   1620
   gttttaatta caccgaaagg tgtt                                         1644
<210> 8
   <211> 548
   <212> PRT
   <213> Artificial
<220>
   <223> Synthetic
<400> 8
   Met Pro Gly Ser Thr Gly Lys Ile Val Pro Leu His Ala Val Lys Val
   1               5                   10                  15
   Val Asp Pro Thr Thr Gly Lys Ile Leu Gly Pro Asn Glu Pro Gly Glu
               20                  25                  30
   Leu Tyr Phe Lys Gly Pro Met Ile Met Lys Gly Tyr Tyr Asn Asn Glu
           35                  40                  45
   Glu Ala Thr Lys Ala Ile Ile Asp Asn Asp Gly Trp Leu Arg Ser Gly
       50                  55                  60
   Asp Ile Ala Tyr Tyr Asp Asn Asp Gly His Phe Tyr Ile Val Asp Arg
   65                  70                  75                  80
   Leu Lys Ser Leu Ile Lys Tyr Lys Gly Tyr Gln Val Ala Pro Ala Glu
                   85                  90                  95
   Ile Glu Gly Ile Leu Leu Gln His Pro Tyr Ile Val Asp Ala Gly Val
               100                 105                 110
   Thr Gly Ile Pro Asp Glu Ala Ala Gly Glu Leu Pro Ala Ala Gly Val
           115                 120                 125
   Val Val Gln Thr Gly Lys Tyr Leu Asn Glu Gln Ile Val Gln Asp Tyr
       130                 135                 140
   Val Ala Ser Gln Val Ser Ile Ala Lys Trp Leu Arg Gly Gly Val Lys
   145                 150                 155                 160
   Phe Leu Asp Glu Ile Pro Lys Gly Ser Thr Gly Lys Ile Asp Arg Lys
                   165                 170                 175
   Val Leu Arg Gln Met Leu Glu Lys His Thr Asn Gly Ser Asp Glu Val
               180                 185                 190
   Asp Gly Ser Leu Lys Asn Ile Leu Tyr Gly Pro Glu Pro Phe Tyr Pro
           195                 200                 205
   Leu Glu Asp Gly Thr Ala Gly Glu Gln Met Phe Asp Ala Leu Ser Arg
       210                 215                 220
   Tyr Ala Ala Ile Pro Gly Cys Ile Ala Leu Thr Asn Ala His Thr Lys
   225                 230                 235                 240
   Glu Asn Val Leu Tyr Glu Glu Phe Leu Lys Leu Ser Cys Arg Leu Ala
                   245                 250                 255
   Glu Ser Phe Lys Lys Tyr Gly Leu Lys Gln Asn Asp Thr Ile Ala Val
               260                 265                 270
   Cys Ser Glu Asn Ser Leu Gln Phe Phe Leu Pro Val Ile Ala Ser Leu
           275                 280                 285
   Tyr Leu Gly Ile Ile Val Ala Pro Val Asn Asp Lys Tyr Ile Glu Arg
       290                 295                 300
   Glu Leu Ile His Ser Leu Gly Ile Val Lys Pro Arg Ile Val Phe Cys
   305                 310                 315                 320
   Ser Lys Asn Thr Phe Gln Lys Val Leu Asn Val Lys Ser Lys Leu Lys
                       325                 330                 335
   Ser Ile Glu Thr Ile Ile Ile Leu Asp Leu Asn Glu Asp Leu Gly Gly
                   340                 345                 350
   Tyr Gln Cys Leu Asn Asn Phe Ile Ser Gln Asn Ser Asp Ser Asn Leu
               355                 360                 365
   Asp Val Lys Lys Phe Lys Pro Tyr Ser Phe Asn Arg Asp Asp Gln Val
       370                 375                 380
   Ala Ser Ile Met Phe Ser Ser Gly Thr Thr Gly Leu Pro Lys Gly Val
   385                 390                 395                 400
   Met Leu Thr His Lys Asn Ile Val Ala Arg Phe Ser Ile Ala Lys Asp
                       405                 410                 415
   Pro Thr Phe Gly Asn Ala Ile Asn Pro Thr Ser Ala Ile Leu Thr Val
                   420                 425                 430
   Ile Pro Phe His His Gly Phe Gly Met Met Thr Thr Leu Gly Tyr Phe
               435                 440                 445
   Thr Cys Gly Phe Arg Val Val Leu Met His Thr Phe Glu Glu Lys Leu
       450                 455                 460
   Phe Leu Gln Ser Leu Gln Asp Tyr Lys Val Glu Ser Thr Leu Leu Val
   465                 470                 475                 480
   Pro Thr Leu Met Ala Phe Leu Ala Lys Ser Ala Leu Val Glu Lys Tyr
                       485                 490                 495
   Asp Leu Ser His Leu Lys Glu Ile Ala Ser Gly Gly Ala Pro Leu Ser
                   500                 505                 510
   Lys Glu Ile Gly Glu Met Val Lys Lys Arg Phe Lys Leu Asn Phe Val
               515                 520                 525
   Arg Gln Gly Tyr Gly Leu Thr Glu Thr Thr Ser Ala Val Leu Ile Thr
       530                 535                 540
   Pro Lys Gly Val
   545
<210> 9
   <211> 1644
   <212> DNA
   <213> Artificial
<220>
   <223> Synthetic
<400> 9
   atgccgggat caactggtaa aatagtacca ttacacgctg ttaaagttgt cgatcctaca      60
   acaggaaaaa ttttggggcc aaatgaacct ggagaattgt attttaaagg cccgatgata     120
   atgaagggtt attataataa tgaagaagct actaaagcaa ttattgataa tgacggatgg     180
   ttgcgctctg gtgatattgc ttattatgac aatgatggcc atttttatat tgtggacagg     240
   ctgaagtcac tgattaaata taaaggttat caggttgcac ctgctgaaat tgagggaata     300
   ctcttacaac atccgtatat tgttgatgcc ggcgttactg gtacaccgga tgaagccgcg     360
   ggcgagcttc cagctgcagg tgttgtagta cagactggaa aatatctaaa cgaacaaatc     420
   gtacaagatt atgttgccgg tcaagtttca atagccaaat ggctacgtgg tggggtgaaa     480
   tttttggatg aaattcccaa aggatcaact ggaaaaattg acagaaaagt gttaagacaa     540
   atgttagaaa aacacaccaa tggatccgac gaggtggacg gaagcttgaa aaacatcctg     600
   tatggtccgg aaccgttcta cccactggaa gatggtaccg ctggtgaaca gatgtttgac     660
   gcattatctc gttatgcagc tattccgggc tgcatagcat tgacaaatgc tcatacaaaa     720
   gaaaatgttt tatatgaaga gtttctgaaa ctgtcgtgtc gtttagcgga aagttttaaa     780
   aagtatggat taaaacaaaa cgacacaata gcggtgtgta gcgaaaatag tctgcaattt     840
   ttccttcctg taattgcatc attgtatctt ggaataattg tggcacctgt taacgataaa     900
   tacattgaac gtgaattaat acacagtctt ggtattgtaa aaccacgcat agttttttgc      960
   tccaagaata cttttcaaaa agtactgaat gtaaaatcta aattaaaatc tattgaaact    1020
   attattatat tagacttaaa tgaagactta ggaggttatc aatgcctcaa caactttatt    1080
   tctcaaaatt ccgatagtaa tctggacgta aaaaaattta aaccctattc ttttaatcga    1140
   gacgatcagg ttgcgccgat tatgttttct tctggtacaa ctggtctgcc gaagggagtc    1200
   atgctaactc acaagaatat tgttgcacga ttttctattg caaaagatcc tacttttggt    1260
   aacgcaatta atcccacgtc agcaatttta acggtaatac ctttccacca tggttttggt    1320
   atgatgacca cattaggata ctttacttgt ggattccgag ttgttctaat gcacacgttt    1380
   gaagaaaaac tatttctaca atcattacaa gattataaag tggaaagtac tttacttgta    1440
   ccaacattaa tggcatttct tgcaaaaagt gcattagttg aaaagtacga tttatcgcac    1500
   ttaaaagaaa ttgcatctgg tggcgcacct ttatcaaaag aaattgggga gatggtgaaa    1560
   aaacggttta aattaaactt tgtcaggcaa gggtatggat taacagaaac cacttcggct    1620
   gttttaatta caccgaaagg tgtt                                           1644
<210> 10
   <211> 548
   <212> PRT
   <213> Artificial
<220>
   <223> Synthetic
<400> 10
   Met Pro Gly Ser Thr Gly Lys Ile Val Pro Leu His Ala Val Lys Val
   1               5                   10                  15
   Val Asp Pro Thr Thr Gly Lys Ile Leu Gly Pro Asn Glu Pro Gly Glu
               20                  25                  30
   Leu Tyr Phe Lys Gly Pro Met Ile Met Lys Gly Tyr Tyr Asn Asn Glu
           35                  40                  45
   Glu Ala Thr Lys Ala Ile Ile Asp Asn Asp Gly Trp Leu Arg Ser Gly
       50                  55                  60
   Asp Ile Ala Tyr Tyr Asp Asn Asp Gly His Phe Tyr Ile Val Asp Arg
   65                  70                  75                  80
   Leu Lys Ser Leu Ile Lys Tyr Lys Gly Tyr Gln Val Ala Pro Ala Glu
                   85                  90                  95
   Ile Glu Gly Ile Leu Leu Gln His Pro Tyr Ile Val Asp Ala Gly Val
               100                 105                 110
   Thr Gly Thr Pro Asp Glu Ala Ala Gly Glu Leu Pro Ala Ala Gly Val
           115                 120                 125
   Val Val Gln Thr Gly Lys Tyr Leu Asn Glu Gln Ile Val Gln Asp Tyr
       130                 135                 140
   Val Ala Gly Gln Val Ser Ile Ala Lys Trp Leu Arg Gly Gly Val Lys
   145                 150                 155                 160
   Phe Leu Asp Glu Ile Pro Lys Gly Ser Thr Gly Lys Ile Asp Arg Lys
                   165                 170                 175
   Val Leu Arg Gln Met Leu Glu Lys His Thr Asn Gly Ser Asp Glu Val
               180                 185                 190
   Asp Gly Ser Leu Lys Asn Ile Leu Tyr Gly Pro Glu Pro Phe Tyr Pro
           195                 200                 205
   Leu Glu Asp Gly Thr Ala Gly Glu Gln Met Phe Asp Ala Leu Ser Arg
       210                 215                 220
   Tyr Ala Ala Ile Pro Gly Cys Ile Ala Leu Thr Asn Ala His Thr Lys
   225 230 235 240
   Glu Asn Val Leu Tyr Glu Glu Phe Leu Lys Leu Ser Cys Arg Leu Ala
   245 250 255
   Glu Ser Phe Lys Lys Tyr Gly Leu Lys Gln Asn Asp Thr Ile Ala Val
   260 265 270
   Cys Ser Glu Asn Ser Leu Gln Phe Phe Leu Pro Val Ile Ala Ser Leu
   275 280 285
   Tyr Leu Gly Ile Ile Val Ala Pro Val Asn Asp Lys Tyr Ile Glu Arg
   290 295 300
   Glu Leu Ile His Ser Leu Gly Ile Val Lys Pro Arg Ile Val Phe Cys
   305 310 315 320
   Ser Lys Asn Thr Phe Gln Lys Val Leu Asn Val Lys Ser Lys Leu Lys
   325 330 335
   Ser Ile Glu Thr Ile Ile Ile Leu Asp Leu Asn Glu Asp Leu Gly Gly
   340 345 350
   Tyr Gln Cys Leu Asn Asn Phe Ile Ser Gln Asn Ser Asp Ser Asn Leu
   355 360 365
   Asp Val Lys Lys Phe Lys Pro Tyr Ser Phe Asn Arg Asp Asp Gln Val
   370 375 380
   Ala Pro Ile Met Phe Ser Ser Gly Thr Thr Gly Leu Pro Lys Gly Val
   385 390 395 400
   Met Leu Thr His Lys Asn Ile Val Ala Arg Phe Ser Ile Ala Lys Asp
   405 410 415
   Pro Thr Phe Gly Asn Ala Ile Asn Pro Thr Ser Ala Ile Leu Thr Val
   420 425 430
   Ile Pro Phe His His Gly Phe Gly Met Met Thr Thr Leu Gly Tyr Phe
   435 440 445
   Thr Cys Gly Phe Arg Val Val Leu Met His Thr Phe Glu Glu Lys Leu
   450 455 460
   Phe Leu Gln Ser Leu Gln Asp Tyr Lys Val Glu Ser Thr Leu Leu Val
   465 470 475 480
   Pro Thr Leu Met Ala Phe Leu Ala Lys Ser Ala Leu Val Glu Lys Tyr
   485 490 495
   Asp Leu Ser His Leu Lys Glu Ile Ala Ser Gly Gly Ala Pro Leu Ser
   500 505 510
   Lys Glu Ile Gly Glu Met Val Lys Lys Arg Phe Lys Leu Asn Phe Val
   515 520 525
   Arg Gln Gly Tyr Gly Leu Thr Glu Thr Thr Ser Ala Val Leu Ile Thr
   530 535 540
   Pro Lys Gly Val
   545
<210> 11
   <211> 1644
   <212> DNA
   <213> Artificial
<220>
   <223> Synthetic
<400> 11
   atgccgggat caactggtaa aatagtacca ttacacgctg ttaaagttgt cgatcctaca      60
   acaggaaaaa ttttggggcc aaatgaacct ggagaattgt attttaaagg cccgatgata     120
   atgaagggtt attataataa tgaagaagct actaaagcaa ttattgataa tgacggatgg     180
   ttgcgctctg gtgatattgc ttattatgac aatgatggcc atttttatat tgtggacagg     240
   ctgaagtcac tgattaaata taaaggttat caggttgcac ctgctgaaat tgagggaata     300
   ctcttacaac atccgtatat tgttgatgcc ggcgttactg gtacaccgga tgaagccgcg     360
   ggcgagcttc cagctgcagg tgttgtagta cagactggaa aatatctaaa cgaacaaatc     420
   gtacaagatt atgttgccgg tcaagtttca atagccaaat ggctacgtgg tggggtgaaa     480
   tttttggatg aaattcccaa aggatcaact ggaaaaattg acagaaaagt gttaagacaa     540
   atgttagaaa aacacaccaa tggatccgac gaggtggacg gaagcttgaa aaacatcctg     600
   tatggtccgg aaccgttcta cccactggaa gatggtaccg ctggtgaaca gatgtttgac     660
   gcattatctc gttatgcagc tattccgggc tgcatagcat tgacaaatgc tcatacaaaa     720
   gaaaatgttt tatatgaaga gtttctgaaa ctgtcgtgtc gtttagcgga aagttttaaa     780
   aagtatggat taaaacaaaa cgacacaata gcggtgtgta gcgaaaatag tctgcaattt     840
   ttccttcctg taattgcatc attgtatctt ggaataattg tggcacctgt taacgataaa     900
   tacattgaac gtgaattaat acacagtctt ggtattgtaa aaccacgcat agttttttgc    960
   tccaagaata cttttcaaaa agtactgaat gtaaaatcta aattaaaatc tattgaaact    1020
   attattatat tagacttaaa tgaagactta ggaggttatc aatgcctcaa caactttatt    1080
   tctcaaaatt ccgatagtaa tctggacgta aaaaaattta aaccctattc ttttaatcga    1140
   gacgatcagg ttgcgtcgat tatgttttct tctggtacaa ctggtctgcc gaagggagtc    1200
   atgctaactc acaagaatat tgttgcacga ttttctattg caaaagatcc tacttttggt    1260
   aacgcaatta atcccacgtc agcaatttta acggtaatac ctttccacca tggttttggt    1320
   atgatgacca cattaggata ctttacttgt ggattccgag ttgttctaat gcacacgttt    1380
   gaagaaaaac tatttctaca atcattacaa gattataaag tggaaagtac tttacttgta    1440
   ccaacattaa tggcatttct tgcaaaaagt gcattagttg aaaagtacga tttatcgcac    1500
   ttaaaagaaa ttgcatctgg tggcgcacct ttatcaaaag aaattgggga gatggtgaaa    1560
   aaacggttta aattaaactt tgtcaggcaa gggtatggat taacagaaac cacttcggct    1620
   gttttaatta caccgaaagg tgtt                                           1644
<210> 12
   <211> 548
   <212> PRT
   <213> Artificial
<220>
   <223> Synthetic
<400> 12
   Met Pro Gly Ser Thr Gly Lys Ile Val Pro Leu His Ala Val Lys Val
   1               5                   10                  15
   Val Asp Pro Thr Thr Gly Lys Ile Leu Gly Pro Asn Glu Pro Gly Glu
               20                  25                  30
   Leu Tyr Phe Lys Gly Pro Met Ile Met Lys Gly Tyr Tyr Asn Asn Glu
           35                  40                  45
   Glu Ala Thr Lys Ala Ile Ile Asp Asn Asp Gly Trp Leu Arg Ser Gly
       50                  55                  60
   Asp Ile Ala Tyr Tyr Asp Asn Asp Gly His Phe Tyr Ile Val Asp Arg
   65                  70                  75                  80
   Leu Lys Ser Leu Ile Lys Tyr Lys Gly Tyr Gln Val Ala Pro Ala Glu
                   85                  90                  95
   Ile Glu Gly Ile Leu Leu Gln His Pro Tyr Ile Val Asp Ala Gly Val
               100                 105                 110
   Thr Gly Thr Pro Asp Glu Ala Ala Gly Glu Leu Pro Ala Ala Gly Val
   115 120 125
   Val Val Gln Thr Gly Lys Tyr Leu Asn Glu Gln Ile Val Gln Asp Tyr
   130 135 140
   Val Ala Gly Gln Val Ser Ile Ala Lys Trp Leu Arg Gly Gly Val Lys
   145 150 155 160
   Phe Leu Asp Glu Ile Pro Lys Gly Ser Thr Gly Lys Ile Asp Arg Lys
   165 170 175
   Val Leu Arg Gln Met Leu Glu Lys His Thr Asn Gly Ser Asp Glu Val
   180 185 190
   Asp Gly Ser Leu Lys Asn Ile Leu Tyr Gly Pro Glu Pro Phe Tyr Pro
   195 200 205
   Leu Glu Asp Gly Thr Ala Gly Glu Gln Met Phe Asp Ala Leu Ser Arg
   210 215 220
   Tyr Ala Ala Ile Pro Gly Cys Ile Ala Leu Thr Asn Ala His Thr Lys
   225 230 235 240
   Glu Asn Val Leu Tyr Glu Glu Phe Leu Lys Leu Ser Cys Arg Leu Ala
   245 250 255
   Glu Ser Phe Lys Lys Tyr Gly Leu Lys Gln Asn Asp Thr Ile Ala Val
   260 265 270
   Cys Ser Glu Asn Ser Leu Gln Phe Phe Leu Pro Val Ile Ala Ser Leu
   275 280 285
   Tyr Leu Gly Ile Ile Val Ala Pro Val Asn Asp Lys Tyr Ile Glu Arg
   290 295 300
   Glu Leu Ile His Ser Leu Gly Ile Val Lys Pro Arg Ile Val Phe Cys
   305 310 315 320
   Ser Lys Asn Thr Phe Gln Lys Val Leu Asn Val Lys Ser Lys Leu Lys
   325 330 335
   Ser Ile Glu Thr Ile Ile Ile Leu Asp Leu Asn Glu Asp Leu Gly Gly
   340 345 350
   Tyr Gln Cys Leu Asn Asn Phe Ile Ser Gln Asn Ser Asp Ser Asn Leu
   355 360 365
   Asp Val Lys Lys Phe Lys Pro Tyr Ser Phe Asn Arg Asp Asp Gln Val
       370                 375                 380
   Ala Ser Ile Met Phe Ser Ser Gly Thr Thr Gly Leu Pro Lys Gly Val
   385                 390                 395                 400
   Met Leu Thr His Lys Asn Ile Val Ala Arg Phe Ser Ile Ala Lys Asp
                   405                 410                 415
   Pro Thr Phe Gly Asn Ala Ile Asn Pro Thr Ser Ala Ile Leu Thr Val
               420                 425                 430
   Ile Pro Phe His His Gly Phe Gly Met Met Thr Thr Leu Gly Tyr Phe
           435                 440                 445
   Thr Cys Gly Phe Arg Val Val Leu Met His Thr Phe Glu Glu Lys Leu
       450                 455                 460
   Phe Leu Gln Ser Leu Gln Asp Tyr Lys Val Glu Ser Thr Leu Leu Val
   465                 470                 475                 480
   Pro Thr Leu Met Ala Phe Leu Ala Lys Ser Ala Leu Val Glu Lys Tyr
                   485                 490                 495
   Asp Leu Ser His Leu Lys Glu Ile Ala Ser Gly Gly Ala Pro Leu Ser
               500                 505                 510
   Lys Glu Ile Gly Glu Met Val Lys Lys Arg Phe Lys Leu Asn Phe Val
           515                 520                 525
   Arg Gln Gly Tyr Gly Leu Thr Glu Thr Thr Ser Ala Val Leu Ile Thr
       530                 535                 540
   Pro Lys Gly Val
   545
<210> 13
   <211> 1644
   <212> DNA
   <213> Artificial
<220>
   <223> Synthetic
<400> 13
   atgccgggat caactggtaa aatagtacca ttacacgctg ttaaagttgt cgatcctaca      60
   acaggaaaaa ttttggggcc aaatgaacct ggagaattgt attttaaagg cccgatgata     120
   atgaagggtt attataataa tgaagaagct actaaagcaa ttattgataa tgacggatgg 180
   ttgcgctctg gtgatattgc ttattatgac aatgatggcc atttttatat tgtggacagg 240
   ctgaagtcac tgattaaata taaaggttat caggttgcac ctgctgaaat tgagggaata 300
   ctcttacaac atccgtatat tgttgatgcc ggcgttactg gtataccgga tgaagccgcg 360
   ggcgagcttc cagctgcagg tgttgtagta cagactggaa aatatctaaa cgaacaaatc 420
   gtacaagatt atgttgccgg tcaagtttca atagccaaat ggctacgtgg tggggtgaaa 480
   tttttggatg aaattcccaa aggatcaact ggaaaaattg acagaaaagt gttaagacaa 540
   atgttagaaa aacacaccaa tggatccgac gaggtggacg gaagcttgaa aaacatcctg 600
   tatggtccgg aaccgttcta cccactggaa gatggtaccg ctggtgaaca gatgtttgac 660
   gcattatctc gttatgcagc tattccgggc tgcatagcat tgacaaatgc tcatacaaaa 720
   gaaaatgttt tatatgaaga gtttctgaaa ctgtcgtgtc gtttagcgga aagttttaaa 780
   aagtatggat taaaacaaaa cgacacaata gcggtgtgta gcgaaaatag tctgcaattt 840
   ttccttcctg taattgcatc attgtatctt ggaataattg tggcacctgt taacgataaa 900
   tacattgaac gtgaattaat acacagtctt ggtattgtaa aaccacgcat agttttttgc 960
   tccaagaata cttttcaaaa agtactgaat gtaaaatcta aattaaaatc tattgaaact 1020
   attattatat tagacttaaa tgaagactta ggaggttatc aatgcctcaa caactttatt 1080
   tctcaaaatt ccgatagtaa tctggacgta aaaaaattta aaccctattc ttttaatcga 1140
   gacgatcagg ttgcgccgat tatgttttct tctggtacaa ctggtctgcc gaagggagtc 1200
   atgctaactc acaagaatat tgttgcacga ttttctattg caaaagatcc tacttttggt 1260
   aacgcaatta atcccacgtc agcaatttta acggtaatac ctttccacca tggttttggt 1320
   atgatgacca cattaggata ctttacttgt ggattccgag ttgttctaat gcacacgttt 1380
   gaagaaaaac tatttctaca atcattacaa gattataaag tggaaagtac tttacttgta 1440
   ccaacattaa tggcatttct tgcaaaaagt gcattagttg aaaagtacga tttatcgcac 1500
   ttaaaagaaa ttgcatctgg tggcgcacct ttatcaaaag aaattgggga gatggtgaaa 1560
   aaacggttta aattaaactt tgtcaggcaa gggtatggat taacagaaac cacttcggct 1620
   gttttaatta caccgaaagg tgtt 1644
<210> 14
   <211> 548
   <212> PRT
   <213> Artificial
<220>
   <223> Synthetic
<400> 14
   Met Pro Gly Ser Thr Gly Lys Ile Val Pro Leu His Ala Val Lys Val
   1               5                   10                  15
   Val Asp Pro Thr Thr Gly Lys Ile Leu Gly Pro Asn Glu Pro Gly Glu
               20                  25                  30
   Leu Tyr Phe Lys Gly Pro Met Ile Met Lys Gly Tyr Tyr Asn Asn Glu
           35                  40                  45
   Glu Ala Thr Lys Ala Ile Ile Asp Asn Asp Gly Trp Leu Arg Ser Gly
       50                  55                  60
   Asp Ile Ala Tyr Tyr Asp Asn Asp Gly His Phe Tyr Ile Val Asp Arg
   65                  70                  75                  80
   Leu Lys Ser Leu Ile Lys Tyr Lys Gly Tyr Gln Val Ala Pro Ala Glu
                   85                  90                  95
   Ile Glu Gly Ile Leu Leu Gln His Pro Tyr Ile Val Asp Ala Gly Val
               100                 105                 110
   Thr Gly Ile Pro Asp Glu Ala Ala Gly Glu Leu Pro Ala Ala Gly Val
           115                 120                 125
   Val Val Gln Thr Gly Lys Tyr Leu Asn Glu Gln Ile Val Gln Asp Tyr
       130                 135                 140
   Val Ala Gly Gln Val Ser Ile Ala Lys Trp Leu Arg Gly Gly Val Lys
   145                 150                 155                 160
   Phe Leu Asp Glu Ile Pro Lys Gly Ser Thr Gly Lys Ile Asp Arg Lys
                   165                 170                 175
   Val Leu Arg Gln Met Leu Glu Lys His Thr Asn Gly Ser Asp Glu Val
               180                 185                 190
   Asp Gly Ser Leu Lys Asn Ile Leu Tyr Gly Pro Glu Pro Phe Tyr Pro
           195                 200                 205
   Leu Glu Asp Gly Thr Ala Gly Glu Gln Met Phe Asp Ala Leu Ser Arg
       210                 215                 220
   Tyr Ala Ala Ile Pro Gly Cys Ile Ala Leu Thr Asn Ala His Thr Lys
   225                 230                 235                 240
   Glu Asn Val Leu Tyr Glu Glu Phe Leu Lys Leu Ser Cys Arg Leu Ala
                   245                 250                 255
   Glu Ser Phe Lys Lys Tyr Gly Leu Lys Gln Asn Asp Thr Ile Ala Val
               260                 265                 270
   Cys Ser Glu Asn Ser Leu Gln Phe Phe Leu Pro Val Ile Ala Ser Leu
           275                 280                 285
   Tyr Leu Gly Ile Ile Val Ala Pro Val Asn Asp Lys Tyr Ile Glu Arg
       290                 295                 300
   Glu Leu Ile His Ser Leu Gly Ile Val Lys Pro Arg Ile Val Phe Cys
   305                 310                 315                 320
   Ser Lys Asn Thr Phe Gln Lys Val Leu Asn Val Lys Ser Lys Leu Lys
                   325                 330                 335
   Ser Ile Glu Thr Ile Ile Ile Leu Asp Leu Asn Glu Asp Leu Gly Gly
               340                 345                 350
   Tyr Gln Cys Leu Asn Asn Phe Ile Ser Gln Asn Ser Asp Ser Asn Leu
           355                 360                 365
   Asp Val Lys Lys Phe Lys Pro Tyr Ser Phe Asn Arg Asp Asp Gln Val
       370                 375                 380
   Ala Pro Ile Met Phe Ser Ser Gly Thr Thr Gly Leu Pro Lys Gly Val
   385                 390                 395                 400
   Met Leu Thr His Lys Asn Ile Val Ala Arg Phe Ser Ile Ala Lys Asp
                   405                 410                 415
   Pro Thr Phe Gly Asn Ala Ile Asn Pro Thr Ser Ala Ile Leu Thr Val
               420                 425                 430
   Ile Pro Phe His His Gly Phe Gly Met Met Thr Thr Leu Gly Tyr Phe
           435                 440                 445
   Thr Cys Gly Phe Arg Val Val Leu Met His Thr Phe Glu Glu Lys Leu
       450                 455                 460
   Phe Leu Gln Ser Leu Gln Asp Tyr Lys Val Glu Ser Thr Leu Leu Val
   465                 470                 475                 480
   Pro Thr Leu Met Ala Phe Leu Ala Lys Ser Ala Leu Val Glu Lys Tyr
                   485                 490                 495
   Asp Leu Ser His Leu Lys Glu Ile Ala Ser Gly Gly Ala Pro Leu Ser
               500                 505                 510
   Lys Glu Ile Gly Glu Met Val Lys Lys Arg Phe Lys Leu Asn Phe Val
           515                 520                 525
   Arg Gln Gly Tyr Gly Leu Thr Glu Thr Thr Ser Ala Val Leu Ile Thr
       530                 535                 540
   Pro Lys Gly Val
   545
<210> 15
   <211> 5
   <212> PRT
   <213> Artificial
<220>
   <223> Synthetic
<400> 15
   Leu Glu Thr Asp Gly
   1               5
<210> 16
   <211> 7
   <212> PRT
   <213> Artificial
<220>
   <223> Synthetic
<400> 16
   Glu Asn Leu Tyr Phe Gln Ser
   1               5
<210> 17
   <211> 1665
   <212> DNA
   <213> Artificial
<220>
   <223> Synthetic
<400> 17
   atgacgtcag caattttaac ggtaatacct ttccaccatg gttttggtat gatgaccaca      60
   ttaggatact ttacttgtgg attccgagtt gttctaatgc acacgtttga agaaaaacta     120
   tttctacaat cattacaaga ttataaagtg gaaagtactt tacttgtacc aacattaatg     180
   gcatttcttg caaaaagtgc attagttgaa aagtacgatt tatcgcactt aaaagaaatt     240
   gcatctggtg gcgcaccttt atcaaaagaa attggggaga tggtgaaaaa acggtttaaa     300
   ttaaactttg tcaggcaagg gtatggatta acagaaacca cttcggctgt tttaattaca     360
   ccgaaaggtg acgccaaacc gggatcaact ggtaaaatag taccattaca cgctgttaaa    420
   gttgtcgatc ctacaacagg aaaaattttg gggccaaatg aacctggaga attgtatttt    480
   aaaggcccga tgataatgaa gggttattat aataatgaag aagctactaa agcaattatt    540
   gataatgacg gatggttgcg ctctggtgat attgcttatt atgacaatga tggccatttt    600
   tatattgtgg acaggctgaa gtcactgatt aaatataaag gttatcaggt tgcacctgct    660
   gaaattgagg gaatactctt acaacatccg tatattgttg atgccggcgt tactggtata    720
   ccggatgaag ccgcgggcga gcttccagct gcaggtgttg tagtacagac tggaaaatat    780
   ctaaacgaac aaatcgtaca agattatgtt gccagtcaag tttcaacagc caaatggcta    840
   cgtggtgggg tgaaattttt ggatgaaatt cccaaaggat caactggaaa aattgacaga    900
   aaagtgttaa gacaaatgtt agaaaaacac accaatggct cgagcgacga ggtggacggg    960
   agctccggtg ataagaatat tttatatggg cccgaaccat tttatccctt ggaagatggg   1020
   acggctggag aacagatgtt tgacgcatta tctcgttatg cagctattcc gggctgcata   1080
   gcattgacaa atgctcatac aaaagaaaat gttttatatg aagagtttct gaaactgtcg   1140
   tgtcgtttag cggaaagttt taaaaagtat ggattaaaac aaaacgacac aatagcggtg   1200
   tgtagcgaaa atagtctgca atttttcctt cctgtaattg catcattgta tcttggaata   1260
   attgtggcac ctgttaacga taaatacatt gaacgtgaat taatacacag tcttggtatt   1320
   gtaaaaccac gcatagtttt ttgctccaag aatacttttc aaaaagtact gaatgtaaaa   1380
   tctaaattaa aatctattga aactattatt atattagact taaatgaaga cttaggaggt   1440
   tatcaatgcc tcaacaactt tatttctcaa aattccgata gtaatctgga cgtaaaaaaa   1500
   tttaaaccct attcttttaa tcgagacgat caggttgcgt cgattatgtt ttcttctggt   1560
   acaactggtc tgccgaaggg agtcatgcta actcacaaga atattgttgc acgattttct   1620
   attgcaaaag atcctacttt tggtaacgca attaatcccg tttaa                  1665
<210> 18
   <211> 554
   <212> PRT
   <213> Artificial
<220>
   <223> Synthetic
<400> 18
   Met Thr Ser Ala Ile Leu Thr Val Ile Pro Phe His His Gly Phe Gly
   1               5                   10                  15
   Met Met Thr Thr Leu Gly Tyr Phe Thr Cys Gly Phe Arg Val Val Leu
               20                  25                  30
   Met His Thr Phe Glu Glu Lys Leu Phe Leu Gln Ser Leu Gln Asp Tyr
           35                  40                  45
   Lys Val Glu Ser Thr Leu Leu Val Pro Thr Leu Met Ala Phe Leu Ala
       50                  55                  60
   Lys Ser Ala Leu Val Glu Lys Tyr Asp Leu Ser His Leu Lys Glu Ile
   65                  70                  75                  80
   Ala Ser Gly Gly Ala Pro Leu Ser Lys Glu Ile Gly Glu Met Val Lys
                   85                  90                  95
   Lys Arg Phe Lys Leu Asn Phe Val Arg Gln Gly Tyr Gly Leu Thr Glu
               100                 105                 110
   Thr Thr Ser Ala Val Leu Ile Thr Pro Lys Gly Asp Ala Lys Pro Gly
           115                 120                 125
   Ser Thr Gly Lys Ile Val Pro Leu His Ala Val Lys Val Val Asp Pro
       130                 135                 140
   Thr Thr Gly Lys Ile Leu Gly Pro Asn Glu Pro Gly Glu Leu Tyr Phe
   145                 150                 155                 160
   Lys Gly Pro Met Ile Met Lys Gly Tyr Tyr Asn Asn Glu Glu Ala Thr
                   165                 170                 175
   Lys Ala Ile Ile Asp Asn Asp Gly Trp Leu Arg Ser Gly Asp Ile Ala
               180                 185                 190
   Tyr Tyr Asp Asn Asp Gly His Phe Tyr Ile Val Asp Arg Leu Lys Ser
           195                 200                 205
   Leu Ile Lys Tyr Lys Gly Tyr Gln Val Ala Pro Ala Glu Ile Glu Gly
       210                 215                 220
   Ile Leu Leu Gln His Pro Tyr Ile Val Asp Ala Gly Val Thr Gly Ile
   225                 230                 235                 240
   Pro Asp Glu Ala Ala Gly Glu Leu Pro Ala Ala Gly Val Val Val Gln
                   245                 250                 255
   Thr Gly Lys Tyr Leu Asn Glu Gln Ile Val Gln Asp Tyr Val Ala Ser
               260                 265                 270
   Gln Val Ser Thr Ala Lys Trp Leu Arg Gly Gly Val Lys Phe Leu Asp
           275                 280                 285
   Glu Ile Pro Lys Gly Ser Thr Gly Lys Ile Asp Arg Lys Val Leu Arg
   290 295 300
   Gln Met Leu Glu Lys His Thr Asn Gly Ser Ser Asp Glu Val Asp Gly
   305 310 315 320
   Ser Ser Gly Asp Lys Asn Ile Leu Tyr Gly Pro Glu Pro Phe Tyr Pro
   325 330 335
   Leu Glu Asp Gly Thr Ala Gly Glu Gln Met Phe Asp Ala Leu Ser Arg
   340 345 350
   Tyr Ala Ala Ile Pro Gly Cys Ile Ala Leu Thr Asn Ala His Thr Lys
   355 360 365
   Glu Asn Val Leu Tyr Glu Glu Phe Leu Lys Leu Ser Cys Arg Leu Ala
   370 375 380
   Glu Ser Phe Lys Lys Tyr Gly Leu Lys Gln Asn Asp Thr Ile Ala Val
   385 390 395 400
   Cys Ser Glu Asn Ser Leu Gln Phe Phe Leu Pro Val Ile Ala Ser Leu
   405 410 415
   Tyr Leu Gly Ile Ile Val Ala Pro Val Asn Asp Lys Tyr Ile Glu Arg
   420 425 430
   Glu Leu Ile His Ser Leu Gly Ile Val Lys Pro Arg Ile Val Phe Cys
   435 440 445
   Ser Lys Asn Thr Phe Gln Lys Val Leu Asn Val Lys Ser Lys Leu Lys
   450 455 460
   Ser Ile Glu Thr Ile Ile Ile Leu Asp Leu Asn Glu Asp Leu Gly Gly
   465 470 475 480
   Tyr Gln Cys Leu Asn Asn Phe Ile Ser Gln Asn Ser Asp Ser Asn Leu
   485 490 495
   Asp Val Lys Lys Phe Lys Pro Tyr Ser Phe Asn Arg Asp Asp Gln Val
   500 505 510
   Ala Ser Ile Met Phe Ser Ser Gly Thr Thr Gly Leu Pro Lys Gly Val
   515 520 525
   Met Leu Thr His Lys Asn Ile Val Ala Arg Phe Ser Ile Ala Lys Asp
   530 535 540
   Pro Thr Phe Gly Asn Ala Ile Asn Pro Val
   545                 550
<210> 19
   <211> 1656
   <212> DNA
   <213> Artificial
<220>
   <223> Synthetic
<400> 19
   atgcctggcg cagtaggcaa ggtggtgccc ttcttcgagg ctaaggtggt ggacttggac      60
   actggtaaga cactgggtgt gaaccagcgc ggcgagctgt gcgtccgtgg ccccatgatc     120
   atgagcggct acgttaacaa ccccgaggct acaaacgctc tcatcgacaa ggacggctgg     180
   ctgcacagcg gcgacatcgc ctactgggac gaggacgagc acttcttcat cgtggaccgg     240
   ctgaagagcc tgatcaaata caagggctac caggtagccc cagccgaact ggagagcatc     300
   ctgctgcaac accccaacat cttcgacgcc ggggtcgccg gcctgcccga cgacgatgcc     360
   ggcgagctgc ccgccgcagt cgtcgtgctg gaacacggta aaaccatgac cgagaaggag     420
   atcgtggact atgtggccag ccaggttaca accgccaaga agctgcgcgg tggtgttgtg     480
   ttcgtggacg aggtgcctaa aggactgacc ggcaagttgg acgcccgcaa gatccgcgag     540
   attctcatta aggccaagaa gggctcgagc ctggagaccg acggcagctc cggtgccaaa     600
   aacattaaga agggcccagc gccattctac ccactcgaag acgggaccgc cggcgagcag     660
   ctgcacaaag ccatgaagcg ctacgccctg gtgcccggca ccatcgcctt taccgacgca     720
   catatcgagg tggacattac ctacgccgag tacttcgaga tgagcgttcg gctggcagaa     780
   gctatgaagc gctatgggct gaatacaaac catcggatcg tggtgtgcag cgagaatagc     840
   ttgcagttct tcatgcccgt gttgggtgcc ctgttcatcg gtgtggctgt ggccccagct     900
   aacgacatct acaacgagcg cgagctgctg aacagcatgg gcatcagcca gcccaccgtc     960
   gtattcgtga gcaagaaagg gctgcaaaag atcctcaacg tgcaaaagaa gctaccgatc    1020
   atacaaaaga tcatcatcat ggatagcaag accgactacc agggcttcca aagcatgtac    1080
   accttcgtga cttcccattt gccacccggc ttcaacgagt acgacttcgt gcccgagagc    1140
   ttcgaccggg acaaaaccat cgccctgatc atgaacagta gtggcagtac cggattgccc    1200
   aagggcgtag ccctaccgca ccgcaccgct tgtgtccgat tcagtcatgc ccgcgacccc    1260
   atcttcggca accagatcat ccccgacacc gctatcctca gcgtggtgcc atttcaccac    1320
   ggcttcggca tgttcaccac gctgggctac ttgatctgcg gctttcgggt cgtgctcatg    1380
   taccgcttcg aggaggagct attcttgcgc agcttgcaag actataagat tcaatctgcc    1440
   ctgctggtgc ccacactatt tagcttcttc gctaagagca ctctcatcga caagtacgac   1500
   ctaagcaact tgcacgagat cgccagcggc ggggcgccgc tcagcaagga ggtaggtgag   1560
   gccgtggcca aacgcttcca cctaccaggc atccgccagg gctacggcct gacagaaaca   1620
   accagcgcca ttctgatcac tccagaaggg gtttaa                             1656
<210> 20
   <211> 551
   <212> PRT
   <213> Artificial
<220>
   <223> Synthetic
<400> 20
   Met Pro Gly Ala Val Gly Lys Val Val Pro Phe Phe Glu Ala Lys Val
   1               5                   10                  15
   Val Asp Leu Asp Thr Gly Lys Thr Leu Gly Val Asn Gln Arg Gly Glu
               20                  25                  30
   Leu Cys Val Arg Gly Pro Met Ile Met Ser Gly Tyr Val Asn Asn Pro
           35                  40                  45
   Glu Ala Thr Asn Ala Leu Ile Asp Lys Asp Gly Trp Leu His Ser Gly
       50                  55                  60
   Asp Ile Ala Tyr Trp Asp Glu Asp Glu His Phe Phe Ile Val Asp Arg
   65                  70                  75                  80
   Leu Lys Ser Leu Ile Lys Tyr Lys Gly Tyr Gln Val Ala Pro Ala Glu
                   85                  90                  95
   Leu Glu Ser Ile Leu Leu Gln His Pro Asn Ile Phe Asp Ala Gly Val
               100                 105                 110
   Ala Gly Leu Pro Asp Asp Asp Ala Gly Glu Leu Pro Ala Ala Val Val
           115                 120                 125
   Val Leu Glu His Gly Lys Thr Met Thr Glu Lys Glu Ile Val Asp Tyr
       130                 135                 140
   Val Ala Ser Gln Val Thr Thr Ala Lys Lys Leu Arg Gly Gly Val Val
   145                 150                 155                 160
   Phe Val Asp Glu Val Pro Lys Gly Leu Thr Gly Lys Leu Asp Ala Arg
                   165                 170                 175
   Lys Ile Arg Glu Ile Leu Ile Lys Ala Lys Lys Gly Ser Ser Leu Glu
               180                 185                 190
   Thr Asp Gly Ser Ser Gly Ala Lys Asn Ile Lys Lys Gly Pro Ala Pro
           195                 200                 205
   Phe Tyr Pro Leu Glu Asp Gly Thr Ala Gly Glu Gln Leu His Lys Ala
       210                 215                 220
   Met Lys Arg Tyr Ala Leu Val Pro Gly Thr Ile Ala Phe Thr Asp Ala
   225                 230                 235                 240
   His Ile Glu Val Asp Ile Thr Tyr Ala Glu Tyr Phe Glu Met Ser Val
                   245                 250                 255
   Arg Leu Ala Glu Ala Met Lys Arg Tyr Gly Leu Asn Thr Asn His Arg
               260                 265                 270
   Ile Val Val Cys Ser Glu Asn Ser Leu Gln Phe Phe Met Pro Val Leu
           275                 280                 285
   Gly Ala Leu Phe Ile Gly Val Ala Val Ala Pro Ala Asn Asp Ile Tyr
       290                 295                 300
   Asn Glu Arg Glu Leu Leu Asn Ser Met Gly Ile Ser Gln Pro Thr Val
   305                 310                 315                 320
   Val Phe Val Ser Lys Lys Gly Leu Gln Lys Ile Leu Asn Val Gln Lys
                   325                 330                 335
   Lys Leu Pro Ile Ile Gln Lys Ile Ile Ile Met Asp Ser Lys Thr Asp
               340                 345                 350
   Tyr Gln Gly Phe Gln Ser Met Tyr Thr Phe Val Thr Ser His Leu Pro
           355                 360                 365
   Pro Gly Phe Asn Glu Tyr Asp Phe Val Pro Glu Ser Phe Asp Arg Asp
       370                 375                 380
   Lys Thr Ile Ala Leu Ile Met Asn Ser Ser Gly Ser Thr Gly Leu Pro
   385                 390                 395                 400
   Lys Gly Val Ala Leu Pro His Arg Thr Ala Cys Val Arg Phe Ser His
                   405                 410                 415
   Ala Arg Asp Pro Ile Phe Gly Asn Gln Ile Ile Pro Asp Thr Ala Ile
               420                 425                 430
   Leu Ser Val Val Pro Phe His His Gly Phe Gly Met Phe Thr Thr Leu
           435                 440                 445
   Gly Tyr Leu Ile Cys Gly Phe Arg Val Val Leu Met Tyr Arg Phe Glu
       450                 455                 460
   Glu Glu Leu Phe Leu Arg Ser Leu Gln Asp Tyr Lys Ile Gln Ser Ala
   465                 470                 475                 480
   Leu Leu Val Pro Thr Leu Phe Ser Phe Phe Ala Lys Ser Thr Leu Ile
                   485                 490                 495
   Asp Lys Tyr Asp Leu Ser Asn Leu His Glu Ile Ala Ser Gly Gly Ala
               500                 505                 510
   Pro Leu Ser Lys Glu Val Gly Glu Ala Val Ala Lys Arg Phe His Leu
           515                 520                 525
   Pro Gly Ile Arg Gln Gly Tyr Gly Leu Thr Glu Thr Thr Ser Ala Ile
       530                 535                 540
   Leu Ile Thr Pro Glu Gly Val
   545                 550
<210> 21
   <211> 1665
   <212> DNA
   <213> Artificial
<220>
   <223> Synthetic
<400> 21
   atggacaccg ctatcctcag cgtggtgcca tttcaccacg gcttcggcat gttcaccacg      60
   ctgggctact tgatctgcgg ctttcgggtc gtgctcatgt accgcttcga ggaggagcta     120
   ttcttgcgca gcttgcaaga ctataagatt caatctgccc tgctggtgcc cacactattt     180
   agcttcttcg ctaagagcac tctcatcgac aagtacgacc taagcaactt gcacgagatc     240
   gccagcggcg gggcgccgct cagcaaggag gtaggtgagg ccgtggccaa acgcttccac     300
   ctaccaggca tccgccaggg ctacggcctg acagaaacaa ccagcgccat tctgatcacc     360
   cccgaagggg acgacaagcc tggcgcagta ggcaaggtgg tgcccttctt cgaggctaag     420
   gtggtggact tggacactgg taagacactg ggtgtgaacc agcgcggcga gctgtgcgtc     480
   cgtggcccca tgatcatgag cggctacgtt aacaaccccg aggctacaaa cgctctcatc     540
   gacaaggacg gctggctgca cagcggcgac atcgcctact gggacgagga cgagcacttc     600
   ttcatcgtgg accggctgaa gagcctgatc aaatacaagg gctaccaggt agccccagcc     660
   gaactggaga gcatcctgct gcaacacccc aacatcttcg acgccggggt cgccggcctg     720
   cccgacgacg atgccggcga gctgcccgcc gcagtcgtcg tgctggaaca cggtaaaacc     780
   atgaccgaga aggagatcgt ggactatgtg gccagccagg ttacaaccgc caagaagctg     840
   cgcggtggtg ttgtgttcgt ggacgaggtg cctaaaggac tgaccggcaa gttggacgcc     900
   cgcaagatcc gcgagattct cattaaggcc aagaagggct cgagcctgga gaccgacggc     960
   agctccggtg ccaaaaacat taagaagggc ccagcgccat tctacccact cgaagacggg    1020
   accgccggcg agcagctgca caaagccatg aagcgctacg ccctggtgcc cggcaccatc    1080
   gcctttaccg acgcacatat cgaggtggac attacctacg ccgagtactt cgagatgagc    1140
   gttcggctgg cagaagctat gaagcgctat gggctgaata caaaccatcg gatcgtggtg    1200
   tgcagcgaga atagcttgca gttcttcatg cccgtgttgg gtgccctgtt catcggtgtg    1260
   gctgtggccc cagctaacga catctacaac gagcgcgagc tgctgaacag catgggcatc    1320
   agccagccca ccgtcgtatt cgtgagcaag aaagggctgc aaaagatcct caacgtgcaa    1380
   aagaagctac cgatcataca aaagatcatc atcatggata gcaagaccga ctaccagggc    1440
   ttccaaagca tgtacacctt cgtgacttcc catttgccac ccggcttcaa cgagtacgac    1500
   ttcgtgcccg agagcttcga ccgggacaaa accatcgccc tgatcatgaa cagtagtggc    1560
   agtaccggat tgcccaaggg cgtagcccta ccgcaccgca ccgcttgtgt ccgattcagt    1620
   catgcccgcg accccatctt cggcaaccag atcatccccg tttaa                    1665
<210> 22
   <211> 554
   <212> PRT
   <213> Artificial
<220>
   <223> Synthetic
<400> 22
   Met Asp Thr Ala Ile Leu Ser Val Val Pro Phe His His Gly Phe Gly
   1               5                   10                  15
   Met Phe Thr Thr Leu Gly Tyr Leu Ile Cys Gly Phe Arg Val Val Leu
               20                  25                  30
   Met Tyr Arg Phe Glu Glu Glu Leu Phe Leu Arg Ser Leu Gln Asp Tyr
           35                  40                  45
   Lys Ile Gln Ser Ala Leu Leu Val Pro Thr Leu Phe Ser Phe Phe Ala
       50                  55                  60
   Lys Ser Thr Leu Ile Asp Lys Tyr Asp Leu Ser Asn Leu His Glu Ile
   65 70 75 80
   Ala Ser Gly Gly Ala Pro Leu Ser Lys Glu Val Gly Glu Ala Val Ala
   85 90 95
   Lys Arg Phe His Leu Pro Gly Ile Arg Gln Gly Tyr Gly Leu Thr Glu
   100 105 110
   Thr Thr Ser Ala Ile Leu Ile Thr Pro Glu Gly Asp Asp Lys Pro Gly
   115 120 125
   Ala Val Gly Lys Val Val Pro Phe Phe Glu Ala Lys Val Val Asp Leu
   130 135 140
   Asp Thr Gly Lys Thr Leu Gly Val Asn Gln Arg Gly Glu Leu Cys Val
   145 150 155 160
   Arg Gly Pro Met Ile Met Ser Gly Tyr Val Asn Asn Pro Glu Ala Thr
   165 170 175
   Asn Ala Leu Ile Asp Lys Asp Gly Trp Leu His Ser Gly Asp Ile Ala
   180 185 190
   Tyr Trp Asp Glu Asp Glu His Phe Phe Ile Val Asp Arg Leu Lys Ser
   195 200 205
   Leu Ile Lys Tyr Lys Gly Tyr Gln Val Ala Pro Ala Glu Leu Glu Ser
   210 215 220
   Ile Leu Leu Gln His Pro Asn Ile Phe Asp Ala Gly Val Ala Gly Leu
   225 230 235 240
   Pro Asp Asp Asp Ala Gly Glu Leu Pro Ala Ala Val Val Val Leu Glu
   245 250 255
   His Gly Lys Thr Met Thr Glu Lys Glu Ile Val Asp Tyr Val Ala Ser
   260 265 270
   Gln Val Thr Thr Ala Lys Lys Leu Arg Gly Gly Val Val Phe Val Asp
   275 280 285
   Glu Val Pro Lys Gly Leu Thr Gly Lys Leu Asp Ala Arg Lys Ile Arg
   290 295 300
   Glu Ile Leu Ile Lys Ala Lys Lys Gly Ser Ser Leu Glu Thr Asp Gly
   305 310 315 320
   Ser Ser Gly Ala Lys Asn Ile Lys Lys Gly Pro Ala Pro Phe Tyr Pro
   325 330 335
   Leu Glu Asp Gly Thr Ala Gly Glu Gln Leu His Lys Ala Met Lys Arg
   340 345 350
   Tyr Ala Leu Val Pro Gly Thr Ile Ala Phe Thr Asp Ala His Ile Glu
   355 360 365
   Val Asp Ile Thr Tyr Ala Glu Tyr Phe Glu Met Ser Val Arg Leu Ala
   370 375 380
   Glu Ala Met Lys Arg Tyr Gly Leu Asn Thr Asn His Arg Ile Val Val
   385 390 395 400
   Cys Ser Glu Asn Ser Leu Gln Phe Phe Met Pro Val Leu Gly Ala Leu
   405 410 415
   Phe Ile Gly Val Ala Val Ala Pro Ala Asn Asp Ile Tyr Asn Glu Arg
   420 425 430
   Glu Leu Leu Asn Ser Met Gly Ile Ser Gln Pro Thr Val Val Phe Val
   435 440 445
   Ser Lys Lys Gly Leu Gln Lys Ile Leu Asn Val Gln Lys Lys Leu Pro
   450 455 460
   Ile Ile Gln Lys Ile Ile Ile Met Asp Ser Lys Thr Asp Tyr Gln Gly
   465 470 475 480
   Phe Gln Ser Met Tyr Thr Phe Val Thr Ser His Leu Pro Pro Gly Phe
   485 490 495
   Asn Glu Tyr Asp Phe Val Pro Glu Ser Phe Asp Arg Asp Lys Thr Ile
   500 505 510
   Ala Leu Ile Met Asn Ser Ser Gly Ser Thr Gly Leu Pro Lys Gly Val
   515 520 525
   Ala Leu Pro His Arg Thr Ala Cys Val Arg Phe Ser His Ala Arg Asp
   530 535 540
   Pro Ile Phe Gly Asn Gln Ile Ile Pro Val
   545 550
<210> 23
   <211> 12
   <212> PRT
   <213> Artificial
<220>
   <223> Synthetic
<400> 23
   Gly Ser Ser Gly Gly Ser Gly Gly Ser Gly Gly Gly
   1               5                   10
<210> 24
   <211> 12
   <212> PRT
   <213> Artificial
<220>
   <223> Synthetic
<400> 24
   Gly Ser Ser Ser Asp Ser Asp Ser Ser Ala Gly Ser
   1               5                   10
<210> 25
   <211> 12
   <212> PRT
   <213> Artificial
<220>
   <223> Synthetic
<400> 25
   Gly Ser Asn Asp Ser Ser Gly Gly Ser Glu Gly Gly
   1               5                   10
<210> 26
   <211> 12
   <212> PRT
   <213> Artificial
<220>
   <223> Synthetic
<400> 26
   Gly Ser Asn Gly Gly Phe Asp Ser Ser Glu Gly Gly
   1               5                   10
<210> 27
   <211> 12
   <212> PRT
   <213> Artificial
<220>
   <223> Synthetic
<400> 27
   Gly Ser Ile Arg Trp Ser Gly Leu Ser Gly Gly Asp
   1 5 10
<210> 28
   <211> 12
   <212> PRT
   <213> Artificial
<220>
   <223> Synthetic
<400> 28
   Gly Ser Arg Gly Gly Ser Val Tyr Ser Glu Gly Gly
   1 5 10
<210> 29
   <211> 12
   <212> PRT
   <213> Artificial
<220>
   <223> Synthetic
<400> 29
   Gly Ser Ser Glu Gly Ser Ser Asp Phe Gly Gly Asp
   1 5 10
<210> 30
   <211> 12
   <212> PRT
   <213> Artificial
<220>
   <223> Synthetic
<400> 30
   Gly Ser Ile Val Val Ser Cys Ser Ser Glu Gly Gly
   1 5 10
<210> 31
   <211> 12
   <212> PRT
   <213> Artificial
<220>
   <223> Synthetic
<400> 31
   Gly Ser Asn Trp Asp Ser Gly Cys Ser Arg Glu Gly
   1 5 10
<210> 32
   <211> 12
   <212> PRT
   <213> Artificial
<220>
   <223> Synthetic
<400> 32
   Gly Ser Asn Trp Asp Ser Gly Cys Ser Arg Glu Cys
   1               5                   10
<210> 33
   <211> 12
   <212> PRT
   <213> Artificial
<220>
   <223> Synthetic
<400> 33
   Gly Ser Ser Gly Cys Thr Gly Asp Ala Gly Gly Ser
   1               5                   10
<210> 34
   <211> 12
   <212> PRT
   <213> Artificial
<220>
   <223> Synthetic
<400> 34
   Gly Ser Asn Trp Asp Ser Gly Cys Ser Arg Gln Cys
   1               5                   10
<210> 35
   <211> 22
   <212> PRT
   <213> Artificial
<220>
   <223> Synthetic
<400> 35
   Gly Ser Ser Asn Ser Asp Gly Asp Ser Gly Ser Phe Asp Gly Ser Gly
   1               5                   10                  15
   Ser Ala Glu Gly Ser Gly
               20
<210> 36
   <211> 24
   <212> PRT
   <213> Artificial
<220>
   <223> Synthetic
<400> 36
   Gly Ser Ile Arg Ser Arg Gly Glu Trp Gly Ser Gly Val Ser Leu Tyr
   1               5                   10                  15
   Asp Ser Phe Gly Glu Gly Asp Gly
               20
<210> 37
   <211> 27
   <212> PRT
   <213> Artificial
<220>
   <223> Synthetic
<400> 37
   Gly Ser Ile Asn Ser Val Trp Gly Val Asp Cys Ser Thr Cys Gly Ser
   1               5                   10                  15
   Cys Asp Ser Ala Glu Arg Gly Gly Glu Gly Ser
               20                  25
<210> 38
   <211> 24
   <212> PRT
   <213> Artificial
<220>
   <223> Synthetic
<400> 38
   Gly Ser Ile Ser Val Gly Ala Val Gly Ser Cys Gly Asp Gly Asp Ser
   1               5                   10                  15
   Ser Ala Gly Glu Gly Glu Gly Asn
               20
<210> 39
   <211> 30
   <212> PRT
   <213> Artificial
<220>
   <223> Synthetic
<400> 39
   Gly Ser Ile Asn Ser Val Trp Gly Ala Val Asp Cys Gly Ser Thr Cys
   1 5 10 15
   Cys Gly Ser Cys Asp Ser Ala Glu Arg Gly Gly Glu Gly Ser
   20 25 30
<210> 40
   <211> 12
   <212> PRT
   <213> Artificial
<220>
   <223> Synthetic
<400> 40
   Gly Ser Ile Ala Gly Cys Gly Asp Ala Gly Glu Gly
   1 5 10
<210> 41
   <211> 11
   <212> PRT
   <213> Artificial
<220>
   <223> Synthetic
<400> 41
   Gly Ser Asn Trp Asp Ser Gly Cys Ser Arg Glu
   1 5 10
<210> 42
   <211> 12
   <212> PRT
   <213> Artificial
<220>
   <223> Synthetic
<400> 42
   Gly Ser Ile Ala Gly Cys Gly Asp Ala Gly Glu Gly
   1 5 10
<210> 43
   <211> 12
   <212> PRT
   <213> Artificial
<220>
   <223> Synthetic
<400> 43
   Gly Ser Asn Trp Asp Ser Gly Cys Ser Arg Glu Gly
   1 5 10
<210> 44
   <211> 10
   <212> PRT
   <213> Artificial
<220>
   <223> Synthetic
<400> 44
   Asn Trp Asp Ser Gly Cys Ser Arg Glu Gly
   1 5 10
<210> 45
   <211> 10
   <212> PRT
   <213> Artificial
<220>
   <223> Synthetic
<400> 45
   Ile Ala Gly Cys Gly Asp Ala Gly Glu Gly
   1 5 10
<210> 46
   <211> 11
   <212> PRT
   <213> Artificial
<220>
   <223> Synthetic
<400> 46
   Ser Thr Leu Gln Ser Gly Leu Arg Lys Met Ala
   1 5 10
<210> 47
   <211> 8
   <212> PRT
   <213> Artificial
<220>
   <223> Synthetic
<400> 47
   Ser Gln Asn Tyr Pro Ile Val Gln
   1 5
<210> 48
   <211> 10
   <212> PRT
   <213> Artificial
<220>
   <223> Synthetic
<400> 48
   Lys Ala Val Arg Leu Ala Glu Ala Met Ser
   1               5                   10
<210> 49
   <211> 12
   <212> PRT
   <213> Artificial
<220>
   <223> Synthetic
<400> 49
   Ala Glu Asp Val Val Cys Cys Ser Met Ser Tyr Ser
   1               5                   10
<210> 50
   <211> 20
   <212> PRT
   <213> Artificial
<220>
   <223> Synthetic
<400> 50
   Gln Thr Ser Ile Thr Ser Ala Val Leu Gln Ser Gly Phe Arg Lys Met
   1               5                   10                  15
   Ala Phe Pro Ser
               20
<210> 51
   <211> 20
   <212> PRT
   <213> Artificial
<220>
   <223> Synthetic
<400> 51
   Val Arg Gln Cys Ser Gly Val Thr Phe Gln Gly Lys Phe Lys Lys Ile
   1               5                   10                  15
   Val Lys Gly Thr
               20
<210> 52
   <211> 10
   <212> PRT
   <213> Artificial
<220>
   <223> Synthetic
<400> 52
   Ser Ser Asp Glu Val Asp Gly Ser Ser Gly
   1               5                   10
<210> 53
   <211> 14
   <212> PRT
   <213> Artificial
<220>
   <223> Synthetic
<400> 53
   Ser Ser Gly Ser Asp Glu Val Asp Gly Ser Leu Ser Ser Gly
   1               5                   10
<210> 54
   <211> 8
   <212> PRT
   <213> Artificial
<220>
   <223> Synthetic
<400> 54
   Ser Asp Glu Val Asp Gly Ser Leu
   1               5
<210> 55
   <211> 5
   <212> PRT
   <213> Artificial
<220>
   <223> Synthetic
<400> 55
   Asp Glu Val Asp Gly
   1               5
<210> 56
   <211> 7
   <212> PRT
   <213> Artificial
<220>
   <223> Synthetic
<400> 56
   Val Lys Met Asp Ala Glu Phe
   1               5
<210> 57
   <211> 1665
   <212> DNA
   <213> Artificial
<220>
   <223> Synthetic
<400> 57
   atgacgtcag caattttaac ggtaatacct ttccaccatg gttttggtat gatgaccaca      60
   ttaggatact ttacttgtgg attccgagtt gttctaatgc acacgtttga agaaaaacta     120
   tttctacaat cattacaaga ttataaagtg gaaagtactt tacttgtacc aacattaatg     180
   gcatttcttg caaaaagtgc attagttgaa aagtacgatt tatcgcactt aaaagaaatt     240
   gcatctggtg gcgcaccttt atcaaaagaa attggggaga tggtgaaaaa acggtttaaa     300
   ttaaactttg tcaggcaagg gtatggatta acagaaacca cttcggctgt tttaattaca     360
   ccgaaaggtg acgccaaacc gggatcaact ggtaaaatag taccattaca cgctgttaaa     420
   gttgtcgatc ctacaacagg aaaaattttg gggccaaatg aacctggaga attgtatttt     480
   aaaggcccga tgataatgaa gggttattat aataatgaag aagctactaa agcaattatt     540
   gataatgacg gatggttgcg ctctggtgat attgcttatt atgacaatga tggccatttt     600
   tatattgtgg acaggctgaa gtcactgatt aaatataaag gttatcaggt tgcacctgct     660
   gaaattgagg gaatactctt acaacatccg tatattgttg atgccggcgt tactggtata     720
   ccggatgaag ccgcgggcga gcttccagct gcaggtgttg tagtacagac tggaaaatat     780
   ctaaacgaac aaatcgtaca agattatgtt gccagtcaag tttcaacagc caaatggcta     840
   cgtggtgggg tgaaattttt ggatgaaatt cccaaaggat caactggaaa aattgacaga     900
   aaagtgttaa gacaaatgtt agaaaaacac accaatggct cgagcctgga gaccgacggc     960
   agctccggtg ataagaatat tttatatggg cccgaaccat tttatccctt ggaagatggg    1020
   acggctggag aacagatgtt tgacgcatta tctcgttatg cagctattcc gggctgcata    1080
   gcattgacaa atgctcatac aaaagaaaat gttttatatg aagagtttct gaaactgtcg    1140
   tgtcgtttag cggaaagttt taaaaagtat ggattaaaac aaaacgacac aatagcggtg    1200
   tgtagcgaaa atagtctgca atttttcctt cctgtaattg catcattgta tcttggaata    1260
   attgtggcac ctgttaacga taaatacatt gaacgtgaat taatacacag tcttggtatt    1320
   gtaaaaccac gcatagtttt ttgctccaag aatacttttc aaaaagtact gaatgtaaaa    1380
   tctaaattaa aatctattga aactattatt atattagact taaatgaaga cttaggaggt    1440
   tatcaatgcc tcaacaactt tatttctcaa aattccgata gtaatctgga cgtaaaaaaa    1500
   tttaaaccct attcttttaa tcgagacgat caggttgcgt cgattatgtt ttcttctggt    1560
   acaactggtc tgccgaaggg agtcatgcta actcacaaga atattgttgc acgattttct    1620
   attgcaaaag atcctacttt tggtaacgca attaatcccg tttaa                    1665
<210> 58
   <211> 554
   <212> PRT
   <213> Artificial
<220>
   <223> Synthetic
<400> 58
   Met Thr Ser Ala Ile Leu Thr Val Ile Pro Phe His His Gly Phe Gly
   1               5                   10                  15
   Met Met Thr Thr Leu Gly Tyr Phe Thr Cys Gly Phe Arg Val Val Leu
               20                  25                  30
   Met His Thr Phe Glu Glu Lys Leu Phe Leu Gln Ser Leu Gln Asp Tyr
           35                  40                  45
   Lys Val Glu Ser Thr Leu Leu Val Pro Thr Leu Met Ala Phe Leu Ala
       50                  55                  60
   Lys Ser Ala Leu Val Glu Lys Tyr Asp Leu Ser His Leu Lys Glu Ile
   65                  70                  75                  80
   Ala Ser Gly Gly Ala Pro Leu Ser Lys Glu Ile Gly Glu Met Val Lys
                   85                  90                  95
   Lys Arg Phe Lys Leu Asn Phe Val Arg Gln Gly Tyr Gly Leu Thr Glu
               100                 105                 110
   Thr Thr Ser Ala Val Leu Ile Thr Pro Lys Gly Asp Ala Lys Pro Gly
           115                 120                 125
   Ser Thr Gly Lys Ile Val Pro Leu His Ala Val Lys Val Val Asp Pro
       130                 135                 140
   Thr Thr Gly Lys Ile Leu Gly Pro Asn Glu Pro Gly Glu Leu Tyr Phe
   145                 150                 155                 160
   Lys Gly Pro Met Ile Met Lys Gly Tyr Tyr Asn Asn Glu Glu Ala Thr
                   165                 170                 175
   Lys Ala Ile Ile Asp Asn Asp Gly Trp Leu Arg Ser Gly Asp Ile Ala
               180                 185                 190
   Tyr Tyr Asp Asn Asp Gly His Phe Tyr Ile Val Asp Arg Leu Lys Ser
           195                 200                 205
   Leu Ile Lys Tyr Lys Gly Tyr Gln Val Ala Pro Ala Glu Ile Glu Gly
   210 215 220
   Ile Leu Leu Gln His Pro Tyr Ile Val Asp Ala Gly Val Thr Gly Ile
   225 230 235 240
   Pro Asp Glu Ala Ala Gly Glu Leu Pro Ala Ala Gly Val Val Val Gln
   245 250 255
   Thr Gly Lys Tyr Leu Asn Glu Gln Ile Val Gln Asp Tyr Val Ala Ser
   260 265 270
   Gln Val Ser Thr Ala Lys Trp Leu Arg Gly Gly Val Lys Phe Leu Asp
   275 280 285
   Glu Ile Pro Lys Gly Ser Thr Gly Lys Ile Asp Arg Lys Val Leu Arg
   290 295 300
   Gln Met Leu Glu Lys His Thr Asn Gly Ser Ser Leu Glu Thr Asp Gly
   305 310 315 320
   Ser Ser Gly Asp Lys Asn Ile Leu Tyr Gly Pro Glu Pro Phe Tyr Pro
   325 330 335
   Leu Glu Asp Gly Thr Ala Gly Glu Gln Met Phe Asp Ala Leu Ser Arg
   340 345 350
   Tyr Ala Ala Ile Pro Gly Cys Ile Ala Leu Thr Asn Ala His Thr Lys
   355 360 365
   Glu Asn Val Leu Tyr Glu Glu Phe Leu Lys Leu Ser Cys Arg Leu Ala
   370 375 380
   Glu Ser Phe Lys Lys Tyr Gly Leu Lys Gln Asn Asp Thr Ile Ala Val
   385 390 395 400
   Cys Ser Glu Asn Ser Leu Gln Phe Phe Leu Pro Val Ile Ala Ser Leu
   405 410 415
   Tyr Leu Gly Ile Ile Val Ala Pro Val Asn Asp Lys Tyr Ile Glu Arg
   420 425 430
   Glu Leu Ile His Ser Leu Gly Ile Val Lys Pro Arg Ile Val Phe Cys
   435 440 445
   Ser Lys Asn Thr Phe Gln Lys Val Leu Asn Val Lys Ser Lys Leu Lys
   450 455 460
   Ser Ile Glu Thr Ile Ile Ile Leu Asp Leu Asn Glu Asp Leu Gly Gly
   465                 470                 475                 480
   Tyr Gln Cys Leu Asn Asn Phe Ile Ser Gln Asn Ser Asp Ser Asn Leu
                   485                 490                 495
   Asp Val Lys Lys Phe Lys Pro Tyr Ser Phe Asn Arg Asp Asp Gln Val
               500                 505                 510
   Ala Ser Ile Met Phe Ser Ser Gly Thr Thr Gly Leu Pro Lys Gly Val
           515                 520                 525
   Met Leu Thr His Lys Asn Ile Val Ala Arg Phe Ser Ile Ala Lys Asp
       530                 535                 540
   Pro Thr Phe Gly Asn Ala Ile Asn Pro Val
   545                 550
<210> 59
   <211> 1653
   <212> DNA
   <213> Artificial
<220>
   <223> Synthetic
<400> 59
   atgccgggat caactggtaa aatagtacca ttacacgctg ttaaagttgt cgatcctaca      60
   acaggaaaaa ttttggggcc aaatgaacct ggagaattgt attttaaagg cccgatgata     120
   atgaagggtt attataataa tgaagaagct actaaagcaa ttattgataa tgacggatgg     180
   ttgcgctctg gtgatattgc ttattatgac aatgatggcc atttttatat tgtggacagg     240
   ctgaagtcac tgattaaata taaaggttat caggttgcac ctgctgaaat tgagggaata     300
   ctcttacaac atccgtatat tgttgatgcc ggcgttactg gtataccgga tgaagccgcg     360
   ggcgagcttc cagctgcagg tgttgtagta cagactggaa aatatctaaa cgaacaaatc     420
   gtacaagatt atgttgccag tcaagtttca acagccaaat ggctacgtgg tggggtgaaa     480
   tttttggatg aaattcccaa aggatcaact ggaaaaattg acagaaaagt gttaagacaa     540
   atgttagaaa aacacaccaa tggctcgagc ctggagaccg acggcagctc cgggaaaaac     600
   atcctgtatg gtccggaacc gttctaccca ctggaagatg gtaccgctgg tgaacagatg     660
   tttgacgcat tatctcgtta tgcagctatt ccgggctgca tagcattgac aaatgctcat     720
   acaaaagaaa atgttttata tgaagagttt ctgaaactgt cgtgtcgttt agcggaaagt     780
   tttaaaaagt atggattaaa acaaaacgac acaatagcgg tgtgtagcga aaatagtctg     840
   caatttttcc ttcctgtaat tgcatcattg tatcttggaa taattgtggc acctgttaac     900
   gataaataca ttgaacgtga attaatacac agtcttggta ttgtaaaacc acgcatagtt     960
   ttttgctcca agaatacttt tcaaaaagta ctgaatgtaa aatctaaatt aaaatctatt    1020
   gaaactatta ttatattaga cttaaatgaa gacttaggag gttatcaatg cctcaacaac    1080
   tttatttctc aaaattccga tagtaatctg gacgtaaaaa aatttaaacc ctattctttt    1140
   aatcgagacg atcaggttgc gtcgattatg ttttcttctg gtacaactgg tctgccgaag    1200
   ggagtcatgc taactcacaa gaatattgtt gcacgatttt ctattgcaaa agatcctact    1260
   tttggtaacg caattaatcc cacgtcagca attttaacgg taataccttt ccaccatggt   1320
   tttggtatga tgaccacatt aggatacttt acttgtggat tccgagttgt tctaatgcac    1380
   acgtttgaag aaaaactatt tctacaatca ttacaagatt ataaagtgga aagtacttta    1440
   cttgtaccaa cattaatggc atttcttgca aaaagtgcat tagttgaaaa gtacgattta    1500
   tcgcacttaa aagaaattgc atctggtggc gcacctttat caaaagaaat tggggagatg    1560
   gtgaaaaaac ggtttaaatt aaactttgtc aggcaagggt atggattaac agaaaccact    1620
   tcggctgttt taattacacc gaaaggtgtt taa                                1653
<210> 60
   <211> 550
   <212> PRT
   <213> Artificial
<220>
   <223> Synthetic
<400> 60
   Met Pro Gly Ser Thr Gly Lys Ile Val Pro Leu His Ala Val Lys Val
   1               5                   10                  15
   Val Asp Pro Thr Thr Gly Lys Ile Leu Gly Pro Asn Glu Pro Gly Glu
               20                  25                  30
   Leu Tyr Phe Lys Gly Pro Met Ile Met Lys Gly Tyr Tyr Asn Asn Glu
           35                  40                  45
   Glu Ala Thr Lys Ala Ile Ile Asp Asn Asp Gly Trp Leu Arg Ser Gly
       50                  55                  60
   Asp Ile Ala Tyr Tyr Asp Asn Asp Gly His Phe Tyr Ile Val Asp Arg
   65                  70                  75                  80
   Leu Lys Ser Leu Ile Lys Tyr Lys Gly Tyr Gln Val Ala Pro Ala Glu
                   85                  90                  95
   Ile Glu Gly Ile Leu Leu Gln His Pro Tyr Ile Val Asp Ala Gly Val
               100                 105                 110
   Thr Gly Ile Pro Asp Glu Ala Ala Gly Glu Leu Pro Ala Ala Gly Val
           115                 120                 125
   Val Val Gln Thr Gly Lys Tyr Leu Asn Glu Gln Ile Val Gln Asp Tyr
       130                 135                 140
   Val Ala Ser Gln Val Ser Thr Ala Lys Trp Leu Arg Gly Gly Val Lys
   145                 150                 155                 160
   Phe Leu Asp Glu Ile Pro Lys Gly Ser Thr Gly Lys Ile Asp Arg Lys
                   165                 170                 175
   Val Leu Arg Gln Met Leu Glu Lys His Thr Asn Gly Ser Ser Leu Glu
               180                 185                 190
   Thr Asp Gly Ser Ser Gly Lys Asn Ile Leu Tyr Gly Pro Glu Pro Phe
           195                 200                 205
   Tyr Pro Leu Glu Asp Gly Thr Ala Gly Glu Gln Met Phe Asp Ala Leu
       210                 215                 220
   Ser Arg Tyr Ala Ala Ile Pro Gly Cys Ile Ala Leu Thr Asn Ala His
   225                 230                 235                 240
   Thr Lys Glu Asn Val Leu Tyr Glu Glu Phe Leu Lys Leu Ser Cys Arg
                   245                 250                 255
   Leu Ala Glu Ser Phe Lys Lys Tyr Gly Leu Lys Gln Asn Asp Thr Ile
               260                 265                 270
   Ala Val Cys Ser Glu Asn Ser Leu Gln Phe Phe Leu Pro Val Ile Ala
           275                 280                 285
   Ser Leu Tyr Leu Gly Ile Ile Val Ala Pro Val Asn Asp Lys Tyr Ile
       290                 295                 300
   Glu Arg Glu Leu Ile His Ser Leu Gly Ile Val Lys Pro Arg Ile Val
   305                 310                 315                 320
   Phe Cys Ser Lys Asn Thr Phe Gln Lys Val Leu Asn Val Lys Ser Lys
                   325                 330                 335
   Leu Lys Ser Ile Glu Thr Ile Ile Ile Leu Asp Leu Asn Glu Asp Leu
               340                 345                 350
   Gly Gly Tyr Gln Cys Leu Asn Asn Phe Ile Ser Gln Asn Ser Asp Ser
   355 360 365
   Asn Leu Asp Val Lys Lys Phe Lys Pro Tyr Ser Phe Asn Arg Asp Asp
   370 375 380
   Gln Val Ala Ser Ile Met Phe Ser Ser Gly Thr Thr Gly Leu Pro Lys
   385 390 395 400
   Gly Val Met Leu Thr His Lys Asn Ile Val Ala Arg Phe Ser Ile Ala
   405 410 415
   Lys Asp Pro Thr Phe Gly Asn Ala Ile Asn Pro Thr Ser Ala Ile Leu
   420 425 430
   Thr Val Ile Pro Phe His His Gly Phe Gly Met Met Thr Thr Leu Gly
   435 440 445
   Tyr Phe Thr Cys Gly Phe Arg Val Val Leu Met His Thr Phe Glu Glu
   450 455 460
   Lys Leu Phe Leu Gln Ser Leu Gln Asp Tyr Lys Val Glu Ser Thr Leu
   465 470 475 480
   Leu Val Pro Thr Leu Met Ala Phe Leu Ala Lys Ser Ala Leu Val Glu
   485 490 495
   Lys Tyr Asp Leu Ser His Leu Lys Glu Ile Ala Ser Gly Gly Ala Pro
   500 505 510
   Leu Ser Lys Glu Ile Gly Glu Met Val Lys Lys Arg Phe Lys Leu Asn
   515 520 525
   Phe Val Arg Gln Gly Tyr Gly Leu Thr Glu Thr Thr Ser Ala Val Leu
   530 535 540
   Ile Thr Pro Lys Gly Val
   545 550
<210> 61
   <211> 1671
   <212> DNA
   <213> Artificial
<220>
   <223> Synthetic
<400> 61
   atgacgtcag caattttaac ggtaatacct ttccaccatg gttttggtat gatgaccaca 60
   ttaggatact ttacttgtgg attccgagtt gttctaatgc acacgtttga agaaaaacta 120
   tttctacaat cattacaaga ttataaagtg gaaagtactt tacttgtacc aacattaatg 180
   gcatttcttg caaaaagtgc attagttgaa aagtacgatt tatcgcactt aaaagaaatt 240
   gcatctggtg gcgcaccttt atcaaaagaa attggggaga tggtgaaaaa acggtttaaa 300
   ttaaactttg tcaggcaagg gtatggatta acagaaacca cttcggctgt tttaattaca 360
   ccgaaaggtg acgccaaacc gggatcaact ggtaaaatag taccattaca cgctgttaaa 420
   gttgtcgatc ctacaacagg aaaaattttg gggccaaatg aacctggaga attgtatttt 480
   aaaggcccga tgataatgaa gggttattat aataatgaag aagctactaa agcaattatt 540
   gataatgacg gatggttgcg ctctggtgat attgcttatt atgacaatga tggccatttt 600
   tatattgtgg acaggctgaa gtcactgatt aaatataaag gttatcaggt tgcacctgct 660
   gaaattgagg gaatactctt acaacatccg tatattgttg atgccggcgt tactggtata 720
   ccggatgaag ccgcgggcga gcttccagct gcaggtgttg tagtacagac tggaaaatat 780
   ctaaacgaac aaatcgtaca agattatgtt gccagtcaag tttcaacagc caaatggcta 840
   cgtggtgggg tgaaattttt ggatgaaatt cccaaaggat caactggaaa aattgacaga 900
   aaagtgttaa gacaaatgtt agaaaaacac accaatggct cgagcgagaa cctgtacttc 960
   caatcgagct ccggtgataa gaatatttta tatgggcccg aaccatttta tcccttggaa 1020
   gatgggacgg ctggagaaca gatgtttgac gcattatctc gttatgcagc tattccgggc 1080
   tgcatagcat tgacaaatgc tcatacaaaa gaaaatgttt tatatgaaga gtttctgaaa 1140
   ctgtcgtgtc gtttagcgga aagttttaaa aagtatggat taaaacaaaa cgacacaata 1200
   gcggtgtgta gcgaaaatag tctgcaattt ttccttcctg taattgcatc attgtatctt 1260
   ggaataattg tggcacctgt taacgataaa tacattgaac gtgaattaat acacagtctt 1320
   ggtattgtaa aaccacgcat agttttttgc tccaagaata cttttcaaaa agtactgaat 1380
   gtaaaatcta aattaaaatc tattgaaact attattatat tagacttaaa tgaagactta 1440
   ggaggttatc aatgcctcaa caactttatt tctcaaaatt ccgatagtaa tctggacgta 1500
   aaaaaattta aaccctattc ttttaatcga gacgatcagg ttgcgtcgat tatgttttct 1560
   tctggtacaa ctggtctgcc gaagggagtc atgctaactc acaagaatat tgttgcacga 1620
   ttttctattg caaaagatcc tacttttggt aacgcaatta atcccgttta a 1671
<210> 62
   <211> 556
   <212> PRT
   <213> Artificial
<220>
   <223> Synthetic
<400> 62
   Met Thr Ser Ala Ile Leu Thr Val Ile Pro Phe His His Gly Phe Gly
   1               5                   10                  15
   Met Met Thr Thr Leu Gly Tyr Phe Thr Cys Gly Phe Arg Val Val Leu
               20                  25                  30
   Met His Thr Phe Glu Glu Lys Leu Phe Leu Gln Ser Leu Gln Asp Tyr
           35                  40                  45
   Lys Val Glu Ser Thr Leu Leu Val Pro Thr Leu Met Ala Phe Leu Ala
       50                  55                  60
   Lys Ser Ala Leu Val Glu Lys Tyr Asp Leu Ser His Leu Lys Glu Ile
   65                  70                  75                  80
   Ala Ser Gly Gly Ala Pro Leu Ser Lys Glu Ile Gly Glu Met Val Lys
                   85                  90                  95
   Lys Arg Phe Lys Leu Asn Phe Val Arg Gln Gly Tyr Gly Leu Thr Glu
               100                 105                 110
   Thr Thr Ser Ala Val Leu Ile Thr Pro Lys Gly Asp Ala Lys Pro Gly
           115                 120                 125
   Ser Thr Gly Lys Ile Val Pro Leu His Ala Val Lys Val Val Asp Pro
       130                 135                 140
   Thr Thr Gly Lys Ile Leu Gly Pro Asn Glu Pro Gly Glu Leu Tyr Phe
   145                 150                 155                 160
   Lys Gly Pro Met Ile Met Lys Gly Tyr Tyr Asn Asn Glu Glu Ala Thr
                   165                 170                 175
   Lys Ala Ile Ile Asp Asn Asp Gly Trp Leu Arg Ser Gly Asp Ile Ala
               180                 185                 190
   Tyr Tyr Asp Asn Asp Gly His Phe Tyr Ile Val Asp Arg Leu Lys Ser
           195                 200                 205
   Leu Ile Lys Tyr Lys Gly Tyr Gln Val Ala Pro Ala Glu Ile Glu Gly
       210                 215                 220
   Ile Leu Leu Gln His Pro Tyr Ile Val Asp Ala Gly Val Thr Gly Ile
   225                 230                 235                 240
   Pro Asp Glu Ala Ala Gly Glu Leu Pro Ala Ala Gly Val Val Val Gln
                   245                 250                 255
   Thr Gly Lys Tyr Leu Asn Glu Gln Ile Val Gln Asp Tyr Val Ala Ser
               260                 265                 270
   Gln Val Ser Thr Ala Lys Trp Leu Arg Gly Gly Val Lys Phe Leu Asp
           275                 280                 285
   Glu Ile Pro Lys Gly Ser Thr Gly Lys Ile Asp Arg Lys Val Leu Arg
       290                 295                 300
   Gln Met Leu Glu Lys His Thr Asn Gly Ser Ser Glu Asn Leu Tyr Phe
   305                 310                 315                 320
   Gln Ser Ser Ser Gly Asp Lys Asn Ile Leu Tyr Gly Pro Glu Pro Phe
                   325                 330                 335
   Tyr Pro Leu Glu Asp Gly Thr Ala Gly Glu Gln Met Phe Asp Ala Leu
               340                 345                 350
   Ser Arg Tyr Ala Ala Ile Pro Gly Cys Ile Ala Leu Thr Asn Ala His
           355                 360                 365
   Thr Lys Glu Asn Val Leu Tyr Glu Glu Phe Leu Lys Leu Ser Cys Arg
       370                 375                 380
   Leu Ala Glu Ser Phe Lys Lys Tyr Gly Leu Lys Gln Asn Asp Thr Ile
   385                 390                 395                 400
   Ala Val Cys Ser Glu Asn Ser Leu Gln Phe Phe Leu Pro Val Ile Ala
                   405                 410                 415
   Ser Leu Tyr Leu Gly Ile Ile Val Ala Pro Val Asn Asp Lys Tyr Ile
               420                 425                 430
   Glu Arg Glu Leu Ile His Ser Leu Gly Ile Val Lys Pro Arg Ile Val
           435                 440                 445
   Phe Cys Ser Lys Asn Thr Phe Gln Lys Val Leu Asn Val Lys Ser Lys
       450                 455                 460
   Leu Lys Ser Ile Glu Thr Ile Ile Ile Leu Asp Leu Asn Glu Asp Leu
   465                 470                 475                 480
   Gly Gly Tyr Gln Cys Leu Asn Asn Phe Ile Ser Gln Asn Ser Asp Ser
                   485                 490                 495
   Asn Leu Asp Val Lys Lys Phe Lys Pro Tyr Ser Phe Asn Arg Asp Asp
   500 505 510
   Gln Val Ala Ser Ile Met Phe Ser Ser Gly Thr Thr Gly Leu Pro Lys
   515 520 525
   Gly Val Met Leu Thr His Lys Asn Ile Val Ala Arg Phe Ser Ile Ala
   530 535 540
   Lys Asp Pro Thr Phe Gly Asn Ala Ile Asn Pro Val
   545 550 555
<210> 63
   <211> 1671
   <212> DNA
   <213> Artificial
<220>
   <223> Synthetic
<400> 63
   atgaccgcta tcctcagcgt ggtgccattt caccacggct tcggcatgtt caccacgctg      60
   ggctacttga tctgcggctt tcgggtcgtg ctcatgtacc gcttcgagga ggagctattc     120
   ttgcgcagct tgcaagacta taagattcaa tctgccctgc tggtgcccac actatttagc     180
   ttcttcgcta agagcactct catcgacaag tacgacctaa gcaacttgca cgagatcgcc     240
   agcggcgggg cgccgctcag caaggaggta ggtgaggccg tggccaaacg cttccaccta     300
   ccaggcatcc gccagggcta cggcctgaca gaaacaacca gcgccattct gatcaccccc     360
   gaaggggacg acaagcctgg cgcagtaggc aaggtggtgc ccttcttcga ggctaaggtg     420
   gtggacttgg acaccggtaa gacactgggt gtgaaccagc gcggcgagct gtgcgtccgt     480
   ggccccatga tcatgagcgg ctacgttaac aaccccgagg ctacaaacgc tctcatcgac     540
   aaggacggct ggctgcacag cggcgacatc gcctactggg acgaggacga gcacttcttc     600
   atcgtggacc ggctgaagag cctgatcaaa tacaagggct accaggtagc cccagccgaa     660
   ctggagagca tcctgctgca acaccccaac atcttcgacg ccggggtcgc cggcctgccc     720
   gacgacgatg ccggcgagct gcccgccgca gtcgtcgtgc tggaacacgg taaaaccatg     780
   accgagaagg agatcgtgga ctatgtggcc agccaggtta caaccgccaa gaagctgcgc     840
   ggtggtgttg tgttcgtgga cgaggtgcct aaaggactga ccggcaagtt ggacgcccgc     900
   aagatccgcg agattctcat taaggccaag aagggctcga gcgagaacct gtacttccaa     960
   tcgagctccg gtgccaaaaa cattaagaag ggcccagcgc cattctaccc actcgaagac    1020
   gggaccgccg gcgagcagct gcacaaagcc atgaagcgct acgccctggt gcccggcacc    1080
   atcgccttta ccgacgcaca tatcgaggtg gacattacct acgccgagta cttcgagatg    1140
   agcgttcggc tggcagaagc tatgaagcgc tatgggctga atacaaacca tcggatcgtg    1200
   gtgtgcagcg agaatagctt gcagttcttc atgcccgtgt tgggtgccct gttcatcggt    1260
   gtggctgtgg ccccagctaa cgacatctac aacgagcgcg agctgctgaa cagcatgggc    1320
   atcagccagc ccaccgtcgt attcgtgagc aagaaagggc tgcaaaagat cctcaacgtg    1380
   caaaagaagc taccgatcat acaaaagatc atcatcatgg atagcaagac cgactaccag    1440
   ggcttccaaa gcatgtacac cttcgtgact tcccatttgc cacccggctt caacgagtac    1500
   gacttcgtgc ccgagagctt cgaccgggac aaaaccatcg ccctgatcat gaacagtagt    1560
   ggcagtaccg gattgcccaa gggcgtagcc ctaccgcacc gcaccgcttg tgtccgattc    1620
   agtcatgccc gcgaccccat cttcggcaac cagatcatcc cctgagttta a             1671
<210> 64
   <211> 554
   <212> PRT
   <213> Artificial
<220>
   <223> Synthetic
<400> 64
   Met Thr Ala Ile Leu Ser Val Val Pro Phe His His Gly Phe Gly Met
   1               5                   10                  15
   Phe Thr Thr Leu Gly Tyr Leu Ile Cys Gly Phe Arg Val Val Leu Met
               20                  25                  30
   Tyr Arg Phe Glu Glu Glu Leu Phe Leu Arg Ser Leu Gln Asp Tyr Lys
           35                  40                  45
   Ile Gln Ser Ala Leu Leu Val Pro Thr Leu Phe Ser Phe Phe Ala Lys
       50                  55                  60
   Ser Thr Leu Ile Asp Lys Tyr Asp Leu Ser Asn Leu His Glu Ile Ala
   65                  70                  75                  80
   Ser Gly Gly Ala Pro Leu Ser Lys Glu Val Gly Glu Ala Val Ala Lys
                   85                  90                  95
   Arg Phe His Leu Pro Gly Ile Arg Gln Gly Tyr Gly Leu Thr Glu Thr
               100                 105                 110
   Thr Ser Ala Ile Leu Ile Thr Pro Glu Gly Asp Asp Lys Pro Gly Ala
           115                 120                 125
   Val Gly Lys Val Val Pro Phe Phe Glu Ala Lys Val Val Asp Leu Asp
       130                 135                 140
   Thr Gly Lys Thr Leu Gly Val Asn Gln Arg Gly Glu Leu Cys Val Arg
   145 150 155 160
   Gly Pro Met Ile Met Ser Gly Tyr Val Asn Asn Pro Glu Ala Thr Asn
   165 170 175
   Ala Leu Ile Asp Lys Asp Gly Trp Leu His Ser Gly Asp Ile Ala Tyr
   180 185 190
   Trp Asp Glu Asp Glu His Phe Phe Ile Val Asp Arg Leu Lys Ser Leu
   195 200 205
   Ile Lys Tyr Lys Gly Tyr Gln Val Ala Pro Ala Glu Leu Glu Ser Ile
   210 215 220
   Leu Leu Gln His Pro Asn Ile Phe Asp Ala Gly Val Ala Gly Leu Pro
   225 230 235 240
   Asp Asp Asp Ala Gly Glu Leu Pro Ala Ala Val Val Val Leu Glu His
   245 250 255
   Gly Lys Thr Met Thr Glu Lys Glu Ile Val Asp Tyr Val Ala Ser Gln
   260 265 270
   Val Thr Thr Ala Lys Lys Leu Arg Gly Gly Val Val Phe Val Asp Glu
   275 280 285
   Val Pro Lys Gly Leu Thr Gly Lys Leu Asp Ala Arg Lys Ile Arg Glu
   290 295 300
   Ile Leu Ile Lys Ala Lys Lys Gly Ser Ser Glu Asn Leu Tyr Phe Gln
   305 310 315 320
   Ser Ser Ser Gly Ala Lys Asn Ile Lys Lys Gly Pro Ala Pro Phe Tyr
   325 330 335
   Pro Leu Glu Asp Gly Thr Ala Gly Glu Gln Leu His Lys Ala Met Lys
   340 345 350
   Arg Tyr Ala Leu Val Pro Gly Thr Ile Ala Phe Thr Asp Ala His Ile
   355 360 365
   Glu Val Asp Ile Thr Tyr Ala Glu Tyr Phe Glu Met Ser Val Arg Leu
   370 375 380
   Ala Glu Ala Met Lys Arg Tyr Gly Leu Asn Thr Asn His Arg Ile Val
   385 390 395 400
   Val Cys Ser Glu Asn Ser Leu Gln Phe Phe Met Pro Val Leu Gly Ala
                   405                 410                 415
   Leu Phe Ile Gly Val Ala Val Ala Pro Ala Asn Asp Ile Tyr Asn Glu
               420                 425                 430
   Arg Glu Leu Leu Asn Ser Met Gly Ile Ser Gln Pro Thr Val Val Phe
           435                 440                 445
   Val Ser Lys Lys Gly Leu Gln Lys Ile Leu Asn Val Gln Lys Lys Leu
       450                 455                 460
   Pro Ile Ile Gln Lys Ile Ile Ile Met Asp Ser Lys Thr Asp Tyr Gln
   465                 470                 475                 480
   Gly Phe Gln Ser Met Tyr Thr Phe Val Thr Ser His Leu Pro Pro Gly
                   485                 490                 495
   Phe Asn Glu Tyr Asp Phe Val Pro Glu Ser Phe Asp Arg Asp Lys Thr
               500                 505                 510
   Ile Ala Leu Ile Met Asn Ser Ser Gly Ser Thr Gly Leu Pro Lys Gly
           515                 520                 525
   Val Ala Leu Pro His Arg Thr Ala Cys Val Arg Phe Ser His Ala Arg
       530                 535                 540
   Asp Pro Ile Phe Gly Asn Gln Ile Ile Pro
   545                 550
<210> 65
   <211> 1671
   <212> DNA
   <213> Artificial
<220>
   <223> Synthetic
<400> 65
   atggaggagg agctattctt gcgcagcttg caagactata agattcaatc tgccctgctg      60
   gtgcccacac tatttagctt cttcgctaag agcactctca tcgacaagta cgacctaagc     120
   aacttgcacg agatcgccag cggcggggcg ccgctcagca aggaggtagg tgaggccgtg     180
   gccaaacgct tccacctacc aggcatccgc cagggctacg gcctgacaga aacaaccagc     240
   gccattctga tcacccccga aggggacgac aagcctggcg cagtaggcaa ggtggtgccc     300
   ttcttcgagg ctaaggtggt ggacttggac accggtaaga cactgggtgt gaaccagcgc     360
   ggcgagctgt gcgtccgtgg ccccatgatc atgagcggct acgttaacaa ccccgaggct    420
   acaaacgctc tcatcgacaa ggacggctgg ctgcacagcg gcgacatcgc ctactgggac    480
   gaggacgagc acttcttcat cgtggaccgg ctgaagagcc tgatcaaata caagggctac    540
   caggtagccc cagccgaact ggagagcatc ctgctgcaac accccaacat cttcgacgcc    600
   ggggtcgccg gcctgcccga cgacgatgcc ggcgagctgc ccgccgcagt cgtcgtgctg    660
   gaacacggta aaaccatgac cgagaaggag atcgtggact atgtggccag ccaggttaca    720
   accgccaaga agctgcgcgg tggtgttgtg ttcgtggacg aggtgcctaa aggactgacc    780
   ggcaagttgg acgcccgcaa gatccgcgag attctcatta aggccaagaa gggctcgagc    840
   gagaacctgt acttccaatc gagctccggt gccaaaaaca ttaagaaggg cccagcgcca    900
   ttctacccac tcgaagacgg gaccgccggc gagcagctgc acaaagccat gaagcgctac    960
   gccctggtgc ccggcaccat cgcctttacc gacgcacata tcgaggtgga cattacctac   1020
   gccgagtact tcgagatgag cgttcggctg gcagaagcta tgaagcgcta tgggctgaat   1080
   acaaaccatc ggatcgtggt gtgcagcgag aatagcttgc agttcttcat gcccgtgttg   1140
   ggtgccctgt tcatcggtgt ggctgtggcc ccagctaacg acatctacaa cgagcgcgag   1200
   ctgctgaaca gcatgggcat cagccagccc accgtcgtat tcgtgagcaa gaaagggctg   1260
   caaaagatcc tcaacgtgca aaagaagcta ccgatcatac aaaagatcat catcatggat   1320
   agcaagaccg actaccaggg cttccaaagc atgtacacct tcgtgacttc ccatttgcca   1380
   cccggcttca acgagtacga cttcgtgccc gagagcttcg accgggacaa aaccatcgcc   1440
   ctgatcatga acagtagtgg cagtaccgga ttgcccaagg gcgtagccct accgcaccgc   1500
   accgcttgtg tccgattcag tcatgcccgc gaccccatct tcggcaacca gatcatcccc   1560
   gacaccgcta tcctcagcgt ggtgccattt caccacggct tcggcatgtt caccacgctg   1620
   ggctacttga tctgcggctt tcgggtcgtg ctcatgtacc gctgagttta a            1671
<210> 66
   <211> 554
   <212> PRT
   <213> Artificial
<220>
   <223> Synthetic
<400> 66
   Met Glu Glu Glu Leu Phe Leu Arg Ser Leu Gln Asp Tyr Lys Ile Gln
   1               5                   10                  15
   Ser Ala Leu Leu Val Pro Thr Leu Phe Ser Phe Phe Ala Lys Ser Thr
               20                  25                  30
   Leu Ile Asp Lys Tyr Asp Leu Ser Asn Leu His Glu Ile Ala Ser Gly
           35                  40                  45
   Gly Ala Pro Leu Ser Lys Glu Val Gly Glu Ala Val Ala Lys Arg Phe
       50                  55                  60
   His Leu Pro Gly Ile Arg Gln Gly Tyr Gly Leu Thr Glu Thr Thr Ser
   65                  70                  75                  80
   Ala Ile Leu Ile Thr Pro Glu Gly Asp Asp Lys Pro Gly Ala Val Gly
                   85                  90                  95
   Lys Val Val Pro Phe Phe Glu Ala Lys Val Val Asp Leu Asp Thr Gly
               100                 105                 110
   Lys Thr Leu Gly Val Asn Gln Arg Gly Glu Leu Cys Val Arg Gly Pro
           115                 120                 125
   Met Ile Met Ser Gly Tyr Val Asn Asn Pro Glu Ala Thr Asn Ala Leu
       130                 135                 140
   Ile Asp Lys Asp Gly Trp Leu His Ser Gly Asp Ile Ala Tyr Trp Asp
   145                 150                 155                 160
   Glu Asp Glu His Phe Phe Ile Val Asp Arg Leu Lys Ser Leu Ile Lys
                   165                 170                 175
   Tyr Lys Gly Tyr Gln Val Ala Pro Ala Glu Leu Glu Ser Ile Leu Leu
               180                 185                 190
   Gln His Pro Asn Ile Phe Asp Ala Gly Val Ala Gly Leu Pro Asp Asp
           195                 200                 205
   Asp Ala Gly Glu Leu Pro Ala Ala Val Val Val Leu Glu His Gly Lys
       210                 215                 220
   Thr Met Thr Glu Lys Glu Ile Val Asp Tyr Val Ala Ser Gln Val Thr
   225                 230                 235                 240
   Thr Ala Lys Lys Leu Arg Gly Gly Val Val Phe Val Asp Glu Val Pro
                   245                 250                 255
   Lys Gly Leu Thr Gly Lys Leu Asp Ala Arg Lys Ile Arg Glu Ile Leu
               260                 265                 270
   Ile Lys Ala Lys Lys Gly Ser Ser Glu Asn Leu Tyr Phe Gln Ser Ser
           275                 280                 285
   Ser Gly Ala Lys Asn Ile Lys Lys Gly Pro Ala Pro Phe Tyr Pro Leu
   290 295 300
   Glu Asp Gly Thr Ala Gly Glu Gln Leu His Lys Ala Met Lys Arg Tyr
   305 310 315 320
   Ala Leu Val Pro Gly Thr Ile Ala Phe Thr Asp Ala His Ile Glu Val
   325 330 335
   Asp Ile Thr Tyr Ala Glu Tyr Phe Glu Met Ser Val Arg Leu Ala Glu
   340 345 350
   Ala Met Lys Arg Tyr Gly Leu Asn Thr Asn His Arg Ile Val Val Cys
   355 360 365
   Ser Glu Asn Ser Leu Gln Phe Phe Met Pro Val Leu Gly Ala Leu Phe
   370 375 380
   Ile Gly Val Ala Val Ala Pro Ala Asn Asp Ile Tyr Asn Glu Arg Glu
   385 390 395 400
   Leu Leu Asn Ser Met Gly Ile Ser Gln Pro Thr Val Val Phe Val Ser
   405 410 415
   Lys Lys Gly Leu Gln Lys Ile Leu Asn Val Gln Lys Lys Leu Pro Ile
   420 425 430
   Ile Gln Lys Ile Ile Ile Met Asp Ser Lys Thr Asp Tyr Gln Gly Phe
   435 440 445
   Gln Ser Met Tyr Thr Phe Val Thr Ser His Leu Pro Pro Gly Phe Asn
   450 455 460
   Glu Tyr Asp Phe Val Pro Glu Ser Phe Asp Arg Asp Lys Thr Ile Ala
   465 470 475 480
   Leu Ile Met Asn Ser Ser Gly Ser Thr Gly Leu Pro Lys Gly Val Ala
   485 490 495
   Leu Pro His Arg Thr Ala Cys Val Arg Phe Ser His Ala Arg Asp Pro
   500 505 510
   Ile Phe Gly Asn Gln Ile Ile Pro Asp Thr Ala Ile Leu Ser Val Val
   515 520 525
   Pro Phe His His Gly Phe Gly Met Phe Thr Thr Leu Gly Tyr Leu Ile
   530 535 540
   Cys Gly Phe Arg Val Val Leu Met Tyr Arg
   545                 550
<210> 67
   <211> 1662
   <212> DNA
   <213> Artificial
<220>
   <223> Synthetic
<400> 67
   atgcctggcg cagtaggcaa ggtggtgccc ttcttcgagg ctaaggtggt ggacttggac      60
   actggtaaga cactgggtgt gaaccagcgc ggcgagctgt gcgtccgtgg ccccatgatc     120
   atgagcggct acgttaacaa ccccgaggct acaaacgctc tcatcgacaa ggacggctgg     180
   ctgcacagcg gcgacatcgc ctactgggac gaggacgagc acttcttcat cgtggaccgg     240
   ctgaagagcc tgatcaaata caagggctac caggtagccc cagccgaact ggagagcatc     300
   ctgctgcaac accccaacat cttcgacgcc ggggtcgccg gcctgcccga cgacgatgcc     360
   ggcgagctgc ccgccgcagt cgtcgtgctg gaacacggta aaaccatgac cgagaaggag     420
   atcgtggact atgtggccag ccaggttaca accgccaaga agctgcgcgg tggtgttgtg     480
   ttcgtggacg aggtgcctaa aggactgacc ggcaagttgg acgcccgcaa gatccgcgag     540
   attctcatta aggccaagaa gggctcgagc gagaacctgt acttccaatc gagctccggt     600
   gccaaaaaca ttaagaaggg cccagcgcca ttctacccac tcgaagacgg gaccgccggc     660
   gagcagctgc acaaagccat gaagcgctac gccctggtgc ccggcaccat cgcctttacc     720
   gacgcacata tcgaggtgga cattacctac gccgagtact tcgagatgag cgttcggctg     780
   gcagaagcta tgaagcgcta tgggctgaat acaaaccatc ggatcgtggt gtgcagcgag     840
   aatagcttgc agttcttcat gcccgtgttg ggtgccctgt tcatcggtgt ggctgtggcc     900
   ccagctaacg acatctacaa cgagcgcgag ctgctgaaca gcatgggcat cagccagccc     960
   accgtcgtat tcgtgagcaa gaaagggctg caaaagatcc tcaacgtgca aaagaagcta    1020
   ccgatcatac aaaagatcat catcatggat agcaagaccg actaccaggg cttccaaagc    1080
   atgtacacct tcgtgacttc ccatttgcca cccggcttca acgagtacga cttcgtgccc    1140
   gagagcttcg accgggacaa aaccatcgcc ctgatcatga acagtagtgg cagtaccgga    1200
   ttgcccaagg gcgtagccct accgcaccgc accgcttgtg tccgattcag tcatgcccgc    1260
   gaccccatct tcggcaacca gatcatcccc gacaccgcta tcctcagcgt ggtgccattt    1320
   caccacggct tcggcatgtt caccacgctg ggctacttga tctgcggctt tcgggtcgtg    1380
   ctcatgtacc gcttcgagga ggagctattc ttgcgcagct tgcaagacta taagattcaa    1440
   tctgccctgc tggtgcccac actatttagc ttcttcgcta agagcactct catcgacaag 1500
   tacgacctaa gcaacttgca cgagatcgcc agcggcgggg cgccgctcag caaggaggta 1560
   ggtgaggccg tggccaaacg cttccaccta ccaggcatcc gccagggcta cggcctgaca 1620
   gaaacaacca gcgccattct gatcactcca gaaggggttt aa 1662
<210> 68
   <211> 553
   <212> PRT
   <213> Artificial
<220>
   <223> Synthetic
<400> 68
   Met Pro Gly Ala Val Gly Lys Val Val Pro Phe Phe Glu Ala Lys Val
   1               5                   10                  15
   Val Asp Leu Asp Thr Gly Lys Thr Leu Gly Val Asn Gln Arg Gly Glu
               20                  25                  30
   Leu Cys Val Arg Gly Pro Met Ile Met Ser Gly Tyr Val Asn Asn Pro
           35                  40                  45
   Glu Ala Thr Asn Ala Leu Ile Asp Lys Asp Gly Trp Leu His Ser Gly
       50                  55                  60
   Asp Ile Ala Tyr Trp Asp Glu Asp Glu His Phe Phe Ile Val Asp Arg
   65                  70                  75                  80
   Leu Lys Ser Leu Ile Lys Tyr Lys Gly Tyr Gln Val Ala Pro Ala Glu
                   85                  90                  95
   Leu Glu Ser Ile Leu Leu Gln His Pro Asn Ile Phe Asp Ala Gly Val
               100                 105                 110
   Ala Gly Leu Pro Asp Asp Asp Ala Gly Glu Leu Pro Ala Ala Val Val
           115                 120                 125
   Val Leu Glu His Gly Lys Thr Met Thr Glu Lys Glu Ile Val Asp Tyr
       130                 135                 140
   Val Ala Ser Gln Val Thr Thr Ala Lys Lys Leu Arg Gly Gly Val Val
   145                 150                 155                 160
   Phe Val Asp Glu Val Pro Lys Gly Leu Thr Gly Lys Leu Asp Ala Arg
                   165                 170                 175
   Lys Ile Arg Glu Ile Leu Ile Lys Ala Lys Lys Gly Ser Ser Glu Asn
               180                 185                 190
   Leu Tyr Phe Gln Ser Ser Ser Gly Ala Lys Asn Ile Lys Lys Gly Pro
           195                 200                 205
   Ala Pro Phe Tyr Pro Leu Glu Asp Gly Thr Ala Gly Glu Gln Leu His
       210                 215                 220
   Lys Ala Met Lys Arg Tyr Ala Leu Val Pro Gly Thr Ile Ala Phe Thr
   225                 230                 235                 240
   Asp Ala His Ile Glu Val Asp Ile Thr Tyr Ala Glu Tyr Phe Glu Met
                   245                 250                 255
   Ser Val Arg Leu Ala Glu Ala Met Lys Arg Tyr Gly Leu Asn Thr Asn
               260                 265                 270
   His Arg Ile Val Val Cys Ser Glu Asn Ser Leu Gln Phe Phe Met Pro
           275                 280                 285
   Val Leu Gly Ala Leu Phe Ile Gly Val Ala Val Ala Pro Ala Asn Asp
       290                 295                 300
   Ile Tyr Asn Glu Arg Glu Leu Leu Asn Ser Met Gly Ile Ser Gln Pro
   305                 310                 315                 320
   Thr Val Val Phe Val Ser Lys Lys Gly Leu Gln Lys Ile Leu Asn Val
                   325                 330                 335
   Gln Lys Lys Leu Pro Ile Ile Gln Lys Ile Ile Ile Met Asp Ser Lys
               340                 345                 350
   Thr Asp Tyr Gln Gly Phe Gln Ser Met Tyr Thr Phe Val Thr Ser His
           355                 360                 365
   Leu Pro Pro Gly Phe Asn Glu Tyr Asp Phe Val Pro Glu Ser Phe Asp
       370                 375                 380
   Arg Asp Lys Thr Ile Ala Leu Ile Met Asn Ser Ser Gly Ser Thr Gly
   385                 390                 395                 400
   Leu Pro Lys Gly Val Ala Leu Pro His Arg Thr Ala Cys Val Arg Phe
                   405                 410                 415
   Ser His Ala Arg Asp Pro Ile Phe Gly Asn Gln Ile Ile Pro Asp Thr
               420                 425                 430
   Ala Ile Leu Ser Val Val Pro Phe His His Gly Phe Gly Met Phe Thr
           435                 440                 445
   Thr Leu Gly Tyr Leu Ile Cys Gly Phe Arg Val Val Leu Met Tyr Arg
       450                 455                 460
   Phe Glu Glu Glu Leu Phe Leu Arg Ser Leu Gln Asp Tyr Lys Ile Gln
   465                 470                 475                 480
   Ser Ala Leu Leu Val Pro Thr Leu Phe Ser Phe Phe Ala Lys Ser Thr
                   485                 490                 495
   Leu Ile Asp Lys Tyr Asp Leu Ser Asn Leu His Glu Ile Ala Ser Gly
               500                 505                 510
   Gly Ala Pro Leu Ser Lys Glu Val Gly Glu Ala Val Ala Lys Arg Phe
           515                 520                 525
   His Leu Pro Gly Ile Arg Gln Gly Tyr Gly Leu Thr Glu Thr Thr Ser
       530                 535                 540
   Ala Ile Leu Ile Thr Pro Glu Gly Val
   545                 550
<210> 69
   <211> 2385
   <212> DNA
   <213> Artificial
<220>
   <223> Synthetic
<400> 69
   atgtccccta tactaggtta ttggaaaatt aagggccttg tgcaacccac tcgacttctt 60
   ttggaatatc ttgaagaaaa atatgaagag catttgtatg agcgcgatga aggtgataaa 120
   tggcgaaaca aaaagtttga attgggtttg gagtttccca atcttcctta ttatattgat 180
   ggtgatgtta aattaacaca gtctatggcc atcatacgtt atatagctga caagcacaac 240
   atgttgggtg gttgtccaaa agagcgtgca gagatttcaa tgcttgaagg agcggttttg 300
   gatattagat acggtgtttc gagaattgca tatagtaaag actttgaaac tctcaaagtt 360
   gattttctta gcaagctacc tgaaatgctg aaaatgttcg aagatcgttt atgtcataaa 420
   acatatttga atggtgatca tgtaacccat cctgacttca tgttgtatga cgctcttgat 480
   gttgttttat acatggaccc aatgtgcctg gatgcgttcc caaaattagt ttgtttcaaa 540
   aaacgtattg aagctatccc acaaattgat aagtacttga aatccagcaa gtatatagca 600
   tggcctttgc agggctggca agccacgttt ggtggtggcg accatcctcc aaaatccgga 660
   ggtggtggcg gagaaaacct gtacttccaa gcgatcgcca tgccgggatc aactggtaaa     720
   atagtaccat tacacgctgt taaagttgtc gatcctacaa caggaaaaat tttggggcca     780
   aatgaacctg gagaattgta ttttaaaggc ccgatgataa tgaagggtta ttataataat     840
   gaagaagcta ctaaagcaat tattgataat gacggatggt tgcgctctgg tgatattgct     900
   tattatgaca atgatggcca tttttatatt gtggacaggc tgaagtcact gattaaatat     960
   aaaggttatc aggttgcacc tgctgaaatt gagggaatac tcttacaaca tccgtatatt    1020
   gttgatgccg gcgttactgg tataccggat gaagccgcgg gcgagcttcc agctgcaggt    1080
   gttgtagtac agactggaaa atatctaaac gaacaaatcg tacaagatta tgttgccagt    1140
   caagtttcaa tagccaaatg gctacgtggt ggggtgaaat ttttggatga aattcccaaa    1200
   ggatcaactg gaaaaattga cagaaaagtg ttaagacaaa tgttagaaaa acacaccaat    1260
   ggatccccgt tgggaatgtg gagtcgaagc ttgaaaaaca tcctgtatgg tccggaaccg    1320
   ttctacccac tggaagatgg taccgctggt gaacagatgt ttgacgcatt atctcgttat    1380
   gcagctattc cgggctgcat agcattgaca aatgctcata caaaagaaaa tgttttatat    1440
   gaagagtttc tgaaactgtc gtgtcgttta gcggaaagtt ttaaaaagta tggattaaaa    1500
   caaaacgaca caatagcggt gtgtagcgaa aatagtctgc aatttttcct tcctgtaatt    1560
   gcatcattgt atcttggaat aattgtggca cctgttaacg ataaatacat tgaacgtgaa    1620
   ttaatacaca gtcttggtat tgtaaaacca cgcatagttt tttgctccaa gaatactttt    1680
   caaaaagtac tgaatgtaaa atctaaatta aaatctattg aaactattat tatattagac    1740
   ttaaatgaag acttaggagg ttatcaatgc ctcaacaact ttatttctca aaattccgat    1800
   agtaatctgg acgtaaaaaa atttaaaccc tattctttta atcgagacga tcaggttgcg    1860
   tcgattatgt tttcttctgg tacaactggt ctgccgaagg gagtcatgct aactcacaag    1920
   aatattgttg cacgattttc tattgcaaaa gatcctactt ttggtaacgc aattaatccc    1980
   acgtcagcaa ttttaacggt aatacctttc caccatggtt ttggtatgat gaccacatta    2040
   ggatacttta cttgtggatt ccgagttgtt ctaatgcaca cgtttgaaga aaaactattt    2100
   ctacaatcat tacaagatta taaagtggaa agtactttac ttgtaccaac attaatggca    2160
   tttcttgcaa aaagtgcatt agttgaaaag tacgatttat cgcacttaaa agaaattgca    2220
   tctggtggcg cacctttatc aaaagaaatt ggggagatgg tgaaaaaacg gtttaaatta    2280
   aactttgtca ggcaagggta tggattaaca gaaaccactt cggctgtttt aattacaccg    2340
   aaaggtgttt ctcaccaaca ccaacaccag caccaacacc agtaa                    2385
<210> 70
   <211> 794
   <212> PRT
   <213> Artificial
<220>
   <223> Synthetic
<400> 70
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Gly Gly Gly Gly Gly
       210                 215                 220
   Glu Asn Leu Tyr Phe Gln Ala Ile Ala Met Pro Gly Ser Thr Gly Lys
   225 230 235 240
   Ile Val Pro Leu His Ala Val Lys Val Val Asp Pro Thr Thr Gly Lys
   245 250 255
   Ile Leu Gly Pro Asn Glu Pro Gly Glu Leu Tyr Phe Lys Gly Pro Met
   260 265 270
   Ile Met Lys Gly Tyr Tyr Asn Asn Glu Glu Ala Thr Lys Ala Ile Ile
   275 280 285
   Asp Asn Asp Gly Trp Leu Arg Ser Gly Asp Ile Ala Tyr Tyr Asp Asn
   290 295 300
   Asp Gly His Phe Tyr Ile Val Asp Arg Leu Lys Ser Leu Ile Lys Tyr
   305 310 315 320
   Lys Gly Tyr Gln Val Ala Pro Ala Glu Ile Glu Gly Ile Leu Leu Gln
   325 330 335
   His Pro Tyr Ile Val Asp Ala Gly Val Thr Gly Ile Pro Asp Glu Ala
   340 345 350
   Ala Gly Glu Leu Pro Ala Ala Gly Val Val Val Gln Thr Gly Lys Tyr
   355 360 365
   Leu Asn Glu Gln Ile Val Gln Asp Tyr Val Ala Ser Gln Val Ser Ile
   370 375 380
   Ala Lys Trp Leu Arg Gly Gly Val Lys Phe Leu Asp Glu Ile Pro Lys
   385 390 395 400
   Gly Ser Thr Gly Lys Ile Asp Arg Lys Val Leu Arg Gln Met Leu Glu
   405 410 415
   Lys His Thr Asn Gly Ser Pro Leu Gly Met Trp Ser Arg Ser Leu Lys
   420 425 430
   Asn Ile Leu Tyr Gly Pro Glu Pro Phe Tyr Pro Leu Glu Asp Gly Thr
   435 440 445
   Ala Gly Glu Gln Met Phe Asp Ala Leu Ser Arg Tyr Ala Ala Ile Pro
   450 455 460
   Gly Cys Ile Ala Leu Thr Asn Ala His Thr Lys Glu Asn Val Leu Tyr
   465 470 475 480
   Glu Glu Phe Leu Lys Leu Ser Cys Arg Leu Ala Glu Ser Phe Lys Lys
   485 490 495
   Tyr Gly Leu Lys Gln Asn Asp Thr Ile Ala Val Cys Ser Glu Asn Ser
   500 505 510
   Leu Gln Phe Phe Leu Pro Val Ile Ala Ser Leu Tyr Leu Gly Ile Ile
   515 520 525
   Val Ala Pro Val Asn Asp Lys Tyr Ile Glu Arg Glu Leu Ile His Ser
   530 535 540
   Leu Gly Ile Val Lys Pro Arg Ile Val Phe Cys Ser Lys Asn Thr Phe
   545 550 555 560
   Gln Lys Val Leu Asn Val Lys Ser Lys Leu Lys Ser Ile Glu Thr Ile
   565 570 575
   Ile Ile Leu Asp Leu Asn Glu Asp Leu Gly Gly Tyr Gln Cys Leu Asn
   580 585 590
   Asn Phe Ile Ser Gln Asn Ser Asp Ser Asn Leu Asp Val Lys Lys Phe
   595 600 605
   Lys Pro Tyr Ser Phe Asn Arg Asp Asp Gln Val Ala Ser Ile Met Phe
   610 615 620
   Ser Ser Gly Thr Thr Gly Leu Pro Lys Gly Val Met Leu Thr His Lys
   625 630 635 640
   Asn Ile Val Ala Arg Phe Ser Ile Ala Lys Asp Pro Thr Phe Gly Asn
   645 650 655
   Ala Ile Asn Pro Thr Ser Ala Ile Leu Thr Val Ile Pro Phe His His
   660 665 670
   Gly Phe Gly Met Met Thr Thr Leu Gly Tyr Phe Thr Cys Gly Phe Arg
   675 680 685
   Val Val Leu Met His Thr Phe Glu Glu Lys Leu Phe Leu Gln Ser Leu
   690 695 700
   Gln Asp Tyr Lys Val Glu Ser Thr Leu Leu Val Pro Thr Leu Met Ala
   705 710 715 720
   Phe Leu Ala Lys Ser Ala Leu Val Glu Lys Tyr Asp Leu Ser His Leu
   725 730 735
   Lys Glu Ile Ala Ser Gly Gly Ala Pro Leu Ser Lys Glu Ile Gly Glu
   740 745 750
   Met Val Lys Lys Arg Phe Lys Leu Asn Phe Val Arg Gln Gly Tyr Gly
   755 760 765
   Leu Thr Glu Thr Thr Ser Ala Val Leu Ile Thr Pro Lys Gly Val Ser
   770 775 780
   His Gln His Gln His Gln His Gln His Gln
   785 790
<210> 71
   <211> 2583
   <212> DNA
   <213> Artificial
<220>
   <223> Synthetic
<400> 71
   atggcagaaa tcggtactgg ctttccattc gacccccatt atgtggaagt cctgggcgag 60
   cgcatgcact acgtcgatgt tggtccgcgc gatggcaccc ctgtgctgtt cctgcacggt 120
   aacccgacct cctcctacgt gtggcgcaac atcatcccgc atgttgcacc gacccatcgc 180
   tgcattgctc cagacctgat cggtatgggc aaatccgaca aaccagacct gggttatttc 240
   ttcgacgacc acgtccgctt catggatgcc ttcatcgaag ccctgggtct ggaagaggtc 300
   gtcctggtca ttcacgactg gggctccgct ctgggtttcc actgggccaa gcgcaatcca 360
   gagcgcgtca aaggtattgc atttatggag ttcatccgcc ctatcccgac ctgggacgaa 420
   tggccagaat ttgcccgcga gaccttccag gccttccgca ccaccgacgt cggccgcaag 480
   ctgatcatcg atcagaacgt ttttatcgag ggtacgctgc cgatgggtgt cgtccgcccg 540
   ctgactgaag tcgagatgga ccattaccgc gagccgttcc tgaatcctgt tgaccgcgag 600
   ccactgtggc gcttcccaaa cgagctgcca atcgccggtg agccagcgaa catcgtcgcg 660
   ctggtcgaag aatacatgga ctggctgcac cagtcccctg tcccgaagct gctgttctgg 720
   ggcaccccag gcgttctgat cccaccggcc gaagccgctc gcctggccaa aagcctgcct 780
   aactgcaagg ctgtggacat cggcccgggt ctgaatctgc tgcaagaaga caacccggac 840
   ctgatcggca gcgagatcgc gcgctggctg tcgacgctcg agatttccgg cgagccaacc 900
   actgaggatc tgtactttca gagcgataac gcgatcgcca tgccgggatc aactggtaaa 960
   atagtaccat tacacgctgt taaagttgtc gatcctacaa caggaaaaat tttggggcca 1020
   aatgaacctg gagaattgta ttttaaaggc ccgatgataa tgaagggtta ttataataat 1080
   gaagaagcta ctaaagcaat tattgataat gacggatggt tgcgctctgg tgatattgct 1140
   tattatgaca atgatggcca tttttatatt gtggacaggc tgaagtcact gattaaatat 1200
   aaaggttatc aggttgcacc tgctgaaatt gagggaatac tcttacaaca tccgtatatt 1260
   gttgatgccg gcgttactgg tataccggat gaagccgcgg gcgagcttcc agctgcaggt 1320
   gttgtagtac agactggaaa atatctaaac gaacaaatcg tacaagatta tgttgccagt 1380
   caagtttcaa tagccaaatg gctacgtggt ggggtgaaat ttttggatga aattcccaaa 1440
   ggatcaactg gaaaaattga cagaaaagtg ttaagacaaa tgttagaaaa acacaccaat 1500
   ggatccgacg aggtggacgg aagcttgaaa aacatcctgt atggtccgga accgttctac 1560
   ccactggaag atggtaccgc tggtgaacag atgtttgacg cattatctcg ttatgcagct 1620
   attccgggct gcatagcatt gacaaatgct catacaaaag aaaatgtttt atatgaagag 1680
   tttctgaaac tgtcgtgtcg tttagcggaa agttttaaaa agtatggatt aaaacaaaac 1740
   gacacaatag cggtgtgtag cgaaaatagt ctgcaatttt tccttcctgt aattgcatca 1800
   ttgtatcttg gaataattgt ggcacctgtt aacgataaat acattgaacg tgaattaata 1860
   cacagtcttg gtattgtaaa accacgcata gttttttgct ccaagaatac ttttcaaaaa 1920
   gtactgaatg taaaatctaa attaaaatct attgaaacta ttattatatt agacttaaat 1980
   gaagacttag gaggttatca atgcctcaac aactttattt ctcaaaattc cgatagtaat 2040
   ctggacgtaa aaaaatttaa accctattct tttaatcgag acgatcaggt tgcgtcgatt 2100
   atgttttctt ctggtacaac tggtctgccg aagggagtca tgctaactca caagaatatt 2160
   gttgcacgat tttctattgc aaaagatcct acttttggta acgcaattaa tcccacgtca 2220
   gcaattttaa cggtaatacc tttccaccat ggttttggta tgatgaccac attaggatac 2280
   tttacttgtg gattccgagt tgttctaatg cacacgtttg aagaaaaact atttctacaa 2340
   tcattacaag attataaagt ggaaagtact ttacttgtac caacattaat ggcatttctt 2400
   gcaaaaagtg cattagttga aaagtacgat ttatcgcact taaaagaaat tgcatctggt 2460
   ggcgcacctt tatcaaaaga aattggggag atggtgaaaa aacggtttaa attaaacttt 2520
   gtcaggcaag ggtatggatt aacagaaacc acttcggctg ttttaattac accgaaaggt 2580
   gtt 2583
<210> 72
   <211> 861
   <212> PRT
   <213> Artificial
<220>
   <223> Synthetic
<400> 72
   Met Ala Glu Ile Gly Thr Gly Phe Pro Phe Asp Pro His Tyr Val Glu
   1               5                   10                  15
   Val Leu Gly Glu Arg Met His Tyr Val Asp Val Gly Pro Arg Asp Gly
               20                  25                  30
   Thr Pro Val Leu Phe Leu His Gly Asn Pro Thr Ser Ser Tyr Val Trp
           35                  40                  45
   Arg Asn Ile Ile Pro His Val Ala Pro Thr His Arg Cys Ile Ala Pro
       50                  55                  60
   Asp Leu Ile Gly Met Gly Lys Ser Asp Lys Pro Asp Leu Gly Tyr Phe
   65                  70                  75                  80
   Phe Asp Asp His Val Arg Phe Met Asp Ala Phe Ile Glu Ala Leu Gly
                   85                  90                  95
   Leu Glu Glu Val Val Leu Val Ile His Asp Trp Gly Ser Ala Leu Gly
               100                 105                 110
   Phe His Trp Ala Lys Arg Asn Pro Glu Arg Val Lys Gly Ile Ala Phe
           115                 120                 125
   Met Glu Phe Ile Arg Pro Ile Pro Thr Trp Asp Glu Trp Pro Glu Phe
       130                 135                 140
   Ala Arg Glu Thr Phe Gln Ala Phe Arg Thr Thr Asp Val Gly Arg Lys
   145                 150                 155                 160
   Leu Ile Ile Asp Gln Asn Val Phe Ile Glu Gly Thr Leu Pro Met Gly
                   165                 170                 175
   Val Val Arg Pro Leu Thr Glu Val Glu Met Asp His Tyr Arg Glu Pro
               180                 185                 190
   Phe Leu Asn Pro Val Asp Arg Glu Pro Leu Trp Arg Phe Pro Asn Glu
           195                 200                 205
   Leu Pro Ile Ala Gly Glu Pro Ala Asn Ile Val Ala Leu Val Glu Glu
       210                 215                 220
   Tyr Met Asp Trp Leu His Gln Ser Pro Val Pro Lys Leu Leu Phe Trp
   225                 230                 235                 240
   Gly Thr Pro Gly Val Leu Ile Pro Pro Ala Glu Ala Ala Arg Leu Ala
                   245                 250                 255
   Lys Ser Leu Pro Asn Cys Lys Ala Val Asp Ile Gly Pro Gly Leu Asn
               260                 265                 270
   Leu Leu Gln Glu Asp Asn Pro Asp Leu Ile Gly Ser Glu Ile Ala Arg
           275                 280                 285
   Trp Leu Ser Thr Leu Glu Ile Ser Gly Glu Pro Thr Thr Glu Asp Leu
       290                 295                 300
   Tyr Phe Gln Ser Asp Asn Ala Ile Ala Met Pro Gly Ser Thr Gly Lys
   305                 310                 315                 320
   Ile Val Pro Leu His Ala Val Lys Val Val Asp Pro Thr Thr Gly Lys
                   325                 330                 335
   Ile Leu Gly Pro Asn Glu Pro Gly Glu Leu Tyr Phe Lys Gly Pro Met
               340                 345                 350
   Ile Met Lys Gly Tyr Tyr Asn Asn Glu Glu Ala Thr Lys Ala Ile Ile
           355                 360                 365
   Asp Asn Asp Gly Trp Leu Arg Ser Gly Asp Ile Ala Tyr Tyr Asp Asn
       370                 375                 380
   Asp Gly His Phe Tyr Ile Val Asp Arg Leu Lys Ser Leu Ile Lys Tyr
   385                 390                 395                 400
   Lys Gly Tyr Gln Val Ala Pro Ala Glu Ile Glu Gly Ile Leu Leu Gln
                   405                 410                 415
   His Pro Tyr Ile Val Asp Ala Gly Val Thr Gly Ile Pro Asp Glu Ala
               420                 425                 430
   Ala Gly Glu Leu Pro Ala Ala Gly Val Val Val Gln Thr Gly Lys Tyr
           435                 440                 445
   Leu Asn Glu Gln Ile Val Gln Asp Tyr Val Ala Ser Gln Val Ser Ile
       450                 455                 460
   Ala Lys Trp Leu Arg Gly Gly Val Lys Phe Leu Asp Glu Ile Pro Lys
   465                 470                 475                 480
   Gly Ser Thr Gly Lys Ile Asp Arg Lys Val Leu Arg Gln Met Leu Glu
                   485                 490                 495
   Lys His Thr Asn Gly Ser Asp Glu Val Asp Gly Ser Leu Lys Asn Ile
               500                 505                 510
   Leu Tyr Gly Pro Glu Pro Phe Tyr Pro Leu Glu Asp Gly Thr Ala Gly
   515 520 525
   Glu Gln Met Phe Asp Ala Leu Ser Arg Tyr Ala Ala Ile Pro Gly Cys
   530 535 540
   Ile Ala Leu Thr Asn Ala His Thr Lys Glu Asn Val Leu Tyr Glu Glu
   545 550 555 560
   Phe Leu Lys Leu Ser Cys Arg Leu Ala Glu Ser Phe Lys Lys Tyr Gly
   565 570 575
   Leu Lys Gln Asn Asp Thr Ile Ala Val Cys Ser Glu Asn Ser Leu Gln
   580 585 590
   Phe Phe Leu Pro Val Ile Ala Ser Leu Tyr Leu Gly Ile Ile Val Ala
   595 600 605
   Pro Val Asn Asp Lys Tyr Ile Glu Arg Glu Leu Ile His Ser Leu Gly
   610 615 620
   Ile Val Lys Pro Arg Ile Val Phe Cys Ser Lys Asn Thr Phe Gln Lys
   625 630 635 640
   Val Leu Asn Val Lys Ser Lys Leu Lys Ser Ile Glu Thr Ile Ile Ile
   645 650 655
   Leu Asp Leu Asn Glu Asp Leu Gly Gly Tyr Gln Cys Leu Asn Asn Phe
   660 665 670
   Ile Ser Gln Asn Ser Asp Ser Asn Leu Asp Val Lys Lys Phe Lys Pro
   675 680 685
   Tyr Ser Phe Asn Arg Asp Asp Gln Val Ala Ser Ile Met Phe Ser Ser
   690 695 700
   Gly Thr Thr Gly Leu Pro Lys Gly Val Met Leu Thr His Lys Asn Ile
   705 710 715 720
   Val Ala Arg Phe Ser Ile Ala Lys Asp Pro Thr Phe Gly Asn Ala Ile
   725 730 735
   Asn Pro Thr Ser Ala Ile Leu Thr Val Ile Pro Phe His His Gly Phe
   740 745 750
   Gly Met Met Thr Thr Leu Gly Tyr Phe Thr Cys Gly Phe Arg Val Val
   755 760 765
   Leu Met His Thr Phe Glu Glu Lys Leu Phe Leu Gln Ser Leu Gln Asp
   770 775 780
   Tyr Lys Val Glu Ser Thr Leu Leu Val Pro Thr Leu Met Ala Phe Leu
   785 790 795 800
   Ala Lys Ser Ala Leu Val Glu Lys Tyr Asp Leu Ser His Leu Lys Glu
   805 810 815
   Ile Ala Ser Gly Gly Ala Pro Leu Ser Lys Glu Ile Gly Glu Met Val
   820 825 830
   Lys Lys Arg Phe Lys Leu Asn Phe Val Arg Gln Gly Tyr Gly Leu Thr
   835 840 845
   Glu Thr Thr Ser Ala Val Leu Ile Thr Pro Lys Gly Val
   850 855 860
<210> 73
   <211> 2616
   <212> DNA
   <213> Artificial
<220>
   <223> Synthetic
<400> 73
   atgaaacatc aacatcagca ccaagcgatc gccatgccgg gatcaactgg taaaatagta     60
   ccattacacg ctgttaaagt tgtcgatcct acaacaggaa aaattttggg gccaaatgaa    120
   cctggagaat tgtattttaa aggcccgatg ataatgaagg gttattataa taatgaagaa    180
   gctactaaag caattattga taatgacgga tggttgcgct ctggtgatat tgcttattat    240
   gacaatgatg gccattttta tattgtggac aggctgaagt cactgattaa atataaaggt    300
   tatcaggttg cacctgctga aattgaggga atactcttac aacatccgta tattgttgat    360
   gccggcgtta ctggtatacc ggatgaagcc gcgggcgagc ttccagctgc aggtgttgta    420
   gtacagactg gaaaatatct aaacgaacaa atcgtacaag attatgttgc cagtcaagtt    480
   tcaatagcca aatggctacg tggtggggtg aaatttttgg atgaaattcc caaaggatca    540
   actggaaaaa ttgacagaaa agtgttaaga caaatgttag aaaaacacac caatggatcc    600
   gacgaggtgg acggaagctt gaaaaacatc ctgtatggtc cggaaccgtt ctacccactg    660
   gaagatggta ccgctggtga acagatgttt gacgcattat ctcgttatgc agctattccg    720
   ggctgcatag cattgacaaa tgctcataca aaagaaaatg ttttatatga agagtttctg    780
   aaactgtcgt gtcgtttagc ggaaagtttt aaaaagtatg gattaaaaca aaacgacaca    840
   atagcggtgt gtagcgaaaa tagtctgcaa tttttccttc ctgtaattgc atcattgtat 900
   cttggaataa ttgtggcacc tgttaacgat aaatacattg aacgtgaatt aatacacagt 960
   cttggtattg taaaaccacg catagttttt tgctccaaga atacttttca aaaagtactg 1020
   aatgtaaaat ctaaattaaa atctattgaa actattatta tattagactt aaatgaagac 1080
   ttaggaggtt atcaatgcct caacaacttt atttctcaaa attccgatag taatctggac 1140
   gtaaaaaaat ttaaacccta ttcttttaat cgagacgatc aggttgcgtc gattatgttt 1200
   tcttctggta caactggtct gccgaaggga gtcatgctaa ctcacaagaa tattgttgca 1260
   cgattttcta ttgcaaaaga tcctactttt ggtaacgcaa ttaatcccac gtcagcaatt 1320
   ttaacggtaa tacctttcca ccatggtttt ggtatgatga ccacattagg atactttact 1380
   tgtggattcc gagttgttct aatgcacacg tttgaagaaa aactatttct acaatcatta 1440
   caagattata aagtggaaag tactttactt gtaccaacat taatggcatt tcttgcaaaa 1500
   agtgcattag ttgaaaagta cgatttatcg cacttaaaag aaattgcatc tggtggcgca 1560
   cctttatcaa aagaaattgg ggagatggtg aaaaaacggt ttaaattaaa ctttgtcagg 1620
   caagggtatg gattaacaga aaccacttcg gctgttttaa ttacaccgaa aggtgtttct 1680
   ctcgagccaa ccactgagga tctgtacttt cagagcgata acgatggatc cgaaatcggt 1740
   actggctttc cattcgaccc ccattatgtg gaagtcctgg gcgagcgcat gcactacgtc 1800
   gatgttggtc cgcgcgatgg cacccctgtg ctgttcctgc acggtaaccc gacctcctcc 1860
   tacgtgtggc gcaacatcat cccgcatgtt gcaccgaccc atcgctgcat tgctccagac 1920
   ctgatcggta tgggcaaatc cgacaaacca gacctgggtt atttcttcga cgaccacgtc 1980
   cgcttcatgg atgccttcat cgaagccctg ggtctggaag aggtcgtcct ggtcattcac 2040
   gactggggct ccgctctggg tttccactgg gccaagcgca atccagagcg cgtcaaaggt 2100
   attgcattta tggagttcat ccgccctatc ccgacctggg acgaatggcc agaatttgcc 2160
   cgcgagacct tccaggcctt ccgcaccacc gacgtcggcc gcaagctgat catcgatcag 2220
   aacgttttta tcgagggtac gctgccgatg ggtgtcgtcc gcccgctgac tgaagtcgag 2280
   atggaccatt accgcgagcc gttcctgaat cctgttgacc gcgagccact gtggcgcttc 2340
   ccaaacgagc tgccaatcgc cggtgagcca gcgaacatcg tcgcgctggt cgaagaatac 2400
   atggactggc tgcaccagtc ccctgtcccg aagctgctgt tctggggcac cccaggcgtt 2460
   ctgatcccac cggccgaagc cgctcgcctg gccaaaagcc tgcctaactg caaggctgtg 2520
   gacatcggcc cgggtctgaa tctgctgcaa gaagacaacc cggacctgat cggcagcgag 2580
   atcgcgcgct ggctgtctac tctggagatt tccggt 2616
<210> 74
   <211> 872
   <212> PRT
   <213> Artificial
<220>
   <223> Synthetic
<400> 74
   Met Lys His Gln His Gln His Gln Ala Ile Ala Met Pro Gly Ser Thr
   1               5                   10                  15
   Gly Lys Ile Val Pro Leu His Ala Val Lys Val Val Asp Pro Thr Thr
               20                  25                  30
   Gly Lys Ile Leu Gly Pro Asn Glu Pro Gly Glu Leu Tyr Phe Lys Gly
           35                  40                  45
   Pro Met Ile Met Lys Gly Tyr Tyr Asn Asn Glu Glu Ala Thr Lys Ala
       50                  55                  60
   Ile Ile Asp Asn Asp Gly Trp Leu Arg Ser Gly Asp Ile Ala Tyr Tyr
   65                  70                  75                  80
   Asp Asn Asp Gly His Phe Tyr Ile Val Asp Arg Leu Lys Ser Leu Ile
                   85                  90                  95
   Lys Tyr Lys Gly Tyr Gln Val Ala Pro Ala Glu Ile Glu Gly Ile Leu
               100                 105                 110
   Leu Gln His Pro Tyr Ile Val Asp Ala Gly Val Thr Gly Ile Pro Asp
           115                 120                 125
   Glu Ala Ala Gly Glu Leu Pro Ala Ala Gly Val Val Val Gln Thr Gly
       130                 135                 140
   Lys Tyr Leu Asn Glu Gln Ile Val Gln Asp Tyr Val Ala Ser Gln Val
   145                 150                 155                 160
   Ser Ile Ala Lys Trp Leu Arg Gly Gly Val Lys Phe Leu Asp Glu Ile
                   165                 170                 175
   Pro Lys Gly Ser Thr Gly Lys Ile Asp Arg Lys Val Leu Arg Gln Met
               180                 185                 190
   Leu Glu Lys His Thr Asn Gly Ser Asp Glu Val Asp Gly Ser Leu Lys
           195                 200                 205
   Asn Ile Leu Tyr Gly Pro Glu Pro Phe Tyr Pro Leu Glu Asp Gly Thr
       210                 215                 220
   Ala Gly Glu Gln Met Phe Asp Ala Leu Ser Arg Tyr Ala Ala Ile Pro
   225 230 235 240
   Gly Cys Ile Ala Leu Thr Asn Ala His Thr Lys Glu Asn Val Leu Tyr
   245 250 255
   Glu Glu Phe Leu Lys Leu Ser Cys Arg Leu Ala Glu Ser Phe Lys Lys
   260 265 270
   Tyr Gly Leu Lys Gln Asn Asp Thr Ile Ala Val Cys Ser Glu Asn Ser
   275 280 285
   Leu Gln Phe Phe Leu Pro Val Ile Ala Ser Leu Tyr Leu Gly Ile Ile
   290 295 300
   Val Ala Pro Val Asn Asp Lys Tyr Ile Glu Arg Glu Leu Ile His Ser
   305 310 315 320
   Leu Gly Ile Val Lys Pro Arg Ile Val Phe Cys Ser Lys Asn Thr Phe
   325 330 335
   Gln Lys Val Leu Asn Val Lys Ser Lys Leu Lys Ser Ile Glu Thr Ile
   340 345 350
   Ile Ile Leu Asp Leu Asn Glu Asp Leu Gly Gly Tyr Gln Cys Leu Asn
   355 360 365
   Asn Phe Ile Ser Gln Asn Ser Asp Ser Asn Leu Asp Val Lys Lys Phe
   370 375 380
   Lys Pro Tyr Ser Phe Asn Arg Asp Asp Gln Val Ala Ser Ile Met Phe
   385 390 395 400
   Ser Ser Gly Thr Thr Gly Leu Pro Lys Gly Val Met Leu Thr His Lys
   405 410 415
   Asn Ile Val Ala Arg Phe Ser Ile Ala Lys Asp Pro Thr Phe Gly Asn
   420 425 430
   Ala Ile Asn Pro Thr Ser Ala Ile Leu Thr Val Ile Pro Phe His His
   435 440 445
   Gly Phe Gly Met Met Thr Thr Leu Gly Tyr Phe Thr Cys Gly Phe Arg
   450 455 460
   Val Val Leu Met His Thr Phe Glu Glu Lys Leu Phe Leu Gln Ser Leu
   465 470 475 480
   Gln Asp Tyr Lys Val Glu Ser Thr Leu Leu Val Pro Thr Leu Met Ala
   485 490 495
   Phe Leu Ala Lys Ser Ala Leu Val Glu Lys Tyr Asp Leu Ser His Leu
   500 505 510
   Lys Glu Ile Ala Ser Gly Gly Ala Pro Leu Ser Lys Glu Ile Gly Glu
   515 520 525
   Met Val Lys Lys Arg Phe Lys Leu Asn Phe Val Arg Gln Gly Tyr Gly
   530 535 540
   Leu Thr Glu Thr Thr Ser Ala Val Leu Ile Thr Pro Lys Gly Val Ser
   545 550 555 560
   Leu Glu Pro Thr Thr Glu Asp Leu Tyr Phe Gln Ser Asp Asn Asp Gly
   565 570 575
   Ser Glu Ile Gly Thr Gly Phe Pro Phe Asp Pro His Tyr Val Glu Val
   580 585 590
   Leu Gly Glu Arg Met His Tyr Val Asp Val Gly Pro Arg Asp Gly Thr
   595 600 605
   Pro Val Leu Phe Leu His Gly Asn Pro Thr Ser Ser Tyr Val Trp Arg
   610 615 620
   Asn Ile Ile Pro His Val Ala Pro Thr His Arg Cys Ile Ala Pro Asp
   625 630 635 640
   Leu Ile Gly Met Gly Lys Ser Asp Lys Pro Asp Leu Gly Tyr Phe Phe
   645 650 655
   Asp Asp His Val Arg Phe Met Asp Ala Phe Ile Glu Ala Leu Gly Leu
   660 665 670
   Glu Glu Val Val Leu Val Ile His Asp Trp Gly Ser Ala Leu Gly Phe
   675 680 685
   His Trp Ala Lys Arg Asn Pro Glu Arg Val Lys Gly Ile Ala Phe Met
   690 695 700
   Glu Phe Ile Arg Pro Ile Pro Thr Trp Asp Glu Trp Pro Glu Phe Ala
   705 710 715 720
   Arg Glu Thr Phe Gln Ala Phe Arg Thr Thr Asp Val Gly Arg Lys Leu
   725 730 735
   Ile Ile Asp Gln Asn Val Phe Ile Glu Gly Thr Leu Pro Met Gly Val
   740 745 750
   Val Arg Pro Leu Thr Glu Val Glu Met Asp His Tyr Arg Glu Pro Phe
   755 760 765
   Leu Asn Pro Val Asp Arg Glu Pro Leu Trp Arg Phe Pro Asn Glu Leu
   770 775 780
   Pro Ile Ala Gly Glu Pro Ala Asn Ile Val Ala Leu Val Glu Glu Tyr
   785 790 795 800
   Met Asp Trp Leu His Gln Ser Pro Val Pro Lys Leu Leu Phe Trp Gly
   805 810 815
   Thr Pro Gly Val Leu Ile Pro Pro Ala Glu Ala Ala Arg Leu Ala Lys
   820 825 830
   Ser Leu Pro Asn Cys Lys Ala Val Asp Ile Gly Pro Gly Leu Asn Leu
   835 840 845
   Leu Gln Glu Asp Asn Pro Asp Leu Ile Gly Ser Glu Ile Ala Arg Trp
   850 855 860
   Leu Ser Thr Leu Glu Ile Ser Gly
   865 870
<210> 75
   <211> 7
   <212> PRT
   <213> Artificial
<220>
   <223> Synthetic
<400> 75
   Pro Leu Gly Met Trp Ser Arg
   1 5
<210> 76
   <211> 10
   <212> PRT
   <213> Artificial
<220>
   <223> Synthetic
<400> 76
   Gly Ser Ser Leu Glu Thr Asp Ser Ser Gly
   1               5                   10
<210> 77
   <211> 10
   <212> PRT
   <213> Artificial
<220>
   <223> Synthetic
<400> 77
   Gly Ser Ser Asp Glu Val Asp Ser Ser Gly
   1               5                   10
<210> 78
   <211> 13
   <212> PRT
   <213> Artificial
<220>
   <223> Synthetic
<400> 78
   Gly Ser Ser Glu Asn Leu Tyr Phe Gln Ser Ser Ser Gly
   1               5                   10
<210> 79
   <211> 10
   <212> PRT
   <213> Artificial
<220>
   <223> Synthetic
<400> 79
   His Gln His Gln His Gln His Gln His Gln
   1               5                   10
<210> 80
   <211> 8
   <212> PRT
   <213> Artificial Sequence
<220>
   <223> Synthetic
<400> 80
   Gly Arg Ile Glu Ala Asp Ser Glu
   1               5
<210> 81
   <211> 8
   <212> PRT
   <213> Artificial Sequence
<220>
   <223> Synthetic
<400> 81
   Lys Ser Val Gly Pro Asp Phe Gly
   1 5
<210> 82
   <211> 7
   <212> PRT
   <213> Artificial Sequence
<220>
   <223> Synthetic
<400> 82
   Gly Ile Glu Thr Asp Ser Gly
   1 5
<210> 83
   <211> 4
   <212> PRT
   <213> Artificial Sequence
<220>
   <223> Synthetic
<400> 83
   Ile Glu Ala Ala
   1
<210> 84
   <211> 4
   <212> PRT
   <213> Artificial Sequence
<220>
   <223> Synthetic
<400> 84
   Ile Glu Thr Ala
   1
<210> 85
   <211> 4
   <212> PRT
   <213> Artificial Sequence
<220>
   <223> Synthetic
<400> 85
   Val Gly Pro Ala
   1